(19) Europäisches Patentamt
European Patent Office
Office européen des brevets

(11) Veröffentlichungsnummer: **0 529 253 A1**

(12) # EUROPÄISCHE PATENTANMELDUNG

(21) Anmeldenummer: **92111591.1**

(22) Anmeldetag: **08.07.92**

(51) Int. Cl.[5]: **C07D 235/08**, A61K 31/415, C07D 235/18, C07D 215/22, C07D 239/74, C07D 403/14, C07D 471/04, A61K 31/47, A61K 31/505, A61K 31/41, A61K 31/44

(30) Priorität: **15.07.91 DE 4123341**

(43) Veröffentlichungstag der Anmeldung:
**03.03.93 Patentblatt 93/09**

(84) Benannte Vertragsstaaten:
**AT BE CH DE DK ES FR GB GR IT LI LU NL PT SE**

(71) Anmelder: **Dr. Karl Thomae GmbH**
**Postfach 1755**
**W-7950 Biberach 1(DE)**

(72) Erfinder: **Ries, Uwe, Dr.**
**Dunantstrasse 10**
**W-7950 Biberach 1(DE)**
Erfinder: **Reiffen, Manfred, Dr.**
**Matthias-Erzberger-Strasse 40**
**W-7950 Biberach 1(DE)**
Erfinder: **Grell, Wolfgang, Dr.**
**Geschwister-Scholl-Strasse 18**
**W-7950 Biberach 1(DE)**
Erfinder: **Hauel, Norbert, Dr.**
**Fliederweg 2**
**W-7951 Eberhardzell(DE)**
Erfinder: **Narr, Berthold, Dr.**
**Obere Au 5**
**W-7950 Biberach 1(DE)**
Erfinder: **Heckel, Armin, Dr.**
**Geschwister-Scholl-Strasse 71**
**W-7950 Biberach 1(DE)**
Erfinder: **Bomhard, Andreas, Dr.**
**Max-Born-Strasse 41**
**W-4000 Düsseldorf 13(DE)**
Erfinder: **van Meel, Jacques, Dr.**
**Schubertweg 4**
**W-7951 Mittelbiberach(DE)**
Erfinder: **Wienen, Wolfgang, Dr.**
**Am Schiessberg 28**
**W-7951 Äpfingen(DE)**
Erfinder: **Entzeroth, Michael, Dr.**
**Sebastian-Sailer-Strasse 20**
**W-7951 Warthausen(DE)**

(54) **Phenylalkylderivate, diese Verbindungen enthaltende Arzneimittel und Verfahren zu ihrer Herstellung.**

(57) Die Erfindung betrifft Phenylalkylderivate der allgemeinen Formel

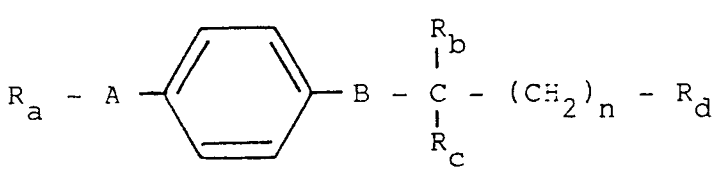

,(I)

in der
$R_a$ bis $R_d$, A, B und n wie im Anspruch 1 definiert sind, deren Isomerengemische, deren Tautomere, deren Enantiomere und deren Salze, welche wertvolle Eigenschaften aufweisen.

EP 0 529 253 A1

Die neuen Verbindungen stellen insbesondere Angiotensin-Antagonisten dar.

Gegenstand der vorliegenden Erfindung sind Phenylalkylderivate der allgemeinen Formel

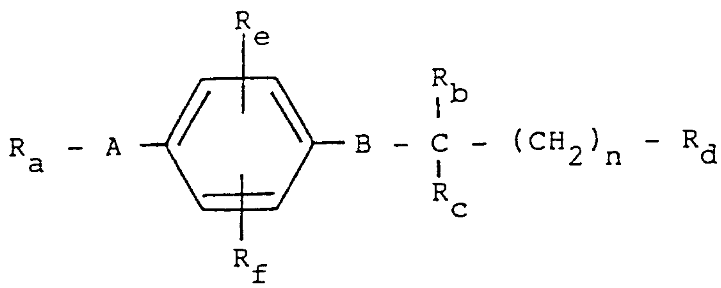

,(I)

und die Verbindung 5,7-Dimethyl-2-ethyl-3-[4-[α-(1H-tetrazol-5-yl)benzyloxy]-3,5-dichlorbenzyl]imidazo[4,5-b]pyridin, deren Isomerengemische, deren Tautomere, deren Enantiomere sowie deren Salze, insbesondere für die pharmazeutische Anwendung deren physiologisch verträglichen Salze mit anorganischen oder organischen Säuren oder Basen.

In der obigen allgemeinen Formel bedeutet

n die Zahl 0 oder 1,

A eine geradkettige oder verzweigte Alkylengruppe,

B ein Sauerstoffatom, eine Carbonyl-, Hydroxymethylen-, Sulfenyl-, Sulfinyl- oder Sulfonylgruppe, eine geradkettige oder verzweigte Alkylengruppe, eine Alkylidengruppe mit 2 bis 4 Kohlenstoffatomen, eine 1,1-Cycloalkylengruppe oder eine gegebenenfalls durch eine Alkylgruppe oder durch eine Alkanoylgruppe mit 1 bis 4 Kohlenstoffatomen substituierte Iminogruppe,

$R_a$ ein Chlor- oder Bromatom, eine Hydroxy-, Alkylsulfonyloxy-, Phenylsulfonyloxy- oder Phenylalkylsulfonyloxygruppe oder eine Gruppe der Formel

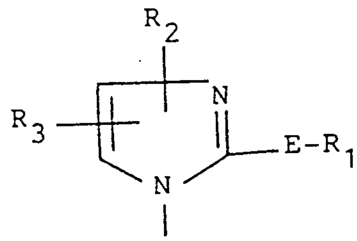

,

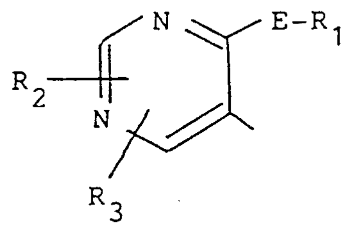

,

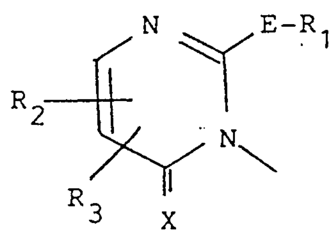

,

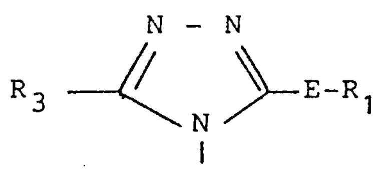

,

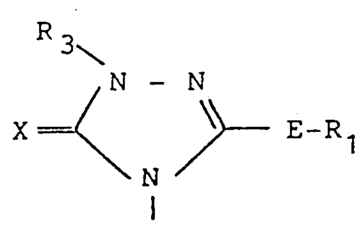

,

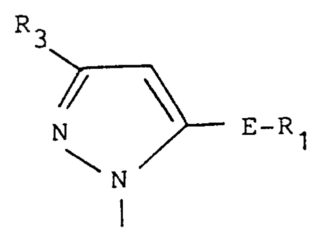

,

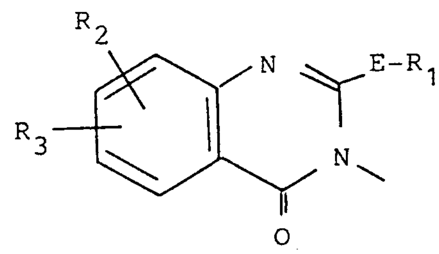

,

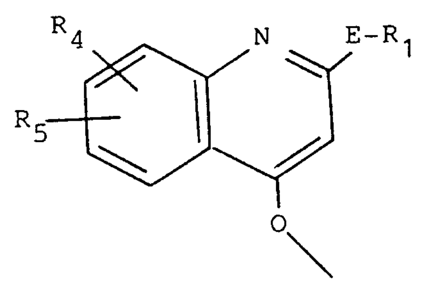

,

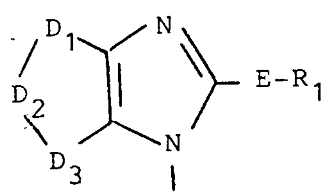

oder

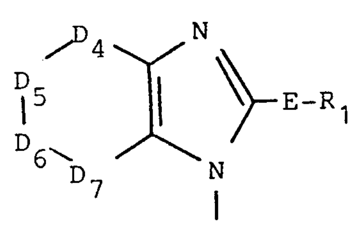

,

in denen einer der Reste $D_1$, $D_2$ oder $D_3$ eine Methylen- oder Iminogruppe und die verbleibenden Reste der Reste $D_1$ bis $D_3$ jeweils Methingruppen, wobei zusätzlich eine Methingruppe durch ein Stickstoffatom ersetzt sein kann und eine der Methingruppen durch den Rest $R_5$ und gegebenenfalls eine weitere der Methingruppen durch den Rest $R_4$ substituiert sein kann,

null, einer oder zwei der Reste $D_4$, $D_5$, $D_6$ oder $D_7$ ein Stickstoffatom und die verbleibenden Reste $D_4$, $D_5$,

$D_6$ oder $D_7$ jeweils Methingruppen, wobei zusätzlich eine Methingruppe durch den Rest $R_4$ und eine weitere Methingruppe durch den Rest $R_5$ substituiert sein kann,

E eine Kohlenstoff-Kohlenstoff-Bindung, ein Sauerstoff- oder Schwefelatom, eine Hydroxymethylen- oder Carbonylgruppe oder eine gegebenenfalls durch eine Alkylgruppe mit 1 bis 6 Kohlenstoffatomen, durch eine Cycloalkylgruppe, durch eine Alkanoylgruppe mit 2 bis 5 Kohlenstoffatomen, durch eine Allyl-, Phenyl- oder Benzylgruppe substituierte Iminogruppe,

X ein Sauerstoff- oder Schwefelatom oder eine gegebenfalls durch eine Alkyl-, Phenyl- oder Phenylalkylgruppe substituierte Iminogruppe,

$R_1$ eine geradkettige oder verzweigte Alkylgruppe mit 1 bis 9 Kohlenstoffatomen, eine geradkettige oder verzweigte Alkenyl- oder Alkinylgruppe mit jeweils 2 bis 6 Kohlenstoffatomen, wobei die vorstehend erwähnten gesättigten und ungesättigten Alkylteile jeweils durch eine Cycloalkylgruppe, durch ein Fluor-, Chlor- oder Bromatom, durch eine Hydroxy-, Amino-, Alkylamino-, Dialkylamino- oder $\alpha,\alpha$-Difluoroethangruppe substituiert sein können, eine Perfluoroalkylgruppe mit 1 bis 4 Kohlenstoffatomen oder eine Cycloalkylgruppe, die durch eine Trifluoromethylgruppe oder eine Alkylgruppe mono- oder disubstituiert sein kann,

$R_2$ ein Wasserstoff-, Fluor-, Chlor- oder Bromatom, eine Alkyl- oder Perfluoralkylgruppe mit jeweils 1 bis 5 Kohlenstoffatomen, eine Cyano- oder Nitrogruppe,

$R_3$ ein Wasserstoffatom, eine Cyanogruppe, eine gegebenfalls durch eine Hydroxy- oder Alkoxygruppe substituierte Alkylgruppe mit 1 bis 6 Kohlenstoffatomen, eine Perfluoralkylgruppe mit 1 bis 6 Kohlenstoffatomen, eine gegebenenfalls durch Fluoratome substituierte Alkenylgruppe mit 3 bis 6 Kohlenstoffatomen, eine Phenylalkyl- oder Phenylalkenylgruppe mit 2 bis 4 Kohlenstoffatomen im Alkenylteil, eine Alkylgruppe mit 1 bis 5 Kohlenstoffatomen, die endständig durch eine Imidazol-1-yl-, Triazolyl-, Tetrazolyl-, Phthalimido-, $R_6COO$-, $R_7S$-, $R_7SO$-, $R_7SO_2$-, $R_7CO$-, $R_7NHCOO$-, $R_7NHCO$-, $R_7NHCONR_7$-, $R_8CONR_7$- oder $R_8SO_2NR_7$-Gruppe substituiert ist, wobei

die Triazolylgruppe zusätzlich mit einer Acetoxy- oder Alkylgruppe mono- oder disubstituiert sein kann und

$R_6$ eine Alkyl- oder Perfluoralkylgruppe mit jeweils 1 bis 8 Kohlenstoffatomen, eine Cycloalkyl-, Phenyl-, Benzyl-, Phenylethyl-, Adamantyl-, Naphthyl-, Naphthylmethyl- oder Naphthylethylgruppe,

$R_7$ ein Wasserstoffatom und die für $R_6$ vorstehend erwähnten Bedeutungen besitzt,

$R_8$ die für $R_7$ vorstehend erwähnten Bedeutungen besitzt und eine gegebenenfalls in 4-Stellung durch eine Alkyl- oder Phenylgruppe substituierte Piperazinogruppe, eine Pyrrolidino-, Piperidino-, Hexamethylenimino-, Morpholino-, $R_7O$- oder $(R_7)_2N$-Gruppe darstellen,

$R_4$ ein Wasserstoff-, Fluor-, Chlor- oder Bromatom, eine Trifluormethylgruppe, eine Cycloalkylgruppe, eine gegebenenfalls durch eine Cycloalkylgruppe, durch eine Hydroxy-, Alkoxy-, Alkylamino-, Dialkylamino-, Alkoxycarbonyl-, Alkylaminocarbonyl- oder Dialkylaminocarbonylgruppe substituierte Alkylgruppe mit 1 bis 6 Kohlenstoffatomen und

$R_5$ ein Wasserstoff-, Fluor-, Chlor- oder Bromatom,

eine geradkettige oder verzweigte Alkyl- oder Perfluoralkylgruppe mit jeweils 1 bis 6 Kohlenstoffatomen, eine Alkenyl- oder Alkinylgruppe mit jeweils 2 bis 6 Kohlenstoffatomen, wobei die vorstehend erwähnten Alkyl- und Alkenylteile jeweils durch eine Heteroaryl-, Hydroxy-, Alkoxy-, Amino-, Alkylamino-, Dialkylamino-, Alkylcarbonylamino-, N-Alkyl-alkylcarbonylamino-, Carboxy-, Alkoxycarbonyl-, Alkylcarbonyloxy-, Piperidinocarbonyl-, Morpholinocarbonyl-, Aminocarbonyl-, Alkylaminocarbonyl-, Dialkylaminocarbonyl-, Tetrazol-5-yl-, Tetrazol-5-yl-aminocarbonyl-, Alkylsulfenyl-, Alkylsulfinyl-, Alkylsulfonyl-, Aminosulfonyl-, Alkylaminosulfonyl-, Dialkylaminosulfonyl-, Alkylsulfonylaminocarbonyl-, Heteroarylaminosulfonyl- oder Alkylcarbonylaminosulfonylgruppe mono- oder disubstituiert sein können,

eine Alkoxygruppe mit 1 bis 7 Kohlenstoffatomen, die in 2-, 3-, 4-, 5-, 6- oder 7-Position durch eine Imidazolyl-, Tetrazolyl-, Benzimidazolyl- oder Tetrahydrobenzimidazolylgruppe substituiert ist, oder eine gegebenenfalls im Alkoxyteil durch eine 1H-Tetrazol-5-yl- oder 1-Triphenylmethyl-tetrazol-5-yl-gruppe substituierte Phenylalkoxygruppe,

eine Carboxygruppe oder eine Gruppe, die in vivo metabolisch in eine Carboxygruppe umgewandelt wird,

eine Alkylsulfonyloxygruppe mit 1 bis 4 Kohlenstoffatomen, eine Benzolsulfonyloxy- oder Phenylalkansulfonyloxygruppe,

eine gegebenenfalls am Stickstoffatom durch eine Alkylgruppe mit 1 bis 6 Kohlenstoffatomen, durch eine Phenyl-, Cycloalkyl-, Phenylalkyl-, Cycloalkylalkyl-, Bicyclohexyl- oder Biphenylgruppe substituierte Acylaminogruppe, in welcher der Acylrest eine Alkanoylgruppe mit 1 bis 7 Kohlenstoffatomen, eine Alkoxycarbonylgruppe mit insgesamt 2 bis 4 Kohlenstoffatomen, eine Alkylsulfonylgruppe mit 1 bis 6 Kohlenstoffatomen, eine Benzoyl-, Benzolsulfonyl-, Phenylalkansulfonyl-, Naphthalinsulfonyl-, Cycloalkylcarbonyl-, Phenylalkanoyl- oder Cycloalkylalkanoylgruppe darstellt, wobei die vorstehend erwähnten Phenylkerne

jeweils durch ein Fluor-, Chlor- oder Bromatom, durch eine Methyl- oder Methoxygruppe mono- oder disubstituiert und die Substituenten gleich oder verschieden sein können,
eine Phthalimino-, Homophthalimino-, 2-Carboxyphenylcarbonylamino- oder 2-Carboxyphenylmethylaminogruppe, wobei eine Carbonylgruppe in einer Phthaliminogruppe durch eine Methylen-, Alkyl-methylen- oder Dialkyl-methylengruppe ersetzt sowie eine Methylengruppe in einer Homophthaliminogruppe durch eine oder zwei Alkylgruppen substituiert sein kann, und zusätzlich die vorstehend erwähnten Phenylkerne durch Alkyl- oder Alkoxygruppen mono- oder disubstituiert und gleichzeitig ganz oder teilweise hydriert sein können, wobei die Substituenten gleich oder verschieden sein können,
eine gegebenenfalls durch eine oder zwei Alkylgruppen oder durch eine Tetramethylen- oder Pentamethylengruppe substituierte 5-, 6- oder 7-gliedrige Alkylenimino- oder Alkenyleniminogruppe, in welcher eine Methylengruppe durch eine Carbonyl- oder Sulfonylgruppe ersetzt sein kann,
eine Bicycloalkan-2,3-dicarbonsäureimino- oder Bicycloalken-2,3-dicarbonsäureiminogruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann,
eine Glutarsäureiminogruppe, in der die n-Propylengruppe perfluoriert, durch ein oder zwei Alkylgruppen oder durch eine Tetramethylen- oder Pentamethylengruppe substituiert sein kann,
eine gegebenenfalls durch eine Alkyl- oder Phenylgruppe mono- oder disubstituierte Maleinsäureimido- oder Succinimidogruppe, wobei die Substituenten gleich oder verschieden sein können,
einen über ein Kohlenstoffatom oder über eine Iminogruppe gebundenen 5-gliedrigen heteroaromatischen Ring, der eine Iminogruppe, ein Sauerstoff- oder Schwefelatom oder eine Iminogruppe und ein Sauerstoff-, Schwefel- oder Stickstoffatom enthält, oder einen über ein Kohlenstoffatom gebundenen 6-gliedrigen heteroaromatischen Ring, der 1 oder 2 Stickstoffatome enthält, wobei sowohl an die 5-gliedrigen als auch an die 6-gliedrigen heteroaromatischen Ringe jeweils über zwei benachbarte Kohlenstoffatome eine n-Propylen-, n-Butylen- oder 1,3-Butadienylgruppe oder über eine Iminogruppe und ein benachbartes Kohlenstoffatom eine n-Propylen-, n-Butylen- oder 1,3-Butadienylgruppe angefügt ist und in einem so gebildeten anellierten Pyridinring eine Methingruppe durch ein Stickstoffatom und eine Vinylengruppe in 3-, 4-Stellung zu dem Stickstoffatom des gebildeten Pyridinringes durch ein Schwefelatom oder in einem so gebildeten anellierten Phenylring eine oder zwei Methingruppen durch N-Atome ersetzt sein können, wobei zusätzlich die vorstehend erwähnten ankondensierten aromatischen oder heteroaromatischen Ringe im Kohlenstoffgerüst durch ein Fluor-, Chlor- oder Bromatom, durch eine Alkyl-, Alkoxy-, Hydroxy-, Phenyl-, Nitro-, Amino-, Alkylamino-, Dialkylamino-, Alkanoylamino-, Cyano-, Carboxy-, Alkoxycarbonyl-, Aminocarbonyl-, Alkylaminocarbonyl-, Dialkylaminocarbonyl-, Trifluormethyl-, Alkanoyl-, Aminosulfonyl-, Alkylaminosulfonyl- oder Dialkylaminosulfonylgruppe monosubstituiert oder durch Fluor- oder Chloratome, durch Methyl-, Methoxy- oder Hydroxygruppen disubstituiert sein können und eine gegebenenfalls in einem Imidazolring vorhandene NH-Gruppe durch eine Alkylgruppe mit 1 bis 6 Kohlenstoffatomen oder durch eine Cycloalkylgruppe substituiert sein kann,
einen über ein Kohlenstoffatom gebundenen Pyrrolidin-, Piperidin- oder Pyridinring, wobei an den Pyridinring über zwei benachbarte Kohlenstoffatome ein Phenylrest ankondensiert und eine zum N-Atom benachbarte Methylengruppe in einem Pyrrolidin- oder Piperidinring durch eine Carbonylgruppe ersetzt sein kann,
eine gegebenenfalls durch eine Alkyl-, Phenylalkyl-, Tetramethylen-, Pentamethylen- oder Hexamethylengruppe substituierte Imidazolidindiongruppe,
eine Pyridazin-3-on- oder Dihydro-pyridazin-3-on-gruppe, die in 2-Stellung durch eine gegebenenfalls durch eine Phenylgruppe substituierte Alkylgruppe und im Kohlenstoffgerüst zusätzlich durch 1 oder 2 Alkylgruppen substituiert sein kann, oder
eine $R_{11}$-$NR_{10}$-CO-$NR_9$-Gruppe, in der

$R_9$ ein Wasserstoffatom, eine Alkylgruppe mit 1 bis 8 Kohlenstoffatomen oder Phenylalkylgruppe,

$R_{10}$ ein Wasserstoffatom, eine Alkylgruppe mit 1 bis 8 Kohlenstoffatomen, eine Alkenylgruppe mit 3 bis 5 Kohlenstoffatomen, eine Phenylalkylgruppe oder eine Cycloalkylgruppe mit 5 bis 7 Kohlenstoffatomen,

$R_{11}$ ein Wasserstoffatom oder eine Alkylgruppe mit 1 bis 6 Kohlenstoffatomen oder

einer der Reste $R_9$, $R_{10}$ oder $R_{11}$ auch eine Bicyclohexyl- oder Biphenylylgruppe oder

$R_{10}$ und $R_{11}$ zusammen mit dem dazwischen liegenden Stickstoffatom eine geradkettige Alkyleniminogruppe mit 4 bis 6 Kohlenstoffatomen oder eine Morpholinogruppe oder

$R_9$ und $R_{11}$ zusammen eine Alkylengruppe mit 2 bis 4 Kohlenstoffatomen darstellen,

$R_b$ eine Cyano-, Trifluormethylcarbonylamino-, Trifluormethylcarbonylaminomethyl-, Trifluormethylsulfonylamino-, Trifluormethylsulfonylaminomethyl-, Alkylsulfonylamino-, Alkylsulfonylaminomethyl-, Arylsulfonylamino-, Arylsulfonylaminomethyl-, Aralkylsulfonylamino-, Aralkylsulfonylaminomethyl-, Arylsulfonylaminocarbonyl-, Benzylsulfonylaminocarbonyl-, Sulfo-, Aminosulfonyl-, Alkylaminosulfonyl-, Aralkylaminosulfonyl-, Arylaminosulfonyl-, Alkylcarbonylaminosulfonyl-,

Aralkylcarbonylaminosulfonyl-,Arylcarbonylaminosulfonyl-, Sulfomethyl-, Aminosulfonylmethyl-, Alkylaminosulfonylmethyl-, Aralkylaminosulfonylaminomethyl-, Arylaminosulfonylmethyl-, Alkylcarbonylaminosulfonylmethyl-, Aralkylcarbonylaminosulfonylmethyl-, Arylcarbonylaminosulfonylmethyl-, Phosphino-, O-Alkyl-phosphino-, O-Aralkyl-phosphino-, O-Aryl-phosphino-, Phosphono-, O-Alkyl-phosphono-, O-Aralkyl-phosphono-, O-Arylphosphono-, O,O-Dialkylphosphono-, Phosphono-methyl-, O-Alkyl-phosphono-methyl-, O-Aralkyl-phosphono-methyl-, O-Arylphosphono-methyl-, O,O-Dialkylphosphono-methyl-, Phosphato-, O-Alkyl-phosphato-, O-Aralkyl-phosphato-, O-Aryl-phosphato- oder O,O-Dialkoxy-phosphorylgruppe, eine gegebenenfalls durch eine Alkyl-, Trifluormethyl-, Phenylalkyl- oder Triphenylmethylgruppe substituierte 1H-Tetrazolyl-, 1H-Tetrazolylalkyl-, 1H-Tetrazolylaminocarbonyl- oder Triazolylgruppe, eine Alkylsulfonylaminocarbonyl- oder Perfluoralkylsulfonylaminocarbonylgruppe mit jeweils 1 bis 6 Kohlenstoffatomen im Alkylteil, eine Carboxygruppe, eine Gruppe, die in vivo metabolisch in eine Carboxygruppe übergeführt wird, oder eine Aralkoxycarbonylgruppe,

$R_c$ ein Wasserstoffatom, eine Alkyl-, Aralkyl-, Aryl-, Carboxy- oder Alkoxycarbonylgruppe,

$R_d$ eine geradkettige oder verzweigte Alkylkette mit 1 bis 10 Kohlenstoffatomen, eine geradkettige oder verzweigte Alkenyl- oder Alkinylgruppe mit jeweils 2 bis 10 Kohlenstoffatomen, eine Cycloalkyl- oder Cycloalkylalkylgruppe, eine gegebenenfalls durch Fluor-, Chlor- oder Bromatome, durch Methyl- oder Methoxygruppen mono- oder disubstituierte Phenylgruppe, eine Biphenyl-, Naphthyl- oder Heteroarylgruppe,

$R_e$ und $R_f$ Wasserstoffatome und, wenn

$R_5$ eine Phthalimino-, Homophthalimino-, 2-Carboxyphenylcarbonylamino- oder 2-Carboxyphenylmethylaminogruppe, wobei eine Carbonylgruppe in einer Phthaliminogruppe durch eine Methylen-, Alkyl-methylen- oder Dialkyl-methylengruppe ersetzt sowie eine Methylengruppe in einer Homophthaliminogruppe durch eine oder zwei Alkylgruppen substituiert sein kann, und zusätzlich die vorstehend erwähnten Phenylkerne durch Alkyl- oder Alkoxygruppen mono- oder disubstituiert und gleichzeitig ganz oder teilweise hydriert sein können, wobei die Substituenten gleich oder verschieden sein können,

eine Carboxygruppe oder eine Gruppe, die in vivo metabolisch in eine Carboxygruppe umgewandelt wird,

eine gegebenenfalls durch eine oder zwei Alkylgruppen oder durch eine Tetramethylen- oder Pentamethylengruppe substituierte 5-, 6- oder 7-gliedrige Alkylenimino- oder Alkenyleniminogruppe, in welcher eine Methylengruppe durch eine Carbonyl- oder Sulfonylgruppe ersetzt sein kann,

eine Bicycloalkan-2,3-dicarbonsäureimino- oder Bicycloalken-2,3-dicarbonsäureiminogruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann,

eine Glutarsäureiminogruppe, in der die n-Propylengruppe perfluoriert, durch ein oder zwei Alkylgruppen oder durch eine Tetramethylen- oder Pentamethylengruppe substituiert sein kann,

eine gegebenenfalls durch eine Alkyl- oder Phenylgruppe mono- oder disubstituierte Maleinsäureimido- oder Succinimidogruppe, wobei die Substituenten gleich oder verschieden sein können,

einen über ein Kohlenstoffatom oder über eine Iminogruppe gebundenen 5-gliedrigen heteroaromatischen Ring, der eine Iminogruppe, ein Sauerstoff- oder Schwefelatom oder eine Iminogruppe und ein Sauerstoff-, Schwefel- oder Stickstoffatom enthält, oder einen über ein Kohlenstoffatom gebundenen 6-gliedrigen heteroaromatischen Ring, der 1 oder 2 Stickstoffatome enthält, wobei sowohl an die 5-gliedrigen als auch an die 6-gliedrigen heteroaromatischen Ringe jeweils über zwei benachbarte Kohlenstoffatome eine n-Propylen-, n-Butylen- oder 1,3-Butadienylgruppe oder über eine Iminogruppe und ein benachbartes Kohlenstoffatom eine n-Propylen-, n-Butylen- oder 1,3-Butadienylgruppe angefügt ist und in einem so gebildeten anellierten Pyridinring eine Methingruppe durch ein Stickstoffatom und eine Vinylengruppe in 3-, 4-Stellung zu dem Stickstoffatom des gebildeten Pyridinringes durch ein Schwefelatom oder in einem so gebildeten anellierten Phenylring eine oder zwei Methingruppen durch N-Atome ersetzt sein können, wobei zusätzlich die vorstehend erwähnten ankondensierten aromatischen oder heteroaromatischen Ringe im Kohlenstoffgerüst durch ein Fluor-, Chlor- oder Bromatom, durch eine Alkyl-, Alkoxy-, Hydroxy-, Phenyl-, Nitro-, Amino-, Alkylamino-, Dialkylamino-, Alkanoylamino-, Cyano-, Carboxy-, Alkoxycarbonyl-, Aminocarbonyl-, Alkylaminocarbonyl-, Dialkylaminocarbonyl-, Trifluormethyl-, Alkanoyl-, Aminosulfonyl-, Alkylaminosulfonyl- oder Dialkylaminosulfonylgruppe monosubstituiert oder durch Fluor- oder Chloratome, durch Methyl-, Methoxy- oder Hydroxygruppen disubstituiert sein können und eine gegebenenfalls in einem Imidazolring vorhandene NH-Gruppe durch eine Alkylgruppe mit 1 bis 6 Kohlenstoffatomen oder durch eine Cycloalkylgruppe substituiert sein kann, oder

einen über ein Kohlenstoffatom gebundenen Pyrrolidin-, Piperidin- oder Pyridinring, wobei an den Pyridinring über zwei benachbarte Kohlenstoffatome ein Phenylrest ankondensiert und eine zum N-Atom benachbarte Methylengruppe in einem Pyrrolidin- oder Piperidinring durch eine Carbonylgruppe ersetzt sein kann,

eine gegebenenfalls durch eine Alkyl-, Phenylalkyl-, Tetramethylen-, Pentamethylen- oder Hexamethylengruppe substituierte Imidazolidindiongruppe,
eine Pyridazin-3-on- oder Dihydro-pyridazin-3-on-gruppe, die in 2-Stellung durch eine gegebenenfalls durch eine Phenylgruppe substituierte Alkylgruppe und im Kohlenstoffgerüst zusätzlich durch 1 oder 2 Alkylgruppen substituiert sein kann, oder
eine $R_{11}$-$NR_{10}$-CO-$NR_9$-Gruppe, in der $R_9$, $R_{10}$ und $R_{11}$ wie vorstehend erwähnt definiert sind, darstellt,
auch $R_e$ ein Wasserstoff-, Fluor-, Chlor- oder Bromatom, eine Alkyl- oder Alkoxygruppe und
$R_f$ ein Fluor-, Chlor- oder Bromatom oder eine Alkoxygruppe,
insbesondere diejenigen Verbindungen, in denen $R_a$ bis $R_f$, A, B und n mit der Maßgabe wie vorstehend erwähnt definiert sind, daß
$D_4$ bis $D_7$ mit den zusätzlichen Maßgaben (a) bis (j) Methingruppen darstellen oder
$D_7$ ein Stickstoffatom und die Reste $D_4$, $D_5$ und $D_6$ mit den zusätzlichen Maßgaben (a) bis (g) und (k) bis (m) oder (a) bis (g) und (n) bis (p) Methingruppen darstellen oder
einer der Reste $D_4$, $D_5$ oder $D_6$ ein Stickstoffatom und die verbleibenden Reste der Reste $D_4$ bis $D_6$ sowie $D_7$ Methingruppen darstellen oder
zwei der Reste $D_4$ bis $D_7$ Stickstoffatome und die verbleibenden Reste der Reste $D_4$ bis $D_7$ Methingruppen darstellen,
mit den zusätzlichen Maßgaben, daß entweder

(a) n die Zahl 1 darstellt oder
(b) E mit Ausnahme der Kohlenstoff-Kohlenstoff-Bindung die für E vorstehend erwähnten Bedeutungen besitzt oder
(c) A mit Ausnahme der Methylengruppe die für A eingangs erwähnten Bedeutungen besitzt oder
(d) B mit Ausnahme des Sauerstoffatoms die für B vorstehend erwähnten Bedeutungen besitzt oder
(e) $R_b$ mit Ausnahme der Carboxylgruppe die für $R_b$ vorstehend erwähnten Bedeutungen besitzt oder
(f) $R_c$ mit Ausnahme des Wasserstoffatoms die für $R_c$ vorstehend erwähnten Bedeutungen besitzt oder
(g) $R_d$ mit Ausnahme der Phenylgruppe die für $R_d$ vorstehend erwähnten Bedeutungen besitzt oder
(h) $R_1$ mit Ausnahme der n-Butylgruppe die für $R_1$ vorstehend erwähnten Bedeutungen besitzt oder
(i) $R_4$ mit Ausnahme der Methylgruppe in Position 7 die für $R_4$ vorstehend erwähnten Bedeutungen besitzt oder
(j) $R_5$ mit Ausnahme des Wasserstoffatoms die für $R_5$ vorstehend erwähnten Bedeutungen besitzt, oder, daß,
(k) $R_1$ mit Ausnahme der Ethylgruppe die für $R_1$ vorstehend erwähnten Bedeutungen besitzt oder
(l) $R_4$ mit Ausnahme der Methylgruppe in Position 7 die für $R_4$ vorstehend erwähnten Bedeutungen besitzt oder
(m) $R_5$ mit Ausnahme der Methylgruppe in Position 5 die für $R_5$ vorstehend erwähnten Bedeutungen besitzt oder
(n) $R_1$ mit Ausnahme der n-Propylgruppe für $R_1$ vorstehenderwähnten Bedeutungen besitzt oder
(o) $R_4$ mit Ausnahme des Wasserstoffatoms die für $R_4$ vorstehend erwähnten Bedeutungen besitzt oder
(p) $R_5$ mit Ausnahme des Wasserstoffatoms die für $R_5$ vorstehend erwähnten Bedeutungen besitzt und

die übrigen Reste die vorstehend erwähnten Bedeutungen besitzen,
wobei zusätzlich eine bei den vorstehend erwähnten Definitionen der Reste $D_4$ bis $D_7$ erwähnte Methingruppe durch den Rest $R_4$ und eine weitere bei den vorstehend erwähnten Definitionen der Reste $D_4$ bis $D_7$ erwähnte Methingruppe durch den Rest $R_5$ substituiert sein kann,
wobei, soweit nichts anderes erwähnt wurde,
die vorstehend erwähnten Alkyl-, Alkylen- oder Alkoxyteile jeweils 1 bis 4 Kohlenstoffatome und die Cycloalkylteile jeweils 3 bis 7 Kohlenstoffatome enthalten können, sowie
unter "eine Arylgruppe" eine gegebenenfalls durch ein Fluor-, Chlor- oder Bromatom, durch eine Hydroxy-, Alkyl-, Alkoxy-, Phenylalkoxy-, Phenyl-, Nitro-, Amino-, Alkylamino-, Dialkylamino-, Alkanoylamino-, Cyano-, Carboxy-, Alkoxycarbonyl-, Aminocarbonyl-, Alkylaminocarbonyl-, Dialkylaminocarbonyl-, Trifluormethyl-, Alkanoyl-, Aminosulfonyl-, Alkylaminosulfonyl- oder Dialkylaminosulfonylgruppe mono- oder disubstituierte Phenylgruppe, wobei der Alkylteil jeweils 1 bis 4 Kohlenstoffatome enthalten kann, oder eine Naphthylgruppe,
unter "eine Heteroarylgruppe" ein 5-gliedriger heteroaromatischer Ring, der eine Iminogruppe, ein Sauerstoff- oder Schwefelatom oder eine Iminogruppe und ein Sauerstoff-, Schwefel- oder Stickstoffatom oder eine Iminogruppe und 2 oder 3 Stickstoffatome enthält, und ein 6-gliedriger heteroaromatischer Ring, der 1, 2 oder 3 Stickstoffatome enthält, wobei die vorstehend erwähnten Ringe zusätzlich durch ein Fluor-, Chlor- oder Bromatom, durch eine Alkyl-, Alkoxy-, Hydroxy-, Phenyl-, Nitro-, Amino-, Alkylamino-, Dialkylamino-, Alkanoylamino-, Cyano-, Carboxy-, Alkoxycarbonyl-, Aminocarbonyl-, Alkylaminocarbonyl-, Dialkylaminocarbonyl-, Trifluormethyl-, Alkanoyl-, Aminosulfonyl-, Alkylaminosulfonyl- oder Dialkylaminosulfonylgruppe mono- oder disubstituiert sein können, und
unter dem vorstehend erwähnten Begriff "eine Gruppe, die in vivo metabolisch in eine Carboxygruppe umgewandelt wird," beispielsweise deren Ester der Formeln

- CO - OR',
- CO - O - (HCR'') - O - CO - R''' und
- CO - O - (HCR'') - O - CO - OR '''

in denen
R' eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Pivaloyloxymethyl-, Phthalidylmethyl-, (1,3-Dioxa-2-oxo-4-methyl-cyclopenten-5-yl)-methyl-, Methoxymethyl- oder Cinnamylgruppe,
R'' ein Wasserstoffatom oder eine Methylgruppe und
R''' eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe bedeuten, zu verstehen ist.

Die neuen Verbindungen der obigen allgemeinen Formel I, in denen $R_a$ ein Chlor- oder Bromatom, eine Hydroxy-, Alkylsulfonyloxy-, Phenylsulfonyloxy- oder Phenylalkylsulfonyloxygruppe darstellt, stellen wertvolle Zwischenprodukte dar und die übrigen Verbindungen der obigen allgemeinen Formel I sowie deren physiologisch verträglichen Salze weisen wertvolle pharmakologische Eigenschaften auf, da diese Angiotensin-Antagonisten, insbesondere Angiotensin-II-Antagonisten, darstellen.

Gegenstand der vorliegenden Erfindung sind somit die neuen vorstehend erwähnten Phenylalkylderivate, wobei die entsprechenden O-substituierten Phosphono- oder Phosphato-Verbindungen sowie die entsprechenden Cyano-, Alkoxycarbonyl- oder Triphenylmethylverbindungen zugleich auch wertvolle Zwischenprodukte darstellen.

Ein weiterer Gegenstand der vorliegenden Erfindung sind somit neue Arzneimittel, welche eine der oben erwähnten pharmakologisch wirksamen Verbindungen der allgemeinen Formel I oder ein entsprechendes physiologisch verträgliches Additionssalz enthalten und insbesondere zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen geeignet sind.

Für die bei der Definition durch die Reste $D_4$, $D_5$, $D_6$ und $D_7$ eingangs erwähnten heteroaromatischen Reste kommt die Pyrido-, Pyrimido-, Pyrazino- oder Pyridazinogruppe, welche im Kohlenstoffgerüst durch die Reste $R_4$ und $R_5$ substituiert sein können, in Betracht.

Bevorzugte Verbindungen der obigen allgemeinen Formel I sind mit Ausnahme von
(i) 2-n-Butyl-1-[4-[(α-carboxy)benzyloxy]benzyl]benzimidazol,
(ii) 2-n-Propyl-7-methyl-3-[4-[(α-carboxy)benzyloxy]benzyl]imidazo[4,5-b]pyridin und
(iii) 5,7-Dimethyl-2-ethyl-3-[4-[(α-carboxy)benzyloxy]benzyl]imidazo[4,5-b]pyridin
diejenigen, in denen
n die Zahl 0 oder 1,
A eine geradkettige oder verzweigte Alkylengruppe,
B ein Sauerstoffatom, eine geradkettige oder verzweigte Alkylengruppe mit 1 bis 3 Kohlenstoffatomen oder eine gegebenenfalls durch eine Alkylgruppe oder durch eine Alkanoylgruppe mit 1 bis 4 Kohlenstoffatomen substituierte Iminogruppe,
$R_a$ eine Gruppe der Formel

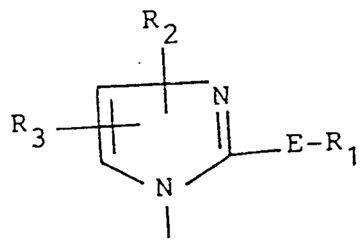

,

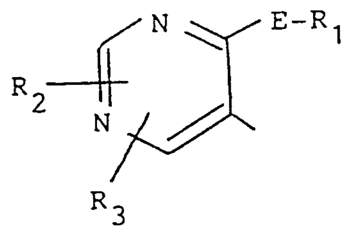

,

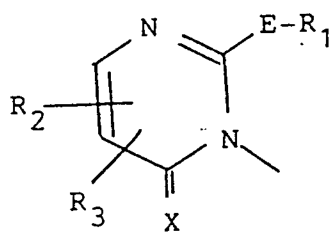

,

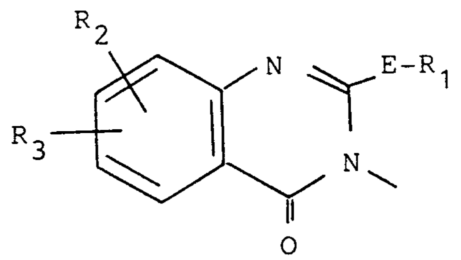

,

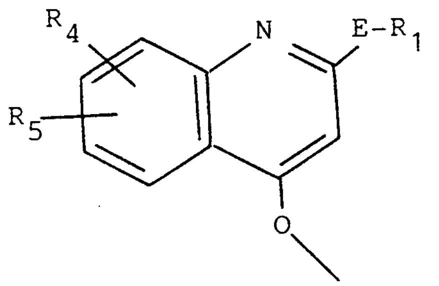

oder

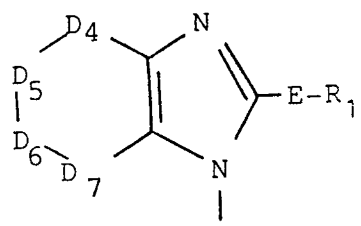

,

in denen null, einer oder zwei der Reste $D_4$, $D_5$, $D_6$ oder $D_7$ ein Stickstoffatom und die verbleibenden Reste $D_4$, $D_5$, $D_6$ oder $D_7$ jeweils Methingruppen, wobei zusätzlich eine Methingruppe durch den Rest $R_4$ und eine weitere Methingruppe durch den Rest $R_5$ substituiert sein kann,

E eine Kohlenstoff-Kohlenstoff-Bindung, ein Sauerstoff- oder Schwefelatom oder eine gegebenenfalls durch eine Alkylgruppe mit 1 bis 4 Kohlenstoffatomen substituierte Iminogruppe,

X ein Sauerstoff- oder Schwefelatom,

$R_1$ eine geradkettige oder verzweigte Alkylgruppe mit 1 bis 6 Kohlenstoffatomen, eine geradkettige oder verzweigte Alkenyl- oder Alkinylgruppe mit jeweils 2 bis 6 Kohlenstoffatomen, wobei die vorstehend erwähnten gesättigten und ungesättigten Alkylteile jeweils durch ein Fluor-, Chlor- oder Bromatom, durch eine Hydroxy- oder Aminogruppe substituiert sein können, oder eine Cycloalkylgruppe mit 3 bis 6 Kohlenstoffatomen,

$R_2$ ein Wasserstoff-, Fluor-, Chlor- oder Bromatom, eine Alkyl- oder Perfluoralkylgruppe mit jeweils 1 bis 5 Kohlenstoffatomen, eine Cyano- oder Nitrogruppe,

$R_3$ ein Wasserstoffatom, eine Cyanogruppe, eine gegebenfalls durch eine Hydroxy- oder Alkoxygruppe substituierte Alkylgruppe mit 1 bis 3 Kohlenstoffatomen, eine Perfluoralkylgruppe mit 1 bis 3 Kohlenstoffatomen, eine gegebenenfalls durch Fluoratome substituierte Alkenylgruppe mit 3 bis 6 Kohlenstoffatomen, eine Alkylgruppe mit 1 bis 3 Kohlenstoffatomen, die endständig durch eine $R_6COO$-, $R_7S$-, $R_7SO$-, $R_7SO_2$-, $R_7CO$-, $R_7NHCOO$-, $R_7NHCO$-, $R_7NHCONR_7$-, $R_8CONR_7$- oder $R_8SO_2NR_7$-Gruppe substituiert ist, wobei

$R_6$ eine Alkyl- oder Perfluoralkylgruppe mit jeweils 1 bis 4 Kohlenstoffatomen, eine Cycloalkyl-, Phenyl-, Benzyl- oder Phenylethylgruppe,

$R_7$ ein Wasserstoffatom und die für $R_6$ vorstehend erwähnten Bedeutungen besitzt,

$R_8$ die für $R_7$ vorstehend erwähnten Bedeutungen besitzt, darstellen,

$R_4$ ein Wasserstoff-, Fluor-, Chlor- oder Bromatom, eine Trifluormethylgruppe, eine Cycloalkylgruppe, eine gegebenenfalls durch eine Hydroxy-, Alkoxy- oder Alkoxycarbonylgruppe substituierte Alkylgruppe mit 1 bis 6 Kohlenstoffatomen und

$R_5$ ein Wasserstoff-, Fluor-, Chlor- oder Bromatom,

eine geradkettige oder verzweigte Alkyl- oder Perfluoralkylgruppe mit jeweils 1 bis 6 Kohlenstoffatomen, eine Alkenyl- oder Alkinylgruppe mit jeweils 2 bis 6 Kohlenstoffatomen, wobei die vorstehend erwähnten Alkyl- und Alkenylteile jeweils durch eine Heteroaryl-, Hydroxy-, Alkoxy-, Amino-, Alkylamino-, Dialkylamino-, Alkylcarbonylamino-, N-Alkyl-alkylcarbonylamino-, Carboxy-, Alkoxycarbonyl-, Alkylcarbonyloxy-, Aminocarbonyl-, Alkylaminocarbonyl-, Dialkylaminocarbonyl- oder Tetrazol-5-yl-gruppe mono- oder disubstituiert sein können,

eine Alkoxygruppe mit 1 bis 5 Kohlenstoffatomen oder eine α-(1H-Tetrazol-5-yl)-benzyloxygruppe,

eine Carboxygruppe oder eine Gruppe, die in vivo metabolisch in eine Carboxygruppe umgewandelt wird,

eine Alkylsulfonyloxygruppe mit 1 bis 4 Kohlenstoffatomen, eine Benzolsulfonyloxy- oder Phenylalkansulfonyloxygruppe,

eine gegebenenfalls am Stickstoffatom durch eine Alkylgruppe mit 1 bis 6 Kohlenstoffatomen, durch eine Phenyl-, Cycloalkyl-, Phenylalkyl- oder Cycloalkylalkylgruppe substituierte Acylaminogruppe, in welcher der Acylrest eine Alkanoylgruppe mit 1 bis 5 Kohlenstoffatomen, eine Alkoxycarbonylgruppe mit insgesamt 2 bis 4 Kohlenstoffatomen, eine Alkylsulfonylgruppe mit 1 bis 6 Kohlenstoffatomen, eine Benzoyl-, Benzolsulfonyl-, Cycloalkylcarbonyl-, Phenylalkanoyl- oder Cycloalkylalkanoylgruppe darstellt, wobei die vorstehend erwähnten Phenylkerne jeweils durch ein Fluor-, Chlor- oder Bromatom, durch eine Methyl- oder Methoxygruppe mono- oder disubstituiert und die Substituenten gleich oder verschieden sein können,

eine Phthalimino- oder Homophthaliminogruppe, wobei eine Carbonylgruppe in einer Phthaliminogruppe durch eine Methylen-, Alkyl-methylen- oder Dialkyl-methylengruppe ersetzt sowie eine Methylengruppe in einer Homophthaliminogruppe durch eine oder zwei Alkylgruppen substituiert sein kann, und zusätzlich die vorstehend erwähnten Phenylkerne durch Alkyl- oder Alkoxygruppen mono- oder disubstituiert und gleichzeitig ganz oder teilweise hydriert sein können, wobei die Substituenten gleich oder verschieden sein können,

eine gegebenenfalls durch eine oder zwei Alkylgruppen oder durch eine Tetramethylen- oder Pentamethylengruppe substituierte 5-, 6- oder 7-gliedrige Alkyleniminogruppe, in welcher eine Methylengruppe durch eine Carbonyl- oder Sulfonylgruppe ersetzt sein kann,

eine gegebenenfalls durch eine Alkyl- oder Phenylgruppe mono- oder disubstituierte Maleinsäureimido- oder Succinimidogruppe, wobei die Substituenten gleich oder verschieden sein können,

einen über ein Kohlenstoffatom oder über eine Iminogruppe gebundenen 5-gliedrigen heteroaromatischen Ring, der eine Iminogruppe, ein Sauerstoff- oder Schwefelatom oder eine Iminogruppe und ein Sauerstoff-, Schwefel- oder Stickstoffatom enthält, oder einen über ein Kohlenstoffatom gebundenen 6-gliedrigen heteroaromatischen Ring, der 1 oder 2 Stickstoffatome enthält, wobei sowohl an die 5-gliedrigen als auch an die 6-gliedrigen heteroaromatischen Ringe jeweils über zwei benachbarte Kohlenstoffatome eine n-Propylen-, n-Butylen- oder 1,3-Butadienylgruppe oder über eine Iminogruppe und ein benachbartes Kohlenstoffatom eine n-Propylen-, n-Butylen- oder 1,3-Butadienylgruppe angefügt ist und in einem so gebildeten anellierten Pyridinring eine Methingruppe durch ein Stickstoffatom und eine Vinylengruppe in 3-, 4-Stellung zu dem Stickstoffatom des gebildeten Pyridinringes durch ein Schwefelatom oder in einem so gebildeten anellierten Phenylring eine oder zwei Methingruppen durch N-Atome ersetzt sein können, wobei zusätzlich die vorstehend erwähnten ankondensierten aromatischen oder heteroaromatischen Ringe im Kohlenstoffgerüst durch ein Fluor-, Chlor- oder Bromatom, durch eine Alkyl-, Alkoxy-, Hydroxy-, Phenyl-, Nitro-, Amino-, Alkylamino-, Dialkylamino-, Alkanoylamino-, Cyano-, Carboxy-, Alkoxycarbonyl-, Aminocarbonyl-, Alkylaminocarbonyl-, Dialkylaminocarbonyl-, Trifluormethyl-, Alkanoyl-, Aminosulfonyl-, Alkylaminosulfonyl- oder Dialkylaminosulfonylgruppe monosubstituiert oder durch Fluor- oder Chloratome, durch Methyl-, Methoxy- oder Hydroxygruppen disubstituiert sein können und eine gegebenenfalls in einem Imidazolring vorhandene NH-Gruppe durch eine Alkylgruppe mit 1 bis 6 Kohlenstoffatomen substituiert sein kann, oder

eine $R_{11}$-$NR_{10}$-CO-$NR_9$-Gruppe, in der

$R_9$ ein Wasserstoffatom, eine Alkylgruppe mit 1 bis 6 Kohlenstoffatomen oder Phenylalkylgruppe,

$R_{10}$ ein Wasserstoffatom, eine Alkylgruppe mit 1 bis 6 Kohlenstoffatomen, eine Phenylalkylgruppe oder eine Cycloalkylgruppe mit 5 bis 7 Kohlenstoffatomen,

$R_{11}$ ein Wasserstoffatom oder eine Alkylgruppe mit 1 bis 6 Kohlenstoffatomen oder

$R_{10}$ und $R_{11}$ zusammen mit dem dazwischen liegenden Stickstoffatom eine geradkettige Alkyleniminogruppe mit 4 bis 6 Kohlenstoffatomen oder eine Morpholinogruppe oder

$R_9$ und $R_{11}$ zusammen eine Alkylengruppe mit 2 bis 4 Kohlenstoffatomen darstellen,

$R_b$ eine Cyano-, Carboxy-, Arylsulfonylaminocarbonyl-, Benzylsulfonylaminocarbonyl-, Sulfo-, Alkylcarbonylaminosulfonyl-, Aralkylcarbonylaminosulfonyl-, Arylcarbonylaminosulfonyl-, Alkylcarbonylaminosulfonylmethyl-, Aralkylcarbonylaminosulfonylmethyl-, Arylcarbonylaminosulfonylmethyl-, Phosphino-, O-Alkyl-phosphino-, Phosphono-, O-Alkyl-phosphono-, O,O-Dialkylphosphono-, Phosphonomethyl-, O-Alkyl-phosphonomethyl-, O,O-Dialkylphosphono-methyl-, Phosphato- oder O-Alkyl-phosphatogruppe, eine gegebenenfalls durch eine Phenylalkyl- oder Triphenylmethylgruppe substituierte 1H-Tetrazolyl- oder 1H-Tetrazolylalkylgruppe, eine Alkylsulfonylaminocarbonyl- oder Perfluoralkylsulfonylaminocarbonylgruppe mit jeweils 1 bis 6 Kohlenstoffatomen im Alkylteil, eine Gruppe, die in vivo metabolisch in eine Carboxygruppe umgewandelt wird, oder eine Aralkoxycarbonylgruppe,

$R_c$ ein Wasserstoffatom, eine Methyl-, Phenyl-, Benzyl-, Carboxy- oder Alkoxycarbonylgruppe und

$R_d$ eine geradkettige oder verzweigte Alkylkette mit 1 bis 6 Kohlenstoffatomen, eine geradkettige oder verzweigte Alkenyl- oder Alkinylgruppe mit jeweils 2 bis 6 Kohlenstoffatomen, eine Cycloalkyl- oder Cycloalkylalkylgruppe, eine gegebenenfalls durch Fluor-, Chlor- oder Bromatome, durch Methyl- oder Methoxygruppen mono- oder disubstituierte Phenylgruppe, eine Biphenyl-, Naphthyl- oder Heteroarylgruppe,

$R_e$ und $R_f$ Wasserstoffatome und, wenn

$R_5$ eine Phthalimino- oder Homophthaliminogruppe, wobei eine Carbonylgruppe in einer Phthaliminogruppe durch eine Methylen-, Alkyl-methylen- oder Dialkyl-methylengruppe ersetzt sowie eine Methylengruppe in einer Homophthaliminogruppe durch eine oder zwei Alkylgruppen substituiert sein kann, und zusätzlich die vorstehend erwähnten Phenylkerne durch Alkyl- oder Alkoxygruppen mono- oder disubstituiert und gleichzeitig ganz oder teilweise hydriert sein können, wobei die Substituenten gleich oder verschieden sein können,

eine Carboxygruppe oder eine Gruppe, die in vivo metabolisch in eine Carboxygruppe umgewandelt wird,

eine gegebenenfalls durch eine oder zwei Alkylgruppen oder durch eine Tetramethylen- oder Pentamethylengruppe substituierte 5-, 6- oder 7-gliedrige Alkyleniminogruppe, in welcher eine Methylengruppe durch eine Carbonyl- oder Sulfonylgruppe ersetzt sein kann,

eine gegebenenfalls durch eine Alkyl- oder Phenylgruppe mono- oder disubstituierte Maleinsäureimido- oder Succinimidogruppe, wobei die Substituenten gleich oder verschieden sein können,

einen über ein Kohlenstoffatom oder über eine Iminogruppe gebundenen 5-gliedrigen heteroaromatischen Ring, der eine Iminogruppe, ein Sauerstoff- oder Schwefelatom oder eine Iminogruppe und ein Sauerstoff-, Schwefel- oder Stickstoffatom enthält, oder einen über ein Kohlenstoffatom gebundenen 6-gliedrigen heteroaromatischen Ring, der 1 oder 2 Stickstoffatome enthält, wobei sowohl an die 5-gliedrigen als auch an die 6-gliedrigen heteroaromatischen Ringe jeweils über zwei benachbarte Kohlenstoffatome eine n-Propylen-, n-Butylen- oder 1,3-Butadienylgruppe oder über eine Iminogruppe und ein benachbartes Kohlenstoffatom eine n-Propylen-, n-Butylen- oder 1,3-Butadienylgruppe angefügt ist und in einem so gebildeten anellierten Pyridinring eine Methingruppe durch ein Stickstoffatom und eine Vinylengruppe in 3-, 4-Stellung zu dem Stickstoffatom des gebildeten Pyridinringes durch ein Schwefelatom oder in einem so gebildeten anellierten Phenylring eine oder zwei Methingruppen durch N-Atome ersetzt sein können, wobei zusätzlich die vorstehend erwähnten ankondensierten aromatischen oder heteroaromatischen Ringe im Kohlenstoffgerüst durch ein Fluor-, Chlor- oder Bromatom, durch eine Alkyl-, Alkoxy-, Hydroxy-, Phenyl-, Nitro-, Amino-, Alkylamino-, Dialkylamino-, Alkanoylamino-, Cyano-, Carboxy-, Alkoxycarbonyl-, Aminocarbonyl-, Alkylaminocarbonyl-, Dialkylaminocarbonyl-, Trifluormethyl-, Alkanoyl-, Aminosulfonyl-, Alkylaminosulfonyl- oder Dialkylaminosulfonylgruppe monosubstituiert oder durch Fluor- oder Chloratome, durch Methyl-, Methoxy- oder Hydroxygruppen disubstituiert sein können und eine gegebenenfalls in einem Imidazolring vorhandene NH-Gruppe durch eine Alkylgruppe mit 1 bis 6 Kohlenstoffatomen substituiert sein kann, oder

eine $R_{11}$ $-NR_{10}-CO-NR_9-$Gruppe, in der $R_9$ bis $R_{11}$ wie vorstehend erwähnt definiert sind,

auch $R_e$ ein Wasserstoff-, Fluor-, Chlor- oder Bromatom, eine Alkyl- oder Alkoxygruppe und

$R_f$ ein Fluor-, Chlor- oder Bromatom oder eine Alkoxygruppe bedeuten,

wobei, soweit nichts anderes erwähnt wurde,

der vorstehend erwähnte Begriff "eine Gruppe, die in vivo metabolisch in eine Carboxygruppe umgewandelt wird," wie vorstehend erwähnt definiert ist,

die vorstehend erwähnten Alkyl-, Alkylen- oder Alkoxyteile jeweils 1 bis 4 Kohlenstoffatome und die Cycloalkylteile jeweils 3 bis 7 Kohlenstoffatome enthalten können, sowie

unter "eine Arylgruppe" eine gegebenenfalls durch ein Fluor-, Chlor- oder Bromatom, durch eine Hydroxy-, Alkyl-, Alkoxy-, Phenylalkoxy-, Phenyl- oder Nitrogruppe mono- oder disubstituierte Phenylgruppe, wobei der Alkylteil jeweils 1 bis 4 Kohlenstoffatome enthalten kann, oder eine Naphthylgruppe und

unter "eine Heteroarylgruppe" ein 5-gliedriger heteroaromatischer Ring, der eine Iminogruppe, ein Sauerstoff- oder Schwefelatom oder eine Iminogruppe und ein Sauerstoff-, Schwefel- oder Stickstoffatom oder eine Iminogruppe und 2 oder 3 Stickstoffatome enthält, und ein 6-gliedriger heteroaromatischer Ring, der 1, 2 oder 3 Stickstoffatome enthält, wobei die vorstehend erwähnten Ringe zusätzlich durch ein Fluor-, Chlor- oder Bromatom, durch eine Alkyl-, Alkoxy-, Hydroxy-, Phenyl- oder Nitrogruppe mono- oder disubstituiert sein können, zu verstehen ist,

deren Isomerengemische, deren Tautomere, deren Enantiomere und deren Salze.

Besonders bevorzugte Verbindungen der obigen allgemeinen Formel I sind mit Ausnahme von

(i) 2-n-Butyl-1-[4-[(α-carboxy)benzyloxy]benzyl]benzimidazol,

(ii) 2-n-Propyl-7-methyl-3-[4-[(α-carboxy)benzyloxy]benzyl]imidazo[4,5-b]pyridin und

(iii) 5,7-Dimethyl-2-ethyl-3-[4-[(α-carboxy)benzyloxy]benzyl]imidazo[4,5-b]pyridin

diejenigen, in denen

n die Zahl 0,

A eine Methylen-, Ethylen- oder Ethylidengruppe,

B ein Sauerstoffatom, eine Methylen-, Imino-, Methylimino- oder Acetyliminogruppe,

$R_a$ eine Gruppe der Formel

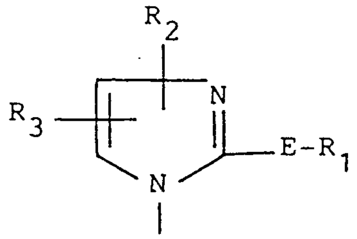

,

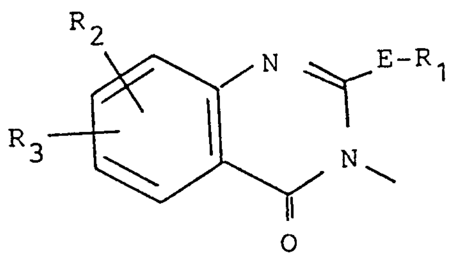

,

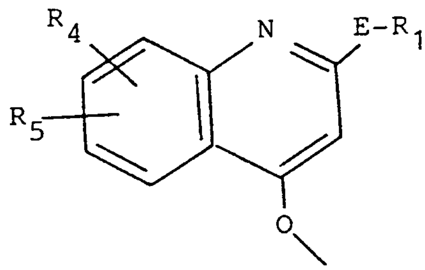

oder

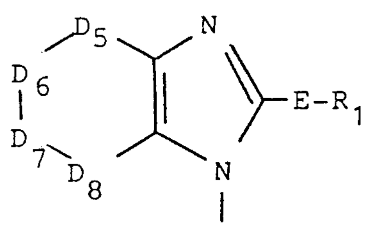

,

in denen null, einer oder zwei der Reste $D_4$, $D_5$, $D_6$ oder $D_7$ ein Stickstoffatom und die verbleibenden Reste $D_4$, $D_5$, $D_6$ oder $D_7$ jeweils Methingruppen, wobei zusätzlich eine Methingruppe durch den Rest $R_4$ und eine weitere Methingruppe durch den Rest $R_5$ substituiert sein kann,

E ein Sauerstoffatom oder eine Kohlenstoff-Kohlenstoff-Bindung,

$R_1$ eine geradkettige oder verzweigte Alkylgruppe mit 1 bis 6 Kohlenstoffatomen,

$R_2$ ein Wasserstoff-, Fluor-, Chlor- oder Bromatom,

$R_3$ eine Hydroxyalkylgruppe mit 1 oder 2 Kohlenstoffatomen,

$R_4$ ein Wasserstoffatom oder eine Alkylgruppe mit 1 bis 3 Kohlenstoffatomen und

$R_5$ ein Wasserstoff-, Fluor-, Chlor- oder Bromatom,

eine geradkettige oder verzweigte Alkylgruppe mit 1 bis 3 Kohlenstoffatomen oder eine α-(1H-Tetrazol-5-yl)-benzyloxygruppe,

eine Amino- oder Nitrogruppe,
eine Carboxygruppe oder eine Alkoxycarbonylgruppe mit insgesamt 2 bis 4 Kohlenstoffatomen,
eine gegebenenfalls am Stickstoffatom durch eine Alkylgruppe mit 1 bis 5 Kohlenstoffatomen substituierte Acylaminogruppe, in welcher der Acylrest eine Alkanoylgruppe mit 1 bis 5 Kohlenstoffatomen oder eine Benzolsulfonylgruppe darstellt, wobei der vorstehend erwähnte Phenylkern durch eine Methyl- oder Methoxygruppe mono- oder disubstituiert und die Substituenten gleich oder verschieden sein können,
eine gegebenenfalls durch eine oder zwei Methylgruppen substituierte 5-, 6- oder 7-gliedrige Alkyleniminogruppe, in welcher eine Methylengruppe durch eine Carbonyl- oder Sulfonylgruppe ersetzt sein kann, oder eine 2,3-Dimethylsuccinimido-, Phthalimino-, Homophthalimino- oder Isoindolin-1-on-yl-gruppe,
eine gegebenenfalls in 1-Stellung durch eine Alkylgruppe mit 1 bis 3 Kohlenstoffatomen substituierte Benzimidazol-2-yl-gruppe oder
eine $R_{11}$-$NR_{10}$-CO-$NR_9$-Gruppe, in der
$R_9$ ein Wasserstoffatom, eine Alkylgruppe mit 1 bis 5 Kohlenstoffatomen oder eine Phenylalkylgruppe mit 1 bis 3 Kohlenstoffatomen im Alkylteil,
$R_{10}$ ein Wasserstoffatom, eine Alkylgruppe mit 1 bis 5 Kohlenstoffatomen oder eine Cyclohezylgruppe,
$R_{11}$ ein Wasserstoffatom, eine Benzylgruppe oder eine Alkylgruppe mit 1 bis 5 Kohlenstoffatomen oder
$R_9$ und $R_{11}$ zusammen eine Alkylengruppe mit 2 oder 3 Kohlenstoffatomen darstellen,
$R_b$ eine Carboxy-, 1H-Tetrazolyl- oder 0-Alkyl-phosphono- oder Alkylsulfonylaminocarbonylgruppe mit jeweils 1 bis 3 Kohlenstoffatomen im Alkylteil,
$R_c$ ein Wasserstoffatom oder eine Phenylgruppe,
$R_d$ eine geradkettige oder verzweigte Alkylkette mit 1 bis 4 Kohlenstoffatomen, eine Cyclohexyl-, Cyclohexylmethyl-, Phenyl-, Biphenyl-, Methoxyphenyl-, Chlorphenyl-, Pyridyl- oder Naphthylgruppe,
$R_e$ und $R_f$ Wasserstoffatome und, wenn
$R_5$ eine gegebenenfalls in 1-Stellung durch eine Alkylgruppe mit 1 bis 3 Kohlenstoffatomen substituierte Benzimidazol-2-yl-gruppe,
eine 2,3-Dimethylsucciniminogruppe,
eine gegebenenfalls durch eine oder zwei Methylgruppen substituierte 5-, 6- oder 7-gliedrige Alkyleniminogruppe, in welcher eine Methylengruppe durch eine Carbonyl- oder Sulfonylgruppe ersetzt sein kann, oder eine $R_{11}$-$NR_{10}$-CO-$NR_9$-Gruppe, in der $R_9$ bis $R_{11}$ wie vorstehend erwähnt definiert sind,
auch $R_e$ ein Wasserstoff-, Chlor- oder Bromatom oder eine Methoxygruppe und
$R_f$ ein Chlor- oder Bromatom oder eine Methoxygruppe bedeuten,
deren Isomerengemische, deren Tautomere, deren Enantiomere und deren Salze.

Erfindungsgemäß erhält man die Verbindungen der allgemeinen Formel I nach folgenden Verfahren:

a) Umsetzung einer Verbindung der allgemeinen Formel

$R_a$ - H ,(II)

in der
$R_a$ wie eingangs definiert ist, mit einer Verbindung der allgemeinen Formel

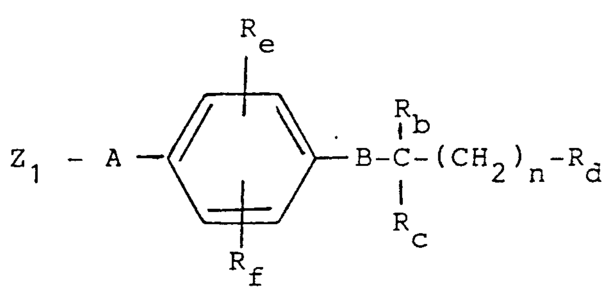
,(III)

in der
A, B, n und $R_c$ bis $R_f$ wie eingangs definiert sind und $Z_1$ eine nukleophile Austrittsgruppe wie ein Halogenatom, z.B. ein Chlor-, Brom- oder Jodatom, oder eine substituierte Sulfonyloxygruppe, z.B. eine Methansulfonyloxy-, Phenylsulfonyloxy- oder p-Toluolsulfonyloxygruppe, darstellt, und erforderlichenfalls anschließende Hydrolyse.

Die Umsetzung wird zweckmäßigerweise in einem Lösungsmittel oder Lösungsmittelgemisch wie Methylenchlorid, Diethylether, Tetrahydrofuran, Dioxan, Dimethylsulfoxid, Dimethylformamid oder Benzol

gegebenenfalls in Gegenwart eines säurebindenden Mittels, wie Natriumcarbonat, Kaliumcarbonat, Natriumhydroxid, Kalium-tert.butylat, Triethylamin oder Pyridin, wobei die beiden letzteren gleichzeitig auch als Lösungsmittel verwendet werden können, vorzugsweise bei Temperaturen zwischen 0 und 100 ° C, z.B. bei Temperaturen zwischen Raumtemperatur und 50 ° C, durchgeführt.

Die anschließende Hydrolyse wird zweckmäßigerweise entweder in Gegenwart einer Säure wie Salzsäure, Schwefelsäure, Phosphorsäure, Trichloressigsäure oder Trifluoressigsäure oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 ° C und 120 ° C, z.B. bei Temperaturen zwischen Raumtemperatur und der Siedetemperatur des Reaktionsgemisches, durchgeführt.

Bei der Umsetzung erhält man vorzugsweise ein Gemisch möglicher Regio-Isomeren, welches gewünschtenfalls anschließend, vorzugsweise chromatographisch unter Verwendung eines Trägers wie Kieselgel oder Aluminiumoxid, in die entsprechenden Isomeren aufgetrennt wird.

b) Umsetzung einer Verbindung der allgemeinen Formel

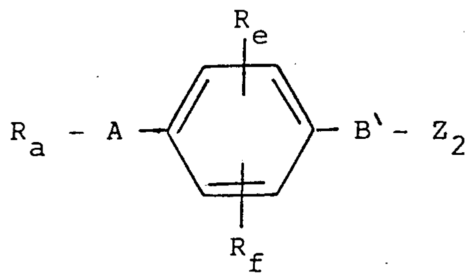

,(IV)

in der

$R_a$, $R_e$, $R_f$ und A wie eingangs definiert sind,

B' eine geradkettige oder verzweigte Alkylengruppe mit 1 bis 4 Kohlenstoffatomen und

$Z_2$ eine nukleophile Austrittsgruppe wie ein Halogenatom, z.B. ein Chlor-, Brom- oder Jodatom, oder eine substituierte Sulfonyloxygruppe, z.B. eine Methansulfonyloxy-, Phenylsulfonyloxy- oder p-Toluolsulfonyloxygruppe, bedeuten, mit einer Verbindung der allgemeinen Formel

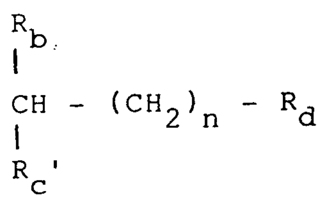

,(V)

$R_b$, $R_d$ und n wie eingangs definiert sind und

$R_c$' eine Alkoxycarbonylgruppe darstellt, erforderlichenfalls anschließende Hydrolyse und/oder Decarboxylierung.

Die Umsetzung wird zweckmäßigerweise in einem Lösungsmittel oder Lösungsmittelgemisch wie Methylenchlorid, Diethylether, Tetrahydrofuran, Dioxan, Dimethylsulfoxid, Dimethylformamid oder Benzol gegebenenfalls in Gegenwart eines säurebindenden Mittels, wie Natriumcarbonat, Kaliumcarbonat, Natriumhydroxid, Kalium-tert.butylat, Triethylamin oder Pyridin, wobei die beiden letzteren gleichzeitig auch als Lösungsmittel verwendet werden können, vorzugsweise bei Temperaturen zwischen 0 und 100 ° C, z.B. bei Temperaturen zwischen Raumtemperatur und 50 ° C, durchgeführt.

Die anschließende Hydrolyse wird zweckmäßigerweise entweder in Gegenwart einer Säure wie Salzsäure, Schwefelsäure, Phosphorsäure, Trichloressigsäure oder Trifluoressigsäure oder in Gegenwart einer Base wie Natriumhydrozid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 ° C und 120 ° C, z.B. bei Temperaturen zwischen Raumtemperatur und der Siedetemperatur des Reaktionsgemisches, durchgeführt.

Die anschließende Decarboxylierung wird zweckmäßigerweise in Gegenwart einer Säure wie Salzsäure, Schwefelsäure, Phosphorsäure, Essigsäure, Trichloressigsäure oder Trifluoressigsäure in einem geeigneten Lösungsmittel wie Wasser, Wasser/Methanol, Äthanol, Wasser/Äthanol, Wasser/Isopropanol oder Wasser/Dioxan bei Temperaturen zwischen 50°C und 120°C, z.B. bei Temperaturen zwischen 60°C und der Siedetemperatur des Reaktionsgemisches, durchgeführt.

c) Zur Herstellung von Verbindungen der allgemeinen Formel I, in der B ein Sauerstoff- oder Schwefelatom oder eine gegebenenfalls durch eine Alkylgruppe substituierte Iminogruppe darstellt:
Umsetzung einer Verbindung der allgemeinen Formel

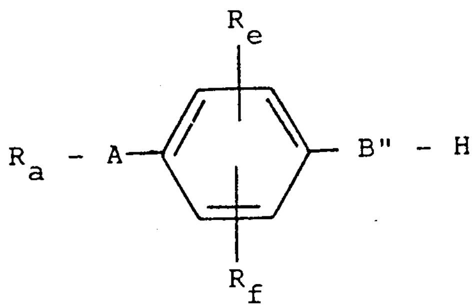

,(VI)

in der
$R_a$, $R_e$, $R_f$ und A wie eingangs definiert sind und
B" ein Sauerstoff- oder Schwefelatom oder eine gegebenenfalls durch eine Alkylgruppe substituierte Iminogruppe darstellt, mit einer Verbindung der allgemeinen Formel

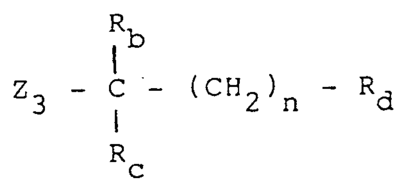

,(VII)

in der
$R_b$ bis $R_d$ und n wie eingangs definiert sind und
$Z_3$ eine nukleophile Austrittsgruppe wie ein Halogenatom, z.B. ein Chlor-, Brom- oder Jodatom, oder eine substituierte Sulfonyloxygruppe, z.B. eine Methansulfonyloxy-, Phenylsulfonyloxy- oder p-Toluolsulfonyloxygruppe, bedeutet, und erforderlichenfalls anschließende Hydrolyse.

Die Umsetzung wird zweckmäßigerweise in einem Lösungsmittel oder Lösungsmittelgemisch wie Methylenchlorid, Diethylether, Tetrahydrofuran, Dioxan, Dimethylsulfoxid, Dimethylformamid oder Benzol gegebenenfalls in Gegenwart eines säurebindenden Mittels, wie Natriumacetat, Natriumcarbonat, Kaliumcarbonat, Natriumhydroxid, Kalium-tert.butylat, Triethylamin oder Pyridin, wobei die beiden letzteren gleichzeitig auch als Lösungsmittel verwendet werden können, vorzugsweise bei Temperaturen zwischen 0 und 100°C, z.B. bei Temperaturen zwischen Raumtemperatur und der Siedetemperatur des Reaktionsgemisches, durchgeführt.

Die anschließende Hydrolyse wird zweckmäßigerweise entweder in Gegenwart einer Säure wie Salzsäure, Schwefelsäure, Phosphorsäure, Trichloressigsäure oder Trifluoressigsäure oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10°C und 120°C, z.B. bei Temperaturen zwischen Raumtemperatur und der Siedetemperatur des Reaktionsgemisches, durchgeführt.

d) Zur Herstellung einer Verbindung der allgemeinen Formel I, in der $R_b$ eine Carboxygruppe darstellt:
Überführung einer Verbindung der allgemeinen Formel

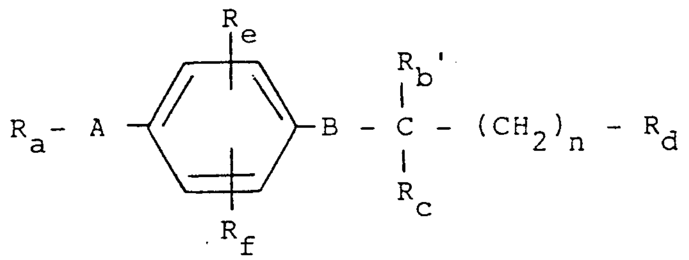

,(VIII)

in der

$R_a$, $R_c$ bis $R_f$, A, B und n wie eingangs definiert sind und

$R_b$' eine mittels Hydrolyse, Thermolyse oder Hydrogenolyse in eine Carboxygruppe überführbare Gruppe darstellt.

Beispielsweise können funktionelle Derivate der Carboxygruppe wie deren unsubstituierte oder substituierte Amide, Ester, Thiolester, Orthoester, Iminoäther, Amidine oder Anhydride, die Nitrilgruppe oder die Tetrazolylgruppe mittels Hydrolyse in eine Carboxygruppe,

Ester mit tertiären Alkoholen, z.B. der tert. Butylester, mittels Thermolyse in eine Carboxygruppe und

Ester mit Aralkanolen, z.B. der Benzylester, mittels Hydrogenolyse in eine Carboxygruppe übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 ° C und 120 ° C, z.B. bei Temperaturen zwischen Raumtemperatur und der Siedetemperatur des Reaktionsgemisches, durchgeführt. Bei der Hydrolyse in Gegenwart einer organischen Säuren wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet $R_b$' in einer Verbindung der allgemeinen Formel VIII eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen O und 50 ° C in die Carboxygruppe übergeführt werden.

Bedeutet $R_b$' in einer Verbindung der allgemeinen Formel VIII beispielsweise die tert. Butyloxycarbonylgruppe, so kann die tert. Butylgruppe auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie Trifluoressigsäure, p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 ° C und 100 ° C, abgespalten werden.

Bedeutet $R_b$' in einer Verbindung der allgemeinen Formel VIII beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid vorzugsweise bei Temperaturen zwischen 0 und 50 ° C, z.B. bei Raumtemperatur, und einem Wasserstoffdruck von 1 bis 5 bar abgespalten werden. Bei der Hydrogenolyse können gleichzeitig andere Reste, z.B. eine Nitrogruppe zur Aminogruppe, eine Benzyloxygruppe zur Hydroxygruppe, eine Vinylidengruppe zur entsprechenden Alkylidengruppe oder eine Zimtsäuregruppe zur entsprechenden Phenyl-propionsäuregruppe, mitreduziert oder durch Wasserstoffatome, z.B. ein Halogenatom durch ein Wasserstoffatom, ersetzt werden.

e) Zur Herstellung einer Verbindung der allgemeinen Formel I, in der $R_b$ eine 1H-Tetrazolylgruppe darstellt:

Abspaltung eines Schutzrestes von einer Verbindung der allgemeinen Formel

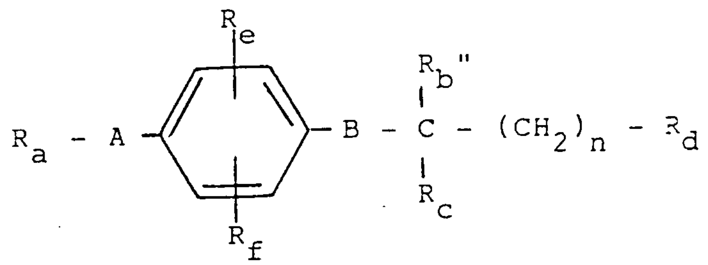

,(IX)

in der
$R_a$, $R_c$ bis $R_f$, A, B und n wie eingangs definiert sind und
$R_b$'' eine in 1- oder 2-Stellung durch einen Schutzrest geschützte 1H-Tetrazolylgruppe darstellt.

Als Schutzrest kommt beispielsweise die Triphenylmethyl-, Tributylzinn- oder Triphenylzinngruppe in Betracht.

Die Abspaltung eines verwendeten Schutzrestes erfolgt vorzugsweise in Gegenwart eines Halogenwasserstoffes, vorzugsweise in Gegenwart von Chlorwasserstoff, in Gegenwart einer Base wie Natriumhydroxid oder alkoholischem Ammoniak in einem geeigneten Lösungsmittel wie Methylenchlorid, Methanol, Methanol/Ammoniak, Ethanol oder Isopropanol bei Temperaturen zwischen 0 und 100°C, vorzugsweise jedoch bei Raumtemperatur, oder auch, falls die Umsetzung in Gegenwart von alkoholischem Ammoniak durchgeführt wird, bei erhöhten Temperaturen, z.B. bei Temperaturen zwischen 100 und 150°C, vorzugsweise bei Temperaturen zwischen 120 und 140°C.

f) Zur Herstellung einer Verbindung der allgemeinen Formel I, in der $R_b$ eine 1H-Tetrazolylgruppe darstellt:
Umsetzung einer Verbindung der allgemeinen Formel

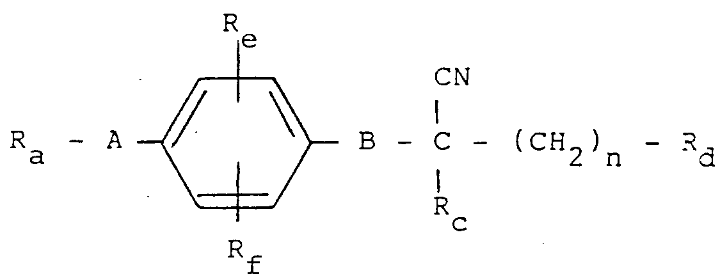

,(X)

in der
$R_a$, $R_c$ bis $R_f$, A, B und n wie eingangs definiert sind, mit Stickstoffwasserstoffsäure oder deren Salzen.

Die Umsetzung wird vorzugsweise in einem Lösungsmittel wie Benzol, Toluol oder Dimethylformamid bei Temperaturen zwischen 80 und 150°C, vorzugsweise bei 125°C, durchgeführt. Hierbei wird zweckmäßigerweise entweder die Stickstoffwasserstoffsäure während der Umsetzung aus einem Alkaliazid, z.B. aus Natriumazid, in Gegenwart einer schwachen Säure wie Ammoniumchlorid freigesetzt oder das im Reaktionsgemisch bei der Umsetzung mit einem Salz der Stickstoffwassersäure, vorzugsweise mit Aluminiumazid oder Tributylzinnazid, welche außerdem zweckmäßigerweise im Reaktionsgemisch durch Umsetzung von Aluminiumchlorid oder Tributylzinnchlorid mit einem Alkaliazid wie Natriumazid hergestellt werden, erhaltene Tetrazolidsalz anschließend durch Ansäuern mit einer verdünnten Säure wie 2N Salzsäure oder 2N Schwefelsäure freigesetzt.

g) Zur Herstellung von Verbindungen der allgemeinen Formel I, in der $R_a$ eine Gruppe der Formel

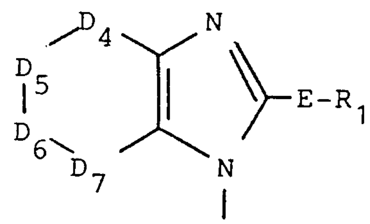

darstellt:
Cyclisierung einer Verbindung der allgemeinen Formel

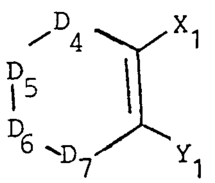 ,(XI)

in der
$D_1$ bis $D_7$ wie eingangs definiert sind,
einer der Reste $X_1$ oder $Y_1$ eine Gruppe der allgemeinen Formel

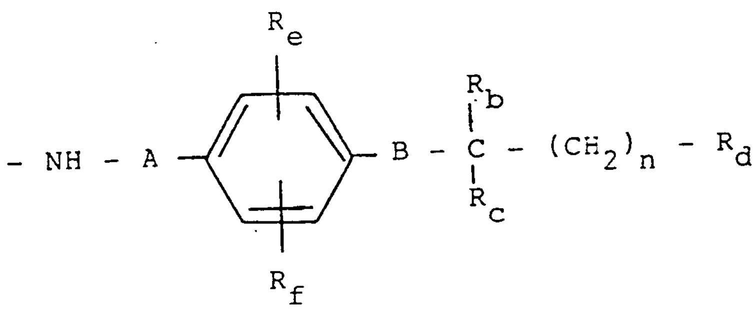

und der andere der Reste $X_1$ oder $Y_1$ eine Gruppe der allgemeinen Formel

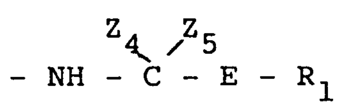

darstellen, wobei
$R_1$, A, B, E, n und $R_b$ bis $R_f$ wie eingangs definiert sind,
$Z_4$ und $Z_5$, die gleich oder verschieden sein können, gegebenenfalls substituierte Aminogruppen oder gegebenenfalls durch niedere Alkylgruppen substituierte Hydroxy- oder Mercaptogruppen oder
$Z_4$ und $Z_5$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin oder in einem Überschuß des zur Herstellung der Verbindung der allgemeinen Formel XI verwendeten Acylierungsmittel, z.B. in dem entsprechenden Nitril, Anhydrid, Säurehalogenid, Ester oder Amid, beispielsweise bei Temperaturen zwischen 0 und 250 °C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Methansulfonsäure, Salzsäure, Phosphorsäure, Polyphosphorsäure, Essigsäureanhydrid oder gegebenenfalls auch in Gegenwart einer Base wie Kaliumäthylat oder Kalium-tert.butylat durchgeführt. Die Cyclisierung kann jedoch auch ohne Lösungsmittel und/oder Kondensationsmittel durchgeführt werden.

Besonders vorteilhaft wird die Umsetzung jedoch in der Weise durchgeführt, daß eine Verbindung der allgemeinen Formel XI im Reaktionsgemisch durch Reduktion einer entsprechenden o-Nitro-aminoverbindung gegebenenfalls in Gegenwart einer Carbonsäure der allgemeinen Formel $R_a$-E-COOH oder durch Acylierung einer entsprechenden o-Diaminoverbindung hergestellt wird. Bei Abbruch der Reduktion der Nitrogruppe auf der Hydroxylaminstufe erhält man bei der anschließenden Cyclisierung das N-Oxid einer Verbindung der allgemeinen Formel I. Das so erhaltene N-Oxid wird anschließend mittels Reduktion in eine entsprechende Verbindung der allgemeinen Formel I übergeführt.

Die anschließende Reduktion des erhaltenen N-Oxids der Formel I wird vorzugsweise in einem Lösungsmittel wie Wasser, Wasser/Äthanol, Methanol, Eisessig, Essigsäureäthylester oder Dimethylformamid mit Wasserstoff in Gegenwart eines Hydrierungskatalysators wie Raney-Nickel, Platin oder Palladium/Kohle, mit Metallen wie Eisen, Zinn oder Zink in Gegenwart einer Säure wie Essigsäure, Salzsäure oder Schwefelsäure, mit Salzen wie Eisen(II)sulfat, Zinn(II)chlorid oder Natriumdithionit, oder mit Hydrazin in Gegenwart von Raney-Nickel bei Temperaturen zwischen 0 und 50 °C, vorzugsweise jedoch bei Raumtemperatur durchgeführt.

h) Zur Herstellung von Verbindungen der allgemeinen Formel I, in der $R_5$ eine $R_{11}$-$NR_{10}$-CO-$NR_9$-Gruppe darstellt:

Umsetzung einer Verbindung der Formel

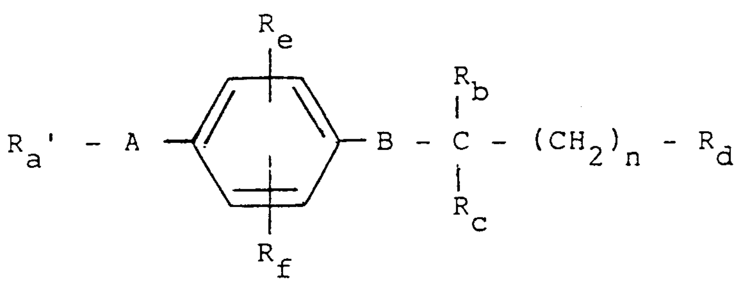

,(XII)

in der

$R_b$ bis $R_f$, A, B und n wie eingangs definiert sind und

$R_a$' einen der für $R_a$ eingangs erwähnten Reste darstellt, in dem $R_5$ eine Aminogruppe, eine Alkylaminogruppe mit 1 bis 8 Kohlenstoffatomen oder eine Phenylalkylaminogruppe mit 1 bis 4 Kohlenstoffatomen im Alkylteil darstellt, mit einer Verbindung der Formel

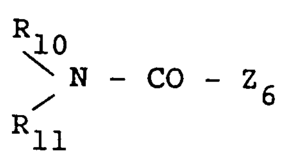

,(XIII)

in der

$R_{10}$ ein Wasserstoffatom, eine Alkylgruppe mit 1 bis 8 Kohlenstoffatomen, eine Alkenylgruppe mit 3 bis 5 Kohlenstoffatomen, eine Phenylalkylgruppe oder eine Cycloalkylgruppe mit 5 bis 7 Kohlenstoffatomen,

$R_{11}$ ein Wasserstoffatom oder eine Alkylgruppe mit 1 bis 6 Kohlenstoffatomen oder

einer der Reste $R_{10}$ oder $R_{11}$ auch eine Bicyclohexyl- oder Biphenylylgruppe oder

$R_{10}$ und $R_{11}$ zusammen mit dem dazwischen liegenden Stickstoffatom eine geradkettige Alkyleniminogruppe mit 4 bis 6 Kohlenstoffatomen oder eine Morpholinogruppe und

$Z_6$ eine nukleophile Austrittsgruppe wie ein Chlor- oder Bromatom oder auch, wenn einer der Reste $R_{10}$ oder $R_{11}$ ein Wasserstoffatom darstellt,

$Z_6$ zusammen mit $R_{10}$ oder $R_{11}$ eine Stickstoff-Kohlenstoff-Bindung darstellen.

Die Umsetzung wird vorzugsweise in einem Lösungsmittel wie Tetrahydrofuran, Dioxan, Ethylenchlorid oder Benzol gegebenenfalls in Gegewart eines säurebindenden Mittels wie Triethylamin oder Pyridin zweckmäßigerweise bei Temperaturen zwischen 0 und 100 °C, vorzugsweise bei Temperaturen zwischen

20 und 80 ° C, durchgeführt.

i) Zur Herstellung von Verbindungen der allgemeinen Formel I, in der $R_5$ eine gegebenenfalls am Stickstoffatom durch eine Alkylgruppe mit 1 bis 6 Kohlenstoffatomen, durch eine Phenyl-, Cycloalkyl-, Phenylalkyl-, Cycloalkylalkyl-, Bicyclohexyl- oder Biphenylgruppe substituierte Acylaminogruppe, in welcher der Acylrest eine Alkanoylgruppe mit 1 bis 7 Kohlenstoffatomen, eine Alkoxycarbonylgruppe mit insgesamt 2 bis 4 Kohlenstoffatomen, eine Alkylsulfonylgruppe mit 1 bis 6 Kohlenstoffatomen, eine Benzoyl-, Benzolsulfonyl-, Phenylalkansulfonyl-, Naphthalinsulfonyl-, Cycloalkylcarbonyl-, Phenylalkanoyl- oder Cycloalkylalkanoylgruppe darstellt, wobei die vorstehend erwähnten Phenylkerne jeweils durch ein Fluor-, Chlor- oder Bromatom, durch eine Methyl- oder Methoxygruppe mono- oder disubstituiert und die Substituenten gleich oder verschieden sein können, eine Phthalimino- oder Homophthaliminogruppe, in denen der Phenylkern jeweils durch Alkyl- oder Alkoxygruppen mit jeweils 1 bis 3 Kohlenstoffatomen mono- oder disubstituiert sein kann, oder eine gegebenenfalls durch eine Alkyl- oder Phenylgruppe mono- oder disubstituierte Maleinsäureimido- oder Succinimidogruppe, wobei die Substituenten gleich oder verschieden sein können, darstellt:

Acylierung einer Verbindung der Formel

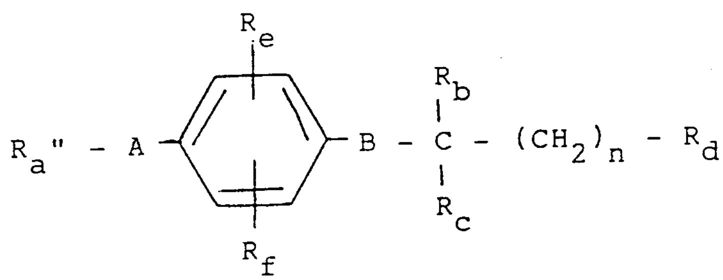

,(XIV)

in der

$R_b$ bis $R_f$, A, B und n wie eingangs definiert sind und

$R_a''$ einen der für $R_a$ eingangs erwähnten Reste darstellt, in dem $R_5$ eine gegebenfalls durch eine Alkylgruppe mit 1 bis 6 Kohlenstoffatomen, durch eine Phenyl-, Cycloalkyl-, Phenylalkyl-, Cycloalkylalkyl-, Bicyclohexyl- oder Biphenylgruppe substituierte Aminogruppe darstellt, mit einer Verbindung der Formel

$HO - U - R_{12}$ ,(XV)

in der

U eine Carbonyl- oder Sulfonylgruppe und

$R_{12}$ eine Alkylgruppe mit 1 bis 6 Kohlenstoffatomen, eine Alkoxygruppe mit 1 bis 3 Kohlenstoffatomen, eine Phenyl-, Phenylalkyl-, Naphthyl- oder Cycloalkylgruppe, wobei die vorstehend erwähnten Phenylkerne jeweils durch ein Fluor-, Chlor- oder Bromatom, durch eine Methyl- oder Methoxygruppe mono- oder disubstituiert und die Substituenten gleich oder verschieden sein können, oder auch, wenn U eine Carbonylgruppe darstellt, ein Wasserstoffatom, eine Phenylgruppe, die in o-Stellung durch eine Carboxy- oder Carboxymethylgruppe substituiert ist und in der der Phenylkern durch Alkyl- oder Alkoxygruppen mit jeweils 1 bis 3 Kohlenstoffatomen mono- oder disubstituiert sowie zusätzlich ganz oder teilweise hydriert sein kann, eine 2-Carboxy-ethyl- oder 2-Carboxy-ethenylgruppe, in welcher der Ethyl- und Ethenylteil jeweils durch eine Alkyl- oder Phenylgruppe mono- oder disubstituiert sein kann, bedeuten, oder mit deren reaktionsfähigen Derivaten wie deren Säurehalogeniden, Säureestern oder Säureanhydriden.

Als reaktionsfähige Derivate einer Verbindung der Formel XV kommen beispielsweise deren Ester wie der Methyl-, Ethyl- oder Benzylester, deren Thioester wie der Methylthio- oder Ethylthioester, deren Halogenide wie das Säurechlorid, deren Anhydride oder Imidazolide in Betracht.

Die Umsetzung wird zweckmäßigerweise in einem Lösungsmittel oder Lösungsmittelgemisch wie Wasser, Methylenchlorid, Chloroform, Äther, Tetrahydrofuran, Dioxan oder Dimethylformamid mit einer entsprechenden Carbonsäure in Gegenwart eines die Säure aktivierenden oder wasserentziehenden Mittels wie Thionylchlorid, mit deren Anhydriden wie Essigsäureanhydrid, mit deren Estern wie Essigsäureäthylester, mit deren Halogeniden wie Acetylchlorid oder Methansulfonylchlorid gegebenenfalls in Gegenwart einer anorganischen oder tertiären organischen Base, wie Natriumhydroxid, Kaliumcarbonat,

Triäthylamin oder Pyridin, wobei die beiden letzteren gleichzeitig auch als Lösungsmittel dienen können, bei Temperaturen zwischen -25 und 100 ° C, vorzugsweise jedoch bei Temperaturen zwischen -10 und 80 ° C, durchgeführt.

j) Zur Herstellung von Verbindungen der allgemeinen Formel I, in der $R_b$ eine Phosphono- oder O-Alkylphosphonogruppe darstellt:
Hydrolyse einer Verbindung der allgemeinen Formel

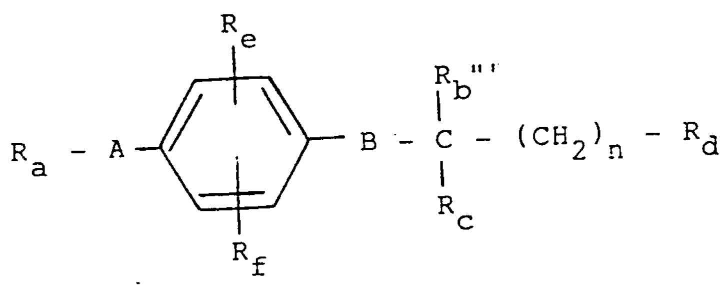

,(XVI)

in der
A, B, $R_a$, $R_c$ bis $R_f$ und n wie eingangs definiert sind und
Rb''' eine O-Alkyl- oder O,O-Dialkyl-phosphonogruppe darstellt, in welcher der Alkylteil jeweils 1 bis 5 Kohlenstoffatome enthalten kann.

Die Umsetzung wird vorzugsweise in einem Lösungsmittel wie Methanol, Methanol/Wasser, Äthanol oder Isopropanol zur Herstellung einer entsprechenden O-Alkylphosphonoverbindung der allgemeinen Formel I in Gegenwart einer Base wie Kaliumhydroxid oder Kaliummethylat oder zur Herstellung einer entsprechenden Phosphonoverbindung der allgemeinen Formel I in Gegenwart einer Säure wie Bromwasserstoffsäure bei der Siedetemperatur des Reaktionsgemisches, durchgeführt.

Eine Phosphonoverbindung der allgemeinen Formel I wird besonders vorteilhaft durch Umsetzung einer entsprechenden O-Alkyl- oder O,O-Dialkylverbindung der allgemeinen Formel XVI mit Brom/Trimethylchlorsilan in Acetonitril und anschließende Hydrolyse mittels Wasser bei Raumtemperatur durchgeführt.

Erhält man erfindungsgemäß eine Verbindung der allgemeinen Formel I, in der $R_2$ oder $R_5$ eine Nitrogruppe darstellt, so kann diese mittels Reduktion in eine entsprechende Verbindung der allgemeinen Formel I, in der $R_2$ oder $R_5$ eine Aminogruppe darstellt, übergeführt werden oder
erhält man erfindungsgemäß eine Verbindung der allgemeinen Formel I, in der B eine Iminogruppe darstellt, so kann diese mittels Alkanoylierung in eine entsprechende Verbindung der allgemeinen Formel I übergeführt werden, in der B eine durch eine Alkanoylgruppe mit 1 bis 4 Kohlenstoffatomen substituierte Iminogruppe darstellt, oder
erhält man erfindungsgemäß eine Verbindung der allgemeinen Formel I, in der B eine Iminogruppe darstellt, so kann diese mittels Alkylierung in eine entsprechende Verbindung der allgemeinen Formel I übergeführt werden, in der B eine durch eine Alkylgruppe mit 1 bis 3 Kohlenstoffatomen substituierte Iminogruppe darstellt, oder
erhält man ein erfindungsgemäß eine Verbindung der allgemeinen Formel I, in der $R_b$ oder $R_b$ und $R_5$ jeweils eine Carboxygruppe darstellen, so kann diese mittels Veresterung in eine entsprechende Verbindung der allgemeinen Formel I, in der $R_b$ oder $R_b$ und $R_5$ eine Gruppe darstellt, die in vivo metabolisch in eine Carboxygruppe umgewandelt wird, übergeführt werden oder
erhält man erfindungsgemäß eine Verbindung der allgemeinen Formel I, in der $R_b$ eine Carboxygruppe darstellt, so kann diese nach Überführung in ein entsprechendes Säurehalogenid mittels Umsetzung mit einem entsprechenden Sulfonamid in eine Verbindung der allgemeinen Formel I, in der $R_b$ eineAlkansulfonylaminocarbonyl-, Perfluoralkansulfonylaminocarbonyl-, Arylsulfonylaminocarbonyl- oder Benzylsulfonylaminocarbonylgruppe darstellt, übergeführt werden.

Die nachträgliche Reduktion der Nitrogruppe wird vorzugsweise in einem Lösungsmittel wie Wasser, Wasser/Ethanol, Methanol, Eisessig, Essigsäurethylester oder Dimethylformamid zweckmäßigerweise mit Wasserstoff in Gegenwart eines Hydrierungskatalysators wie Raney-Nickel, Platin oder Palladium/Kohle, mit Metallen wie Eisen, Zinn oder Zink in Gegenwart einer Säure, mit Salzen wie Eisen(II)sulfat, Zinn(II)chlorid, Natriumsulfid, Natriumhydrogensulfit oder Natriumdithionit, oder mit Hydrazin in Gegenwart von Raney-Nickel bei Temperaturen zwischen 0 und 80 ° C, vorzugsweise jedoch bei Temperaturen zwischen 20 und 40 ° C, durchgeführt.

Die anschließende Alkanoylierung wird zweckmäßigerweise in einem Lösungsmittel oder Lösungsmittelgemisch wie Wasser, Methylenchlorid, Chloroform, Äther, Tetrahydrofuran, Dioxan oder Dimethylformamid mit einer entsprechenden Carbonsäure in Gegenwart eines die Säure aktivierenden oder wasserentziehenden Mittels wie Thionylchlorid, mit deren Anhydriden wie Essigsäureanhydrid, mit deren Estern wie Essigsäureäthylester, mit deren Halogeniden wie Acetylchlorid gegebenenfalls in Gegenwart einer anorganischen oder tertiären organischen Base, wie Natriumhydroxid, Kaliumcarbonat, Triäthylamin oder Pyridin, wobei die beiden letzteren gleichzeitig auch als Lösungsmittel dienen können, bei Temperaturen zwischen -25 und 100 ° C, vorzugsweise jedoch bei Temperaturen zwischen -10 und 80 ° C, durchgeführt.

Die anschließende Alkylierung wird zweckmäßigerweise in einem Lösungsmittel oder Lösungsmittelgemisch wie Methylenchlorid, Diethylether, Tetrahydrofuran, Dioxan, Dimethylsulfoxid, Dimethylformamid oder Benzol gegebenenfalls in Gegenwart eines säurebindenden Mittels, wie Natriumcarbonat, Kaliumcarbonat, Natriumhydroxid, Kalium-tert.butylat, Triethylamin oder Pyridin, wobei die beiden letzteren gleichzeitig auch als Lösungsmittel verwendet werden können, mit einem Alkylierungsmittel wie Methyljodid, Ethyljodid, Isopropylbromid, Dimethylsulfat, Diethylsulfat oder p-Toluolsulfonsäuremethylester vorzugsweise bei Temperaturen zwischen 0 und 100 ° C, z.B. bei Temperaturen zwischen Raumtemperatur und 50 ° C, durchgeführt.

Die Überführung einer Carboxylgruppe in eine Gruppe, die in vivo metabolisch in eine Carboxygruppe umgewandelt wird, erfolgt zweckmäßigerweise durch Veresterung mit einem entsprechenden Alkohol oder mit einem entsprechenden reaktionsfähigen Acylderivat zweckmäßigerweise in einem Lösungsmittel oder Lösungsmittelgemisch wie Wasser, Methylenchlorid, Chloroform, Äther, Tetrahydrofuran, Dioxan oder Dimethylformamid oder in einem Überschuß des Acylierungsmittels als Lösungsmittel gegebenenfalls in Gegenwart eines die Säure aktivierenden oder wasserentziehenden Mittels wie Thionylchlorid, mit deren Anhydriden, Estern oder Halogeniden gegebenenfalls in Gegenwart einer anorganischen oder tertiären organischen Base, wie Natriumhydroxid, Kaliumcarbonat, Triäthylamin oder Pyridin, wobei die beiden letzteren gleichzeitig auch als Lösungsmittel dienen können, bei Temperaturen zwischen -25 und 100 ° C, vorzugsweise jedoch bei Temperaturen zwischen -10 und 80 ° C, durchgeführt.

Die anschließende Umsetzung eines Säurehalogenids mit einem entsprechenden Sulfonamid wird zweckmäßigerweise in einem Lösungsmittel wie Aceton, Tetrahydrofuran oder Dioxan in Gegenwart einer Base wie Triethylamin bei Temperaturen zwischen 0 und 50 ° C, vorzugsweise bei Temperaturen zwischen 10 und 30 ° C, durchgeführt.

Bei den vorstehend beschriebenen Umsetzungen können gegebenenfalls vorhandene reaktive Gruppen wie Hydroxy-, Amino- oder Alkylaminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.Butyl-, Benzyl- oder Tetrahydropyranylgruppe und als Schutzrest für eine Amino-, Alkylamino- oder Iminogruppe die Acetyl-, Benzoyl-, Ethoxycarbonyl- oder Benzylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 ° C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 ° C, vorzugsweise jedoch bei Raumtemperatur, und einem Wasserstoffdruck von 1 bis 7 bar, vorzugsweise jedoch von 3 bis 5 bar.

Ein so erhaltenes Isomerengemisch einer Verbindung der allgemeinen Formel I kann gewünschtenfalls vorzugsweise chromatographisch unter Verwendung eines Trägers wie Kieselgel oder Aluminiumoxid getrennt werden.

Desweiteren können die erhaltenen Verbindungen der allgemeinen Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der allgemeinen Formel I, falls diese eine Carboxy- oder 1H-Tetrazolylgruppe enthalten, gewünschtenfalls anschließend in ihre Salze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der allgemeinen Formeln II bis XVI sind teilweise literaturbekannt oder man erhält diese nach literaturbekannten Verfahren.

So werden beispielsweise substituierte Pyrimidine der allgemeinen Formel II in der EP-A-0,424,317, substituierte Pyrimidinone in der EP-A-0,407,342 und EP-A-0,419,048, substituierte Triazole in der EP-A-0,409,332, substituierte Triazolinone, Triazolthione und Triazolimine in der EP-A-0,412,594, substituierte Pyrazole in der EP-A-0,411,507, substituierte Chinazolinone in der EP-A-0,411,766, substituierte Chinoline in der EP-A-0,412,848, 5-gliedrige kondensierte Imidazoderivate in der EP-A-0,407,102, Benzimidazole in der EP-A-0,392,317, Imidazopyridine und Purine in der EP-A-0,400,974 und EP-A-0,399,731 und substituierte Imidazole in der EP-A-0,253,310 beschrieben.

Die als Ausgangsstoffe verwendeten Verbindungen der allgemeinen Formeln III und IV erhält man durch Umsetzung eines entsprechenden Alkohols mit Halogenwasserstoff, mit Tetrabrommethan/Triphenylphosphin oder mit einem entsprechenden Sulfonsäurehalogenid.

Die als Ausgangsstoffe verwendeten Verbindungen der allgemeinen Formel VIII, IX, X, XII, XIV und XVI erhält man durch Alkylierung einer entsprechenden Verbindung mit einer zu einer Verbindung der allgemeinen Formel III analogen Verbindung.

Die neuen Verbindungen der allgemeinen Formel I und deren physiologisch vertragliche Additionssalze weisen wertvolle pharmakologische Eigenschaften auf. Sie stellen AngiotensinAntagonisten, insbesondere Angiotensin-II-Antagonisten, dar.

Beispielsweise wurden die Verbindungen

A = 2-n-Propyl-4-methyl-1-[4-[(α-carboxy)benzyloxy]benzyl]benzimidazol,

B = 2-n-Butyl-1-[4-[(α-carboxy)benzyloxy]benzyl]-6-dimethylaminocarbonylamino-benzimidazol,

C = 2-n-Propyl-6-(1-methyl-benzimidazol-2-yl)-4-methyl-1-[4-[(α-carboxy)benzyloxy]benzyl]-benzimidazol,

D = 2-Methyl-4-[4'-[(α-carboxy)benzyloxy]benzyloxy]chinolin,

E = 2-n-Butyl-8-methyl-3-[4-[(α-carboxy)benzyloxy]benzyl]chinazolin-4-on-semihydrat,

F = 2-n-Propyl-4-methyl-6-(1-methyl-benzimidazol-2-yl)-1-[4-[α-(1H-tetrazol-5-yl)benzyloxy]benzyl]-benzimidazol,

G = 2-n-Butyl-6-(N-propionyl-methylamino)-1-[4-[(1-carboxy-3-methyl)butyloxy]benzyl]benzimidazol,

H = 2-n-Butyl-5-methyl-6-(3-benzyl-3,4,5,6-tetrahydro-2(1H)-pyrimidinon-1-yl)-3-[4-[α-(1H-tetrazol-5-yl)benzyloxy]benzyl]imidazol[4,5-b]pyridin und

I = 2-n-Butyl-1-[4-[(α-(α-ethyl-phosphono)benzylamino]benzyl]benzimidazol

auf ihre biologischen Wirkungen wie folgt untersucht:

<u>Methodenbeschreibung Angiotensin II-Rezentorbindung</u>

Das Gewebe (Rattenlunge) wird in Tris-Puffer (50 mMol Tris, 150 mMol NaCl, 5 mMol EDTA, pH 7.40) homogenisiert und zweimal je 20 Min. bei 20.000 x g zentrifugiert. Das endgültige Pellet wird in Inkubations-Puffer (50 mMol Tris, 5 mMol $MgCl_2$, 0,2 % BSA, pH 7,40) 1:75, bezogen auf das Feuchtgewicht des Gewebes, resuspendiert. Je 0,1 ml Homogenat wird für 60 Min. bei 37°C mit 50 pM [$^{125}$I]-Angiotensin II (NEN, Dreieich, FRG) und steigenden Konzentrationen der Testsubstanz in einem Gesamtvolumen von 0,25 ml inkubiert. Die Inkubation wird durch rasche Filtration durch Glasfiber-Filtermatten beendet. Die Filter werden je 4 ml eiskaltem Puffer (25 mMol Tris, 2.5 mMol $MgCl_2$, 0,1 % BSA, pH 7,40) gewaschen. Die gebundene Radioaktivität wird in einem Gamma-Counter ermittelt. Aus der Dosis-Wirkungskurve wird der entsprechende $IC_{50}$-Wert ermittelt.

Die Substanzen A bis I zeigen in dem beschriebenen Test folgende $IC_{50}$-Werte:

| Substanz | $IC_{50}$ [nM] |
|---|---|
| A | 76 |
| B | 8 |
| C | 4 |
| D | 2000 |
| E | 2000 |
| F | 12 |
| G | 830 |
| H | 27 |
| I | 560 |

Desweiteren konnten bei der Applikation der vorstehenden Verbindungen bis zu einer Dosis von 30 mg/kg i.v. keine toxischen Nebenwirkungen, z.B. keine negativ inotrope Wirkung und keine Herzrhythmusstörungen, beobachtet werden. Die Verbindungen sind demnach gut vertraglich.

Aufgrund ihrer pharmakologischen Eigenschaften eignen sich die neuen Verbindungen und deren physiologisch vertragliche Additionssalze zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen.

Weiterhin eignen sich die neuen Verbindungen und deren physiologisch verträgliche Additionssalze zur Behandlung pulmonarer Erkrankungen, z.B. von Lungenödemen und der chronischen Bronchitis, zur Prävention von arterieller Re-Stenosis nach Angioplastie, von Verdickungen der Gefäßwand nach Gefäßoperationen, der Arteriosklerose und der diabetischen Angiopathie. Auf Grund der Beeinflußung der Acetylcholin- und Dopamin-Freisetzung durch Angiotensin im Gehirn eignen sich die neuen Angiotensin-Antagonisten auch zur Behebung zentralnervöser Störungen, z.B. von Depressionen, der Alzheimer'-schen Krankheit, des Parkinson-Syndroms, der Bulimie, sowie von Störungen kognitiver Funktionen.

Die zur Erzielung einer entsprechenden Wirkung erforderliche Dosierung beträgt zweckmäßigerweise bei intravenöser Gabe 20 bis 100 mg, vorzugsweise 30 bis 70 mg, und bei oraler Gabe 50 bis 200 mg, vorzugsweise 75 bis 150 mg, jeweils 1 bis 3 x täglich. Hierzu lassen sich die erfindungsgemäß hergestellten Verbindungen der allgemeinen Formel I, gegebenenfalls in Kombination mit anderen Wirksubstanzen wie z.B. Blutdrucksenker, Diuretika und/oder Kalzium-Antagonisten, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Für die oben erwähnten Kombinationen kommen somit als weitere Wirksubstanzen beispielsweise Bendroflumethiazid, Chlorthiazid, Hydrochlorthiazid, Spironolacton, Benzthiazid, Cyclothiazid, Ethacrinsäure, Furosemid, Metoprolol, Prazosin, Atenolol, Propranolol, (Di)hydralazin-hydrochlorid, Diltiazem, Felodipin, Nicardipin, Nifedipin, Nisoldipin und Nitrendipin in Betracht. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 15 bis 200 mg Hydrochlorthiazid, 125 bis 2000 mg Chlorthiazid, 15 bis 200 mg Ethacrinsäure, 5 bis 80 mg Furosemid, 20 bis 480 mg Propranolol, 5 bis 60 mg Felodipin, 5 bis 60 mg Nifedipin oder 5 bis 60 mg Nitrendipin.

Die nachfolgenden Beispiele sollen die Erfindung näher erläutern:

Beispiel A

4-(α-Ethoxycarbonyl-benzyloxy)benzylbromid

a) 2-Brom-phenylessigsäureethylester

43,0 g (0,2 Mol) DL-2-Brom-phenylessigsäure werden in 400 ml Ethanol bei Raumtemperatur unter Rühren gelöst. Bei 5-10°C werden unter Eiskühlung 11,8 g (0,2 Mol) Thionylchlorid langsam zugetropft. Nach 12 Stunden bei Raumtemperatur wird vom Solvens abdestilliert und der Rückstand in Essigester aufgenommen. Nach Extraktion mit gesättigter NatriumbicarbonatLösung und mit gesättigter Kochsalzlösung wird über Natriumsulfat getrocknet und eingeengt.
Ausbeute: 41,85 g (86 % der Theorie),
$R_f$-Wert: 0,60 (Kieselgel; Laufmittel: Essigester/Petrolether = 1:19)

b) 4-(α-Ethoxycarbonyl-benzyloxy)benzylalkohol

41,8 g (0,172 Mol) 2-Brom-phenylessigsäureethylester und 21,3 g (0,172 Mol) 4-Hydroxybenzylalkohol werden in 850 ml Aceton gelöst und mit 24,8 g (0,18 Mol) Kaliumcarbonat und 5,0 g (0,03 Mol) Kaliumjodid versetzt. Die Reaktionsmischung wird 60 Stunden unter Rühren zum Rückfluß erhitzt. Anschließend werden die anorganischen Salze abfiltriert, und der Rückstand wird mit heißem Aceton gewaschen. Das Filtrat wird eingeengt und der Rückstand über eine Kieselgelsäule (Korngröße: 0,063-0,02 mm) gereinigt, wobei als Elutionsmittel anfangs Petrolether, danach Gemische von Petrolether und Essigester mit steigender Polarität (9:1, 8:2 und 7:3) verwendet werden. Die einheitlichen Fraktionen werden im Vakuum eingeengt.
Ausbeute: 30,65 g (62,5 % der Theorie),
$R_f$-Wert: 0,40 (Kieselgel; Laufmittel: Essigester/Petrolether = 3:7)

c) 4-(α-Ethoxycarbonyl-benzyloxy)benzylbromid

28,6 g (0,1 Mol) 4-(α-Ethoxycarbonyl-benzyloxy)benzylalkohol werden in 300 ml Dichlormethan gelöst und mit 31,6 g (0,12 Mol) Triphenylphosphin versetzt. Unter Eiskühlung wird eine Lösung von 40,0 g (0,12 Mol) Tetrabromkohlenstoff in 100 ml Dichlormethan zugetropft. Die Mischung wird 30 Minuten bei Raumtemperatur gerührt und anschließend eingeengt. Der Rückstand wird über eine Kieselgelsäule (Korngröße: 0,063-0,02 mm) gereinigt, wobei als Elutionsmittel anfangs Petrolether, danach Gemische von Petrolether und Essigester mit steigender Polarität (9:1 und 8:2) verwendet werden. Die einheitlichen Fraktionen werden im Vakuum eingeengt.
Ausbeute: 20,45 g (59 % der Theorie),
Öl, $R_f$-Wert: 0,70 (Kieselgel; Laufmittel: Essigester/Petrolether = 1:4)

| $C_{17}H_{16}BrO_3$ (349,24) | | | | | | |
|---|---|---|---|---|---|---|
| Ber.:<br>Gef.: | C | 58,20<br>58,23 | H | 4,95<br>4,84 | Br | 22,80<br>23,12 |

Beispiel 1

2-n-Propyl-4-methyl-1-[4-[(α-carboxy)benzyloxy]benzyl]benzimidazol

a) 2-n-Propyl-4-methyl-1-[4-[(α-ethoxycarbonyl)benzyloxy]benzyl[benzimidazol

Zu einer Lösung von 220 mg Kalium-tert.butylat in 30 ml Dimethylsulfoxid werden 350 mg (2,0 nMol) 2-n-Propyl-4-methylbenzimidazol (hergestellt analog Chemistry of Heterocyclic Compounds, Vol. 40, Part 1, 6-12) zugegeben. Nach 30 Minuten bei Raumtemperatur wird eine Lösung von 700 mg (2,0 mMol) 4-(α-Ethoxycarbonyl-benzyloxy)benzylbromid in 5 ml Dimethylsulfoxid zugetropft. Nach 12 Stunden bei Raumtemperatur wird die Reaktionslösung in Eiswasser eingerührt und 3 x mit je 100 ml Essigester extrahiert. Die vereinigten organischen Phasen werden mit 100 ml Kochsalzlösung gewaschen und über Natriumsulfat getrocknet. Nach Abziehen des Solvens wird der Rückstand über eine Kieselgelsäule (Korngröße: 0,063-0,02 mm) gereinigt, wobei als Elutionsmittel anfangs Methylenchlorid, danach Gemische von Methylenchlorid und Ethanol mit steigender Polarität (50:1 und 25:1) verwendet werden. Die einheitlichen Fraktionen werden im Vakuum eingeengt.
Ausbeute: 500 mg (55 % der Theorie),
Öl, $R_f$-Wert: 0,55 (Kieselgel; Laufmittel: Methylenchlorid/Methanol = 19:1)

b) 2-n-Propyl-4-methyl-1-[4-[(α-carboxy)benzyloxy]benzyl]benzimidazol

500 mg (1,1 mMol) 2-n-Propyl-4-methyl-1-[4-[(α-ethoxycarbonyl)benzyloxy]benzyl]benzimidazol werden in 10 ml Ethanol gelöst und mit 10 ml 1 N Natronlauge versetzt. Die Mischung wird 30 Minuten bei Raumtemperatur gerührt. Anschließend wird die Mischung eingeengt und in Wasser aufgenommen. Durch

Zusatz von Eisessig wird die Lösung angesäuert. Der dabei anfallende Niederschlag wird abgesaugt, mit Wasser neutral gewaschen und getrocknet. Das Rohprodukt wird in Methylenchlorid aufgenommen und über eine Kieselgelsäule (Korngröße: 0,063-0,02 mm) gereinigt, wobei als Elutionsmittel ein Gemisch von Methylenchlorid und Methanol (19:1) verwendet wird. Die einheitlichen Fraktionen werden im Vakuum eingeengt, der Rückstand wird mit Hexan/Ether verrieben und abgesaugt.
Ausbeute: 60 mg (13 % der Theorie),
Schmelzpunkt: amorph
$C_{26}H_{26}N_2O_3$ (414,51)
Massenspektrum: $(M + H)^+ = 415$

Beispiel 2

2-n-Butyl-1-[4-[(α-carboxy)benzyloxy]benzyl]-6-propionylamino-benzimidazol-hydrochlorid

a) 2-n-Butyl-1-[4-[(α-ethoxycarbonyl)benzyloxy]benzyl]-6-nitro-benzimidazol und 2-n-Butyl-1-[4-[(α-ethoxycarbonyl)benzyloxy]benzyl]-5-nitro-benzimidazol

Hergestellt analog Beispiel 1a aus 2-n-Butyl-5-nitro-benzimidazol (hergestellt analog Chemistry of Heterocyclic Compounds, Vol. 40, Part 1, 6-12) und 4-[(α-Ethoxycarbonyl)benzyloxy]benzylbromid.
Ausbeute: 70,5 % der Theorie,
Öl, $R_f$-Wert: 0,25 (Kieselgel; Laufmittel: Methylenchlorid/Ethanol = 50:1)

b) 2-n-Butyl-6-amino-1-[4-[(α-ethoxycarbonyl)benzyloxy]benzyl]-benzimidazol

Hergestellt durch katalytische Hydrierung von 2-n-Butyl-1-[4-[(α-ethoxycarbonyl)benzyloxy]benzyl]-5/6-nitro-benzimidazol in Gegenwart von Raney-Nickel in Ethanol bei 50°C unter 5 bar Wasserstoffdruck und anschließender chromatographischer Abtrennung des 5-Amino-2-n-butyl-1-[4-[(α-ethoxycarbonyl)benzyloxy]-benzyl]benzimidazols.
Ausbeute: 21,8 % der Theorie,
Öl, $R_f$-Wert: 0,30 (Kieselgel; Laufmittel: Petrolether/Methylenchlorid/Ethanol = 7:2:1)

c) 2-n-Butyl-6-propionylamino-1-[4-[(α-ethoxycarbonyl)benzyloxy]benzyl]benzimidazol

Hergestellt aus 2-n-Butyl-6-amino-1-[4-[(α-ethoxycarbonyl)benzyloxy]benzyl]benzimidazol und Propionylchlorid/Pyridin.
Ausbeute: 70,2 % der Theorie,
Schmelzpunkt: 207-209°C

d) 2-n-Butyl-1-[4-[(α-carboxy)benzyloxy]benzyl]-6-propionylamino-benzimidazol-hydrochlorid

Hergestellt analog Beispiel 1b aus 2-n-Butyl-6-propionylamino-1-[4-[(α-ethoxycarbonyl)benzyloxy]-benzyl]benzimidazol und 1 N Natronlauge in Ethanol.
Ausbeute: 43,5 % der Theorie,
Schmelzpunkt: sintert ab 93°C

| $C_{29}H_{31}N_3O_4$ x HCl (522,67) | | | | | | |
|---|---|---|---|---|---|---|
| Ber.:<br>Gef.: | C | 66,50<br>66,73 | H | 6,12<br>6,34 | N | 8,08<br>7,81 |

Beispiel 3

2-n-Butyl-1-[4-[(α-carboxy)benzyloxy]benzyl]-6-dimethylaminocarbonylamino-benzimidazol

Hergestellt analog Beispiel 1b aus 2-n-Butyl-1-[4-[(α-ethoxycarbonyl)benzyloxy]benzyl]-6-dimethylaminocarbonylamino-benzimidazol und 2 N Natronlauge in Ethanol.
Ausbeute: 80 % der Theorie,

Schmelzpunkt: 183-185 ° C

| $C_{29}H_{32}N_4O_4$ (500,60) | | | | | | |
|---|---|---|---|---|---|---|
| Ber.:<br>Gef.: | C | 69,59<br>69,54 | H | 6,44<br>6,43 | N | 11,19<br>10,66 |

Beispiel 4

2-n-Butyl-1-[4-[(α-carboxy)benzyloxy]benzyl]-6-cyclohexyl aminocarbonylamino-benzimidazol-hydrochlorid

a) 2-n-Butyl-1-[4-[(α-ethoxycarbonyl)benzyloxy]benzyl]-6-

cyclohexylaminocarbonylamino-benzimidazol

Hergestellt aus 2-n-Butyl-1-[4-[(α-ethoxycarbonyl)benzyloxy]benzyl]-6-amino-benzimidazol und Cyclohexylisocyanat/Triethylamin in Tetrahydrofuran.
Ausbeute: 65,4 % der Theorie,
Öl, $R_f$-Wert: 0,40 (Kieselgel; Laufmittel: Methylenchlorid/Ethanol = 19:1)

b) 2-n-Butyl-1-[4-[(α-carboxy)benzyloxy]benzyl]-6-cyclo-hexylaminocarbonylamino-benzimidazol-hydrochlorid

Hergestellt analog Beispiel 1b aus 2-n-Butyl-1-[4-[(α-ethoxycarbonyl)benzyloxy]benzyl)-6-cyclohexylaminocarbonylamino-benzimidazol und 1 N Natronlauge in Ethanol.
Ausbeute: 40,7 % der Theorie,
Schmelzpunkt: sintert ab 184 ° C

| $C_{33}H_{38}N_4O_4$ x HCl (591,17) | | | | | | |
|---|---|---|---|---|---|---|
| Ber.:<br>Gef.: | C | 67,00<br>67,15 | H | 6,60<br>6,71 | N | 9,45<br>9,30 |

Beispiel 5

2-n-Butyl-1-[4-[(α-carboxy)benzyloxy]benzyl]-5-cyclohexylaminocarbonylamino-benzimidazol-hydrochlorid

Hergestellt analog Beispiel 1b aus 2-n-Butyl-1-[4-[(α-ethoxycarbonyl)benzyloxy]benzyl]-5-cyclohexylaminocarbonylamino-benzimidazol und 1 N Natronlauge in Ethanol.
Ausbeute: 46,1 % der Theorie,
Schmelzpunkt: sintert ab 196 ° C

| $C_{33}H_{38}N_4O_4$ x HCl (591,17) | | | | | | |
|---|---|---|---|---|---|---|
| Ber.:<br>Gef.: | C | 67,00<br>67,22 | H | 6,60<br>6,74 | N | 9,45<br>9,49 |

Beispiel 6

2-n-Butyl-1-[4-[(α-carboxy)benzyloxy]benzyl]-6-(3,4,5,6-tetrahydro-2(1H)-pyrimidinon-1-yl)-benzimidazol

Hergestellt analog Beispiel 1b aus 2-n-Butyl-1-[4-[(α-ethoxycarbonyl)benzyloxy]benzyl]-6-(3,4,5,6-tetrahydro-2(1H)-pyrimidinon-1-yl)-benzimidazol und 1 N Natronlauge in Ethanol.
Ausbeute: 8,8 % der Theorie,

Schmelzpunkt: 260-262 ° C
$C_{30}H_{32}N_4O_4$ (512,61)
Massenspektrum: $(M + H)^+$ = 513

Beispiel 7

2-n-Butyl-1-[4-[(α-carboxy)benzyloxy]benzyl]-6-(3-benzyl-3,4,5,6-tetrahydro-2(1H)-pyrimidinon-1-yl)-benzimidazol

Hergestellt analog Beispiel 1b aus 2-n-Butyl-1-[4-[(α-ethoxycarbonyl)benzyloxy]benzyl]-6-(3-benzyl-3,4,5,6-tetrahydro-2(1H)-pyrimidinon-1-yl)-benzimidazol und 1 N Natronlauge in Ethanol.
Ausbeute: 80,4 % der Theorie,
Schmelzpunkt: 124-126 ° C

| $C_{37}H_{38}N_4O_4$ (602,74) | | | | | | |
|---|---|---|---|---|---|---|
| Ber.:<br>Gef.: | C | 73,73<br>73,47 | H | 6,35<br>6,50 | N | 9,30<br>9,05 |

Beispiel 8

2-n-Butyl-1-[4-[(α-carboxy)benzyloxy]benzyl]-6-(N-cyclohexylaminocarbonyl-methylamino)-benzimidazol

a) 2-n-Butyl-1-[4-[(α-ethoxycarbonyl)benzyloxy]benzyl]6-(N-cyclohexylaminocarbonyl-methylamino)-benzimidazol

Hergestellt analog Beispiel 1a aus 2-n-Butyl-6-(N-cyclohexylaminocarbonyl-methylamino)benzimidazol und 4-[(α-Ethoxycarbonyl)-benzyloxy]benzylbromid und anschließender chromatographischer Abtrennung des 2-n-Butyl-1-[4-[(α-ethoxycarbonyl)benzyloxy]benzyl]-5-(N-cyclohexylaminocarbonyl-methylamino)-benzimidazols.
Ausbeute: 50,0 % der Theorie,
Öl, $R_f$-Wert: 0,50 (Kieselgel; Laufmittel: Methylethylketon/Xylol = 1:1)

b) 2-n-Butyl-1-[4-[(α-carboxy)benzyloxylbenzyl]-6-(N-cyclohexylaminocarbonyl-methylamino)benzimidazol

Hergestellt analog Beispiel 1b aus 2-n-Butyl-1-[4-[(α-ethoxycarbonyl)benzyloxy]benzyl]-6-(N-cyclohexylaminocarbonylmethylamino)benzimidazol und 1 N Natronlauge in Ethanol.
Ausbeute: 93,0 % der Theorie,
Schmelzpunkt: 168-169 ° C

| $C_{34}H_{40}H_4O_4$ (568,72) | | | | | | |
|---|---|---|---|---|---|---|
| Ber.:<br>Gef.: | C | 71,81<br>71,78 | H | 7,09<br>7,02 | N | 9,85<br>9,71 |

Beispiel 9

2-n-Propyl-5-(2-methyl-propionylamino)-3-[4-[(α-carboxy)benzyloxy]benzyl]imidazo[4,5-b]pyridin

a) 2-n-Propyl-5-(2-methyl-propionylamino)-imidazo[4,5-b]pyridin

2,62 g (15 mMol) 2-n-Propyl-5-amino-imidazo[4,5-b]pyridin werden in 100 ml absolutem Methylenchlorid suspendiert und unter Rühren und Eiskühlung mit 3,16 g (30 mMol) Isobuttersäurechlorid versetzt. Anschließend wird bei -5 ° C eine Lösung von 3,03 g (30 mMol) Triethylamin in 5 ml Methylenchlorid zugetropft. Nach einer Stunde bei Raumtemperatur wird das Reaktionsgemisch mit 100 ml 2 N Salzsäure

versetzt und eine weitere Stunde gerührt. Danach wird die Lösung auf Eiswasser gegossen und mit gesättigter Natriumhydrogencarbonatlösung versetzt. Die wässrige Phase wird 3 x mit je 100 ml Essigester extrahiert, die vereinigten organischen Phasen werden anschließend mit Kochsalzlösung gewaschen, über Natriumsulfat getrocknet und eingeengt. Das Rohprodukt wird in Methylenchlorid aufgenommen und über eine Kieselgelsäule (Korngröße: 0,063-0,2 mm) gereinigt, wobei als Elutionsmittel anfangs Methylenchlorid, später Gemische von Methylenchlorid und Ethanol mit steigender Polarität (25:1 und 19:1) verwendet werden. Die einheitlichen Fraktionen werden eingeengt, der Rückstand wird mit Petrolether/Ether = 1:1 verrieben und abgesaugt.
Ausbeute: 2,05 g (56 % der Theorie),
Schmelzpunkt: 206 ° C

| $C_{13}H_{18}N_4O$ (246,30) | | | | | | |
|---|---|---|---|---|---|---|
| Ber.:<br>Gef.: | C | 63,39<br>63,64 | H | 7,37<br>7,57 | N | 22,75<br>22,98 |

b) 2-n-Propyl-5-(2-methyl-propionylamino)-3-[4-[(α-ethoxycarbonyl)benzyloxylbenzyl]imidazo[4.5-b]pyridin

Hergestellt analog Beispiel 1a aus 2-n-Propyl-5-(2-methylpropionylamino)imidazo[4,5-b]pyridin und 4-[-(α-Ethoxycarbonyl)benzyloxy]benzylbromid.
Ausbeute: 33,7 % der Theorie,
Schmelzpunkt: 100-101 ° C

c) 2-n-Propyl-5-(2-methyl-propionylamino)-3-[4-[(α-carboxy)benzyloxy]benzyl]imidazo[4,5-b]pyridin

Hergestellt analog Beispiel 1b aus 2-n-Propyl-5-(2-methylpropionylamino)-3-[4-[(α-ethoxycarbonyl)-benzyloxy]benzyl]imidazo[4,5-b]pyridin und 1 N Natronlauge in Ethanol. Ausbeute: 52,0 % der Theorie,
Schmelzpunkt: 134 ° C

| $C_{28}H_{30}N_4O_4$ (486,58) | | | | | | |
|---|---|---|---|---|---|---|
| Ber.:<br>Gef.: | C | 69,12<br>69,03 | H | 6,21<br>6,11 | N | 11,51<br>11,36 |

Beispiel 10

2-n-Butyl-5-valeroylamino-3-[4-[(α-carboxy)benzyloxy]benzyl]imidazo[4,5-b]pyridin-semihydrat

Hergestellt analog Beispiel 1b aus 2-n-Butyl-5-valeroylamino-3-[4-[(α-ethoxycarbonyl)benzyloxy]benzyl]-imidazo-[4,5-b]pyridin und 1 N Natronlauge in Ethanol.
Ausbeute: 57,0 % der Theorie,
Schmelzpunkt: sintert ab 96 ° C

| $C_{30}H_{34}N_4O_4$ x 0,5 $H_2O$ (532,65) | | | | | | |
|---|---|---|---|---|---|---|
| Ber.:<br>Gef.: | C | 68,82<br>68,99 | H | 6,74<br>6,67 | N | 10,70<br>10,58 |

Beispiel 11

2-n-Propyl-5-(1-methyl-benzimidazol-2-yl)-7-methyl-3-[4-[(α-carboxy)benzyloxy]benzyl]imidazo[4,5-b]pyridin

Hergestellt analog Beispiel 1b aus 2-n-Propyl-5-(1-methyl-benzimidazol-2-yl)-7-methyl-3-[4-[(α-ethoxycarbonyl)benzyl]oxy]benzyl-imidazo[4,5-b]pyridin und 1 N Natronlauge in Ethanol.

Ausbeute: 41,4 % der Theorie,
Schmelzpunkt: 242-244 ° C

| $C_{33}H_{31}N_5O_3$ (545,65) | | | | | | |
|---|---|---|---|---|---|---|
| Ber.:<br>Gef.: | C | 72,64<br>72,51 | H | 5,73<br>5,75 | N | 12,83<br>12,97 |

Beispiel 12

2-n-Propyl-6-(1-methyl-benzimidazol-2-yl)-4-methyl-1-[4-[(α-carboxy)benzyloxy]benzyl]benzimidazol

Hergestellt analog Beispiel 1b aus 2-n-Propyl-6-(1-methyl-benzimidazol-2-yl)-4-methyl-1-[4-[(α-ethoxycarbonyl)benzyloxy]benzyl]benzimidazol und 1 N Natronlauge in Ethanol.
Ausbeute: 41,0 % der Theorie,
Schmelzpunkt: amorph

| $C_{34}H_{32}N_4O_3$ (544,66) | | | | | | |
|---|---|---|---|---|---|---|
| Ber.:<br>Gef.: | C | 74,98<br>74,55 | H | 5,92<br>6,06 | N | 10,28<br>10,08 |

Massenspektrum: $(M + H)^+ = 545$

Beispiel 13

2-Methyl-4-[4'-[(α-carboxy)benzyloxy]benzyloxy]chinolin

a) 2-Methyl-4-[4'-[(α-ethoxycarbonyl)benzyloxy]benzyloxy]chinolin

Hergestellt analog Beispiel 1a aus 4-Hydroxy-chinaldin und 4-[(α-Ethoxycarbonyl)benzyloxy]-benzylbromid.
Ausbeute: 19,8 % der Theorie,
Öl, $R_f$-Wert: 0,55 (Kieselgel; Laufmittel: Methylenchlorid/Ethanol = 19:1)

b) 2-Methyl-4-[4'-[(α-carboxy)benzyloxy]benzyloxy]chinolin

Hergestellt analog Beispiel 1b aus 2-Methyl-4-[4'-[(α-ethoxycarbonyl)benzyloxy]benzyloxy-chinolin und 1 N Natronlauge in Ethanol.
Ausbeute: 66,6 % der Theorie,
Schmelzpunkt: 142-143 ° C

| $C_{25}H_{21}NO_4$ (399,50) | | | | | | |
|---|---|---|---|---|---|---|
| Ber.:<br>Gef.: | C | 73,17<br>73,56 | H | 5,30<br>5,25 | N | 3,51<br>3,34 |

Massenspektrum: $(M + H)^+ = 400$

Beispiel 14

2-n-Propyl-5-n-butyrylamino-3-[4-[(1-carboxy-3-methyl)butyloxy]benzyl]imidazo[4,5-b]pyridin

a) 2-n-Propyl-5-n-butyrylamino-3-[4-[(1-methoxycarbonyl-3-methyl)butyloxy]benzyl]imidazo[4 5-b]pyridin

Hergestellt analog Beispiel 1a aus 2-n-Propyl-5-n-butyrylamino-imidazo[4,5-b]pyridin und 4-[(1-Methoxycarbonyl-3-methyl)butyloxy]benzylbromid, und anschließender chromatographischer Abtrennung des 2-n-Propyl-5-n-butyrylamino-1-[4-[(1-methoxycarbonyl-3-methyl)butyloxy]benzyl)imidazo[4,5-b]pyridins.
Ausbeute: 47,0 % der Theorie,
Öl, $R_f$-Wert: 0,30 (Kieselgel; Laufmittel: Methylenchlorid/Ethanol = 19:1)

b) 2-n-Propyl-5-n-butyrylamino-3-[4-(1-carboxy-3-methyl)butyloxy]benzyl]imidazo[4,5-b]pyridin

Hergestellt analog Beispiel 1b aus 2-n-Propyl-5-n-butyrylamino-3-[4-(1-methoxycarbonyl-3-methyl)-butyloxy]benzyl]imidazo[4,5-b]pyridin und 1 N Natronlauge in Ethanol.
Ausbeute: 59,0 % der Theorie,
Schmelzpunkt: 147-148 °C

| $C_{26}H_{34}N_4O_4$ (466,59) | | | | | | |
|---|---|---|---|---|---|---|
| Ber.:<br>Gef.: | C | 66,93<br>66,69 | H | 7,35<br>7,48 | N | 12,01<br>11,85 |

Beispiel 15

2-n-Butyl-1-[4-[(α-carboxy)cyclohexylmethyloxy)benzyl]benzimidazol

Hergestellt analog Beispiel 1b aus 2-n-Butyl-1-[4-[(α-methoxycarbonyl)cyclohexylmethyloxy]benzyl]-benzimidazol und 1 N Natronlauge in Ethanol.
Ausbeute: 92,0 % der Theorie,
Schmelzpunkt: 208 °C (Zers.)

| $C_{26}H_{32}N_2O_3$ (420,56) | | | | | | |
|---|---|---|---|---|---|---|
| Ber.:<br>Gef.: | C | 74,25<br>73,98 | H | 7,66<br>7,52 | N | 6,66<br>6,41 |

Beispiel 16

2-n-Butyl-4-chlor-5-hydroxymethyl-1-[4-[(α-carboxy)benzyloxy]benzyl]imidazol

a) 2-n-Butyl-4-chlor-5-hydroxymethyl-1-[4-[(α-methoxycarbonyl)benzyloxy[benzyl]imidazol

Hergestellt analog Beispiel 1a aus 2-n-Butyl-4-chlor-5-hydroxymethyl-imidazol und 4-[(α-Methoxycarbonyl)benzyloxy]benzylbromid, und anschließender chromatographischer Abtrennung des 2-n-Butyl-5-chlor-4-hydroxymethyl-1-[4-[(α-methoxycarbonyl)benzyloxy]benzyl]imidazols.
Ausbeute: 21,0 % der Theorie,
Öl, $R_f$-Wert: 0,70 (Kieselgel; Laufmittel: Methylenchlorid/Methanol = 6:1)

b) 2-n-Butyl-4-chlor-5-hydroxymethyl-1-[4-[(α-carboxy)benzyloxy]benzyl]imidazol-hydrochlorid

Hergestellt analog Beispiel 1b aus 2-n-Butyl-4-chlor-5-hydroxymethyl-1-[4-[(α-methoxycarbonyl)-benzyloxy]benzyl]imidazol und 1 N Natronlauge in Ethanol.
Ausbeute: 34,0 % der Theorie,

Schmelzpunkt: 176 ° C

| $C_{23}H_{25}ClH_2O_4$ x HCl (465,38) | | | | | | |
|---|---|---|---|---|---|---|
| Ber.:<br>Gef.: | C | 59,36<br>59,53 | H | 5,63<br>5,43 | N | 6,02<br>5,97 |

Beispiel 17

2-n-Butyl-5-chlor-4-hydroxymethyl-1-[4-[(1-carboxy-3-methyl)butyloxy]benzyl]imidazol

Hergestellt analog Beispiel 1b aus 2-n-Butyl-5-chlor-4-hydroxymethyl-1-[4-[(1-methoxycarbonyl-3-methyl)butyloxy]benzyl]imidazol und 1 N Natronlauge in Ethanol.
Ausbeute: 66,0 % der Theorie,
Schmelzpunkt: 175-176 ° C

| $C_{21}H_{29}ClN_2O_4$ (408,93) | | | | | | |
|---|---|---|---|---|---|---|
| Ber.:<br>Gef.: | C | 61,68<br>61,34 | H | 7,14<br>7,11 | N | 6,84<br>6,58 |

Beispiel 18

2-n-Butyl-5-chlor-4-hydroxymethyl-1-[4-[(1-carboxy)-n-propyloxy]benzyl]imidazol

Hergestellt analog Beispiel 1b aus 2-n-Butyl-5-chlor-4-hydroxymethyl-1-[4-[(1-methoxycarbonyl)-n-propyloxy]benzyl]imidazol und 1 N Natronlauge in Ethanol.
Ausbeute: 70,0 % der Theorie,
Schmelzpunkt: 182-184 ° C

| $C_{19}H_{25}ClN_2O_4$ (380,88) | | | | | | |
|---|---|---|---|---|---|---|
| Ber.:<br>Gef.: | C | 59,91<br>59,67 | H | 6,61<br>6,53 | N | 7,35<br>7,22 |

Beispiel 19

2-n-Butyl-4-chlor-5-hydroxymethyl-1-[4-[(1-carboxy)-n-propyloxy]benzyl]imidazol

Hergestellt analog Beispiel 1b aus 2-n-Butyl-4-chlor-5-hydroxymethyl-1-[4-[(1-methoxycarbonyl)-n-propyloxy)benzyl]imidazol und 1 N Natronlauge in Ethanol.
Ausbeute: 66,0 % der Theorie,
Schmelzpunkt: 125-127 ° C

| $C_{19}H_{25}ClN_2O_4$ (380,88) | | | | | | |
|---|---|---|---|---|---|---|
| Ber.:<br>Gef.: | C | 59,91<br>59,74 | H | 6,61<br>6,60 | N | 7,35<br>6,90 |

Beispiel 20

2-n-Butyl-4-chlor-5-methoxymethyl-1-[4-[(α-carboxy)benzyloxy]benzyl]imidazol

Hergestellt analog Beispiel 1b aus 2-n-Butyl-4-chlor-5-methoxymethyl-1-[4-[(α-methoxycarbonyl)-benzyloxy]benzyl]-imidazol und 1 N Natronlauge in Ethanol.
Ausbeute: 45,0 % der Theorie,
Schmelzpunkt: 156-158 ° C

| $C_{24}H_{27}ClN_2O_4$ (442,95) | | | | | | |
|---|---|---|---|---|---|---|
| Ber.:<br>Gef.: | C | 65,08<br>64,70 | H | 6,14<br>6,38 | N | 6,32<br>6,03 |

Beispiel 21

2-n-Butyl-4-chlor-5-hydroxymethyl-1-[4-[(1-carboxy-3-methyl)butyloxy]benzyl]imidazol

Hergestellt analog Beispiel 1b aus 2-n-Butyl-4-chlor-5-hydroxy-methyl-1-[4-[(1-methoxycarbonyl-3-methyl)butyloxy]benzyl]-imidazol und 1 N Natronlauge in Ethanol.
Ausbeute: 57,0 % der Theorie,
Schmelzpunkt: 164-165 ° C

| $C_{21}H_{29}ClN_2O_4$ (408,93) | | | | | | |
|---|---|---|---|---|---|---|
| Ber.:<br>Gef.: | C | 61,68<br>61,63 | H | 7,14<br>7,09 | N | 6,84<br>6,71 |

Beispiel 22

2-n-Butyl-4-hydroxymethyl-5-chlor-1-[4-[(α-carboxy)benzyloxy]-benzyl]imidazol

Hergestellt analog Beispiel 1b aus 2-n-Butyl-4-hydroxymethyl-5-chlor-1-[4-[(α-methoxycarbonyl)-benzyloxy]-benzyl]imidazol und 1 N Natronlauge in Ethanol.
Ausbeute: 18,0 % der Theorie,
Schmelzpunkt: 193 ° C
$C_{23}H_{25}ClN_2O_4$ (428,92) $R_f$-Wert: 0,10 (Kieselgel; Laufmittel: Methylenchlorid/Methanol = 9:1)
Massenspektrum: $(M + H)^+$ = 394/396 (Cl)

Beispiel 23

2-n-Butyl-5-chlor-4-hydroxymethyl-1-[4-[(1-carboxy)-n-butyloxy]benzyl]imidazol

Hergestellt analog Beispiel 1b aus 2-n-Butyl-5-chlor-4-hydroxymethyl-1-[4-[(1-methoxycarbonyl)-n-butyloxy]benzyl]imidazol und 1 N Natronlauge in Ethanol.
Ausbeute: 27,0 % der Theorie,
Schmelzpunkt: 154 ° C
$C_{20}H_{27}ClN_2O_4$ (394,90)
$R_f$-Wert: 0,20-0,30 (Kieselgel; Laufmittel: Methylenchlorid/Methanol = 9:1)
Massenspektrum: $(M + H)^+$ = 394/396 (Cl)

Beispiel 24

2-n-Butyl-4-hydroxymethyl-5-chlor-1-[4-[(α-carboxy)-p-phenyl-benzyloxy]benzyl]imidazol

Hergestellt analog Beispiel 1b aus 2-n-Butyl-4-hydroxymethyl-5-chlor-1-[4-[(α-methoxycarbonyl)-p-phenyl-benzyloxy]ben zyl]imidazol und 1 N Natronlauge in Ethanol.
Ausbeute: 72,0 % der Theorie,
Schmelzpunkt: 182 ° C

| $C_{29}H_{29}ClN_2O_4$ (505,02) | | | | | | |
|---|---|---|---|---|---|---|
| Ber.:<br>Gef.: | C | 68,97<br>68,50 | H | 5,78<br>5,83 | N | 5,54<br>5,46 |

Beispiel 25

2-n-Butyl-4-chlor-5-hydroxymethyl-1-[4-[(α-carboxy)-p-phenyl-benzyloxy]benzyl]imidazol

Hergestellt analog Beispiel 1b aus 2-n-Butyl-4-chlor-5-hydroxymethyl-1-[4-[(α-methoxycarbonyl)-p-phenyl-benzyloxy]benzyl]imidazolund 1 N Natronlauge in Ethanol.
Ausbeute: 75,0 % der Theorie,
Schmelzpunkt: 185-186 ° C (Zers.)

| $C_{29}H_{29}ClN_2O_4$ (505,02) | | | | | | |
|---|---|---|---|---|---|---|
| Ber.:<br>Gef.: | C | 68,97<br>68,87 | H | 5,78<br>5,80 | N | 5,54<br>5,42 |

Beispiel 26

2-n-Butyl-4-hydroxymethyl-5-chlor-1-[4-[(α-carboxy)-2-naphthylmethyloxy]benzyl]imidazol-semihydrat

Hergestellt analog Beispiel 1b aus 2-n-Butyl-4-hydroxymethyl-5-chlor-1-[4-[(α-methoxycarbonyl)-2-naphthylmethyloxy]benzyl]imidazol und 1 N Natronlauge in Ethanol.
Ausbeute: 69,0 % der Theorie,
Schmelzpunkt: ab 188 ° C (Zers.)

| $C_{27}H_{27}ClN_2O_4$ x 0,5 $H_20$ (487,99) | | | | | | |
|---|---|---|---|---|---|---|
| Ber.:<br>Gef.: | C | 66,45<br>66,63 | H | 5,78<br>5,66 | N | 5,74<br>5,75 |

Beispiel 27

2-n-Butyl-4-chlor-5-hydroxymethyl-1-[4-[(α-carboxy)-2-naphthylmethyloxy]benzyl]imidazol-semihydrat

Hergestellt analog Beispiel 1b aus 2-n-Butyl-4-chlor-5-hydroxymethyl-1-[4-[αa-methoxycarbonyl)-2-naphthylmethyloxy]benzyl]imidazol und 1 N Natronlauge in Ethanol.
Ausbeute: 82,0 % der Theorie,
Schmelzpunkt: ab 142 ° C (Zers.)

| $C_{27}H_{27}ClN_2O_4$ x 0,5 $H_20$ (487,99) | | | | | | |
|---|---|---|---|---|---|---|
| Ber.:<br>Gef.: | C | 66,45<br>66,62 | H | 5,78<br>5,66 | N | 5,74<br>6,12 |

Beispiel 28

2-n-Butyl-4-hydroxymethyl-5-chlor-1-[4-[(α-carboxy)-1-naphthylmethyloxy]benzyl]imidazol-hydrat

Hergestellt analog Beispiel 1b aus 2-n-Butyl-4-hydroxymethyl-5-chlor-1-[4-[(α-methoxycarbonyl)-1-naphthylmethyloxy]benzyl]imidazol und 1 N Natronlauge in Ethanol.
Ausbeute: 91,0 % der Theorie,
Schmelzpunkt: ab 110 ° C (Zers.)

| $C_{27}H_{27}ClN_2O_4$ x $H_20$ (497,00) | | | | | | |
|---|---|---|---|---|---|---|
| Ber.:<br>Gef.: | C | 65,25<br>65,58 | H | 5,88<br>5,89 | N | 5,63<br>5,83 |

Beispiel 29

2-n-Butyl-4-chlor-5-hydroxymethyl-1-[4-[(α-carboxy)-1-naphthylmethyloxy]benzyl]imidazol-hydrat

Hergestellt analog Beispiel 1b aus 2-n-Butyl-4-chlor-5-hydroxymethyl-1-[4-[(α-methoxycarbonyl)-1-naphthylmethyloxy]benzyl]imidazol und 1 N Natronlauge in Ethanol.
Ausbeute: 42,0 % der Theorie,
Schmelzpunkt: ab 100 ° C (Zers.)

| $C_{27}H_{27}ClN_2O_4$ x $H_20$ (497,00) | | | | | | |
|---|---|---|---|---|---|---|
| Ber.:<br>Gef.: | C | 65,25<br>65,21 | H | 5,88<br>5,70 | N | 5,63<br>6,10 |

Beispiel 30

2-n-Butyl-4-chlor-5-hydroxymethyl-1-[4-[(α-carboxy)-p-methoxy-benzyloxy]benzyl]imidazol

Hergestellt analog Beispiel 1b aus 2-n-Butyl-4-chlor-5-hydroxymethyl-1-[4-[(α-methoxycarbonyl)-p-methoxy-benzyloxy]benzyl]imidazol und 1 N Natronlauge in Ethanol.
Ausbeute: 58,0 % der Theorie,
Schmelzpunkt: 165 ° C

| $C_{24}H_{27}ClH_2O_5$ (458,95) | | | | | | |
|---|---|---|---|---|---|---|
| Ber.:<br>Gef.: | C | 62,81<br>62,69 | H | 5,93<br>6,02 | N | 6,10<br>5,93 |

Beispiel 31

2-n-Butyl-4-chlor-5-methoxymethyl-1-[4-[(1-carboxy)-n-pentyloxy]benzyl]imidazol

Hergestellt analog Beispiel 1b aus 2-n-Butyl-4-chlor-5-methoxymethyl-1-[4-[(1-methoxycarbonyl)-n-pentyloxy]benzyl]imidazol und 1 N Natronlauge in Ethanol.
Ausbeute: 84,0 % der Theorie,
Schmelzpunkt: 168 ° C

| $C_{22}H_{31}ClN_2O_4$ (422,96) | | | | | | |
|---|---|---|---|---|---|---|
| Ber.:<br>Gef.: | C | 62,45<br>62,23 | H | 7,38<br>7,47 | N | 6,62<br>6,79 |

Beispiel 32

2-n-Butyl-4-chlor-5-hydroxymethyl-1-[4-[(1-carboxy)-n-butyloxy]benzyl]imidazol

Hergestellt analog Beispiel 1b aus 2-n-Butyl-4-chlor-5-hydroxymethyl-1-[4-[(1-methoxycarbonyl)-n-butyloxy)benzyl]imidazol und 1 N Natronlauge in Ethanol.
Ausbeute: 50,0 % der Theorie,
Schmelzpunkt: 137-138 ° C

| $C_{20}H_{27}ClN_2O_4$ (394,90) | | | | | | |
|---|---|---|---|---|---|---|
| Ber.:<br>Gef.: | C | 60,83<br>60,67 | H | 6,89<br>6,94 | N | 7,09<br>6,67 |

Beispiel 33

2-n-Butyl-4-chlor-5-hydroxymethyl-1-[4-[(1-carboxy)-n-pentyloxy]benzyl]imidazol

Hergestellt analog Beispiel 1b aus 2-n-Butyl-4-chlor-5-hydroxymethyl-1-[4-[(1-methoxycarbonyl)-n-pentyloxy]benzyl]imidazol und 1 N Natronlauge in Ethanol.
Ausbeute: 61,0 % der Theorie,
Schmelzpunkt: 150 ° C

| $C_{21}H_{29}ClN_2O_4$ (408,93) | | | | | | |
|---|---|---|---|---|---|---|
| Ber.:<br>Gef.: | C | 61,65<br>62,31 | H | 7,14<br>6,85 | N | 6,85<br>7,21 |

Beispiel 34

2-n-Butyl-4-hydroxymethyl-5-chlor-1-[4-[(1-carboxy)-n-pentyloxy]benzyl]imidazol

Hergestellt analog Beispiel 1b aus 2-n-Butyl-4-hydroxymethyl-5-chlor-1-[4-[(1-methoxycarbonyl)-n-pentyloxy]benzyl]imidazol und 1 N Natronlauge in Ethanol.
Ausbeute: 57,0 % der Theorie,
Schmelzpunkt: 168 ° C

| $C_{21}H_{29}ClN_2O_4$ (408,93) | | | | | | |
|---|---|---|---|---|---|---|
| Ber.:<br>Gef.: | C | 61,65<br>61,59 | H | 7,14<br>7,11 | N | 6,85<br>6,91 |

Beispiel 35

2-n-Butyl-8-methyl-3-[4-[(α-carboxy)benzyloxy]benzyl]chinazolin-4-on-semihydrat

a) 2-n-Butyl-8-methyl-3-[4-[(α-ethoxycarbonyl)benzyloxy]benzyl]chinazolin-4-on

Hergestellt analog Beispiel 1a aus 2-n-Butyl-8-methyl-chinazolin-4(1H)-on und 4-(α-Ethoxycarbonyl-benzyloxy)benzylbromid.
Ausbeute: 37 % der Theorie,
Öl, $R_f$-Wert: 0,45 (Kieselgel; Laufmittel: Petrolether/Essigester = 4:1)

b) 2-n-Butyl-8-methyl-3-[4-[(α-carboxy)benzyloxy]benzyl]chinazolin-4-on-semihydrat

Hergestellt analog Beispiel 1b aus 2-n-Butyl-8-methyl-3-[(α-ethoxycarbonyl)benzyloxy)benzyl]chinazolin-4-on und 1N Natronlauge in Ethanol.
Ausbeute: 84 % der Theorie,
Schmelzpunkt: 201-204 ° C,

| $C_{28}H_{28}N_2O_4$ x 0,5 $H_2O$ (465,56) | | | | | | |
|---|---|---|---|---|---|---|
| Ber.:<br>Gef.: | C | 72,24<br>72,41 | H | 6,28<br>6,33 | N | 6,02<br>5,75 |

Beispiel 36

2-n-Butyl-4-chlor-5-hydroxymethyl-1-[4-[(1-carboxy-2-cyclohexyl)ethoxy]benzyl]imidazol

Hergestellt analog Beispiel 1b aus 2-n-Butyl-4-chlor-5-hydroxymethyl-1-[4-[(1-methoxycarbonyl-2-cyclohexyl)ethoxy]benzylimidazol und 1 N Natronlauge in Ethanol.
Ausbeute: 67,1 % der Theorie,
Schmelzpunkt: 145-150 ° C

| $C_{24}H_{33}ClN_2O_4$ (448,96) | | | | | | |
|---|---|---|---|---|---|---|
| Ber.:<br>Gef.: | C | 64,20<br>63,97 | H | 7,40<br>7,38 | N | 6,24<br>6,01 |

Beispiel 37

2-n-Butyl-4-hydroxymethyl-5-chlor-1-[4-[(1-carboxy-2-cyclohexyl)ethoxy]benzyl]imidazol

Hergestellt analog Beispiel 1b aus 2-n-Butyl-4-hydroxymethyl-5-chlor-1-[4-[(1-methoxycarbonyl-2-cyclohexyl)ethoxy]benzylimidazol und 1 N Natronlauge in Ethanol.
Ausbeute: 59,1 % der Theorie,
Schmelzpunkt: 180-183 ° C

| $C_{24}H_{33}ClN_2O_4$ (448,96) | | | | | | |
|---|---|---|---|---|---|---|
| Ber.:<br>Gef.: | C | 64,20<br>63,99 | H | 7,40<br>7,20 | N | 6,24<br>6,27 |

Massenspektrum: m/e = 448/450 (Cl)

Beispiel 38

2-n-Butyl-1-[4-[(1-carboxy-3-methyl)butyloxy]benzyl)benzimidazol

Hergestellt analog Beispiel 1b aus 2-n-Butyl-1-[4-[(1-methoxycarbonyl-3-methyl)butyloxy]benzyl]-benzimidazol und 1 N Natronlauge in Ethanol.
Ausbeute: 82,3 % der Theorie,
Schmelzpunkt: 150-152 °C

| $C_{24}H_{30}N_2O_3$ (394,52) | | | | | | |
|---|---|---|---|---|---|---|
| Ber.:<br>Gef.: | C | 73,07<br>72,83 | H | 7,66<br>7,37 | N | 7,10<br>7,14 |

Massenspektrum: m/e = 394

Beispiel 39

2-n-Butyl-1-[4-[(1-carboxy-2-cyclohexyl)ethoxy]benzyl]benzimidazol

Hergestellt analog Beispiel 1b aus 2-n-Butyl-1-[4-[(1-methoxycarbonyl-2-cyclohexyl)ethoxy]benzyl]-benzimidazol und 1 N Natronlauge in Ethanol.
Ausbeute: 82,0 % der Theorie,
Schmelzpunkt: 165-170 °C

| $C_{27}H_{34}N_2O_3$ (434,58) | | | | | | |
|---|---|---|---|---|---|---|
| Ber.:<br>Gef.: | C | 74,63<br>74,46 | H | 7,88<br>7,77 | N | 6,44<br>6,30 |

Massenspektrum: m/e = 434

Beispiel 40

2-n-Butyl-6-propionylamino-1-[4-[(1-carboxy-3-methyl)butyloxy]benzyl]benzimidazol

Hergestellt analog Beispiel 1b aus 2-n-Butyl-6-propionylamino-1-[4-[(1-methoxycarbonyl-3-methyl)-butyloxy]benzyl]benzimidazol und 1 N Natronlauge in Ethanol.
Ausbeute: 80,3 % der Theorie,
Schmelzpunkt: 120-130 °C

| $C_{27}H_{35}N_3O_4$ (465,59) | | | | | | |
|---|---|---|---|---|---|---|
| Ber.:<br>Gef.: | C | 69,65<br>69,30 | H | 7,58<br>7,84 | N | 9,02<br>8,83 |

Massenspektrum: m/e = 465

Beispiel 41

2-n-Butyl-5-propionylamino-1-[4-[(1-carboxy-3-methyl)butyloxy]benzyl]benzimidazol-semihydrat

Hergestellt analog Beispiel 1b aus 2-n-Butyl-5-propionylamino-1-[4-[(1-methoxycarbonyl-3-methyl)-butyloxy]benzyl]benzimidazol und 1 N Natronlauge in Ethanol.
Ausbeute: 82,9 % der Theorie,
Schmelzpunkt: 165-170 ° C

| $C_{27}H_{35}N_3O_4$ x 0,5 $H_20$ (474,60) | | | | | | |
|---|---|---|---|---|---|---|
| Ber.:<br>Gef.: | C | 68,32<br>68,63 | H | 7,64<br>7,72 | N | 8,85<br>8,90 |

Beispiel 42

2-n-Butyl-6-cyclohexylaminocarbonylamino-1-[4-[(1-carboxy-3-methyl)butyloxy]benzyl]benzimidazol

Hergestellt analog Beispiel 1b aus 2-n-Butyl-6-cyclohexylaminocarbonylamino-1-[4-[(1-methoxycarbonyl-3-methyl)butyloxy]benzyl]benzimidazol und 1 N Natronlauge in Ethanol.
Ausbeute: 90,8 % der Theorie,
Schmelzpunkt: 155-160 ° C

| $C_{31}H_{42}N_4O_4$ (534,71) | | | | | | |
|---|---|---|---|---|---|---|
| Ber.:<br>Gef.: | C | 69,64<br>69,58 | H | 7,92<br>8,03 | N | 10,48<br>10,36 |

Massenspektrum: $(M + H)^+$ = 535

Beispiel 43

2-n-Butyl-5-cyclohexylaminocarbonylamino-1-[4-[(1-carboxy-3-methyl)butyloxy]benzyl]benzimidazol

Hergestellt analog Beispiel 1b aus 2-n-Butyl-5-cyclohexylaminocarbonylamino-1-[4-[(1-methoxycarbonyl-3-methyl)butyloxy]benzyl]benzimidazol und 1 N Natronlauge in Ethanol.
Ausbeute: 95,7 % der Theorie,
Schmelzpunkt: 220-225 ° C

| $C_{31}H_{42}N_4O_4$ (534,71) | | | | | | |
|---|---|---|---|---|---|---|
| Ber.:<br>Gef.: | C | 69,64<br>69,49 | H | 7,92<br>7,99 | N | 10,48<br>10,52 |

Massenspektrum: $(M + H)^+$ = 535

Beispiel 44

2-n-Butyl-6-(N-propionyl-methylamino)-1-[4-[(1-carboxy-3-methyl)butyloxy]benzyl]benzimidazol

Hergestellt analog Beispiel 1b aus 2-n-Butyl-6-(N-propionylmethylamino)-1-[4-[(1-methoxycarbonyl-3-methyl)butyloxy]benzyl]benzimidazol und 1 N Natronlauge in Ethanol.
Ausbeute: 66,8 % der Theorie,
Schmelzpunkt: 170-175 ° C

| $C_{28}H_{37}N_3O_4$ (479,63) | | | | | | |
|---|---|---|---|---|---|---|
| Ber.:<br>Gef.: | C | 70,12<br>70,35 | H | 7,78<br>7,85 | N | 8,76<br>8,91 |

Beispiel 45

2-n-Butyl-5-(N-propionyl-methylamino)-1-[4-[(1-carboxy-3-methyl)butyloxy]benzyl]benzimidazol

Hergestellt analog Beispiel 1b aus 2-n-Butyl-5-(N-propionylmethylamino)-1-[4-[(1-methoxycarbonyl-3-methyl)butyloxy]benz yl]benzimidazol und 1 N Natronlauge in Ethanol.
Ausbeute: 62,0 % der Theorie,
Schmelzpunkt: 172-175 ° C

| $C_{28}H_{37}N_3O_4$ (479,63) | | | | | | |
|---|---|---|---|---|---|---|
| Ber.:<br>Gef.: | C | 70,12<br>70,11 | H | 7,78<br>7,80 | N | 8,76<br>8,63 |

Massenspektrum: m/e = 479

Beispiel 46

2-n-Butyl-6-(N-cyclohexylaminocarbonyl-methylamino)-1-[4-[(1-carboxy-3-methyl)butyloxy]benzyl]-benzimidazol

Hergestellt analog Beispiel 1b aus 2-n-Butyl-6-(N-cyclohexylaminocarbonyl-methylamino)-1-[4-[(1-methoxycarbonyl-3-methyl)butyloxy]benzyl]benzimidazol und 1 N Natronlauge in Ethanol.
Ausbeute: 76,1 % der Theorie,
Schmelzpunkt: 170-175 ° C

| $C_{32}H_{44}N_4O_4$ (548,73) | | | | | | |
|---|---|---|---|---|---|---|
| Ber.:<br>Gef.: | C | 70,04<br>69,95 | H | 8,08<br>8,10 | N | 10,21<br>10,22 |

Massenspektrum: m/e = 548

Beispiel 47

2-n-Butyl-5-(N-cyclohexylaminocarbonyl-methylamino)-1-[4-[(1-carboxy-3-methyl)butyloxy]benzyl]-benzimidazol

Hergestellt analog Beispiel 1b aus 2-n-Butyl-5-(N-cyclohexylaminocarbonyl-methylamino)-1-[4-[(1-methoxycarbonyl-3-methyl)butyloxy]benzyl]benzimidazol und 1 N Natronlauge in Ethanol.
Ausbeute: 72,9 % der Theorie,
Schmelzpunkt: 178-182 ° C

| $C_{32}H_{44}N_4O_4$ (548,73) | | | | | | |
|---|---|---|---|---|---|---|
| Ber.:<br>Gef.: | C | 70,04<br>69,77 | H | 8,08<br>7,97 | N | 10,21<br>10,04 |

Massenspektrum: m/e = 548

Beispiel 48

2-n-Butyl-3-[4-[(α-carboxy)benzyloxy]benzyl]-5-methyl-6-dimethylaminocarbonylamino-imidazo[4,5-b]pyridin

Hergestellt analog Beispiel 1b aus 2-n-Butyl-3-[4-[(α-(ethoxycarbonyl)benzyloxy]benzyl]-5-methyl-6-dimethylaminocarbonylamino-imidazo[4,5-b]pyridin und 2 N Natronlauge in Ethanol.
Ausbeute: 80,0 % der Theorie,
Schmelzpunkt: 191-193 ° C

| $C_{29}H_{33}N_5O_4$ (515,62) | | | | | | |
|---|---|---|---|---|---|---|
| Ber.:<br>Gef.: | C | 67,55<br>67,49 | H | 6,45<br>6,62 | N | 13,58<br>13,57 |

Beispiel 49

2-n-Butyl-3-[4-[(α-carboxy)benzyloxy]benzyl]-5-methyl-6-(3-benzyl-3,4,5,6-tetrahydro-2(1H)-pyrimidinon-1-yl)-imidazo[4,5-b]pyridin

Hergestellt analog Beispiel 1b aus 2-n-Butyl-3-[4-[(α-ethoxycarbonyl)benzyloxy]benzyl]-5-methyl-6-(3-benzyl-3,4,5,6-tetrahydro-2(1H)-pyrimidinon-1-yl)-imidazo[4,5-b]pyridin und 2n Natronlauge in Ethanol.
Ausbeute: 87,5 % der Theorie,
Schmelzpunkt: 157-160 ° C

| $C_{37}H_{39}N_5O_4$ (617,75) | | | | | | |
|---|---|---|---|---|---|---|
| Ber.:<br>Gef.: | C | 71,94<br>71,71 | H | 6,36<br>6,36 | N | 11,34<br>10,96 |

Beispiel 50

2-n-Butyl-6-(N-methylaminocarbonyl-n-pentylamino)-1-[4-[(α-carboxy)benzyloxy]benzyl]benzimidazol

Hergestellt analog Beispiel 1b aus 2-n-Butyl-6-(N-methylaminocarbonyl-n-pentylamino)-1-[4-[(α-methoxycarbonyl)benzyloxy]benzyl]benzimidazol und 1 N Natronlauge in Ethanol.
Ausbeute: 76,0 % der Theorie,
Schmelzpunkt: 170-172 ° C

| $C_{33}H_{40}N_4O_4$ (556,71) | | | | | | |
|---|---|---|---|---|---|---|
| Ber.:<br>Gef.: | C | 71,20<br>70,80 | H | 7,24<br>7,34 | N | 10,07<br>9,96 |

Beispiel 51

2-n-Butyl-6-(N-cyclohexylaminocarbonyl-n-pentylamino)-1-[4-[(α-carboxy)benzyloxy]benzyl]benzimidazol

Hergestellt analog Beispiel 1b aus 2-n-Butyl-6-(N-cyclohexylaminocarbonyl-n-pentylamino)-1-[4-[(α-methoxycarbonyl)benzyloxy]benzyl]benzimidazol und 1 N Natronlauge in Ethanol.
Ausbeute: 65,0 % der Theorie,
Schmelzpunkt: 83 ° C

| $C_{38}H_{48}N_4O_4$ (624,83) | | | | | | |
|---|---|---|---|---|---|---|
| Ber.:<br>Gef.: | C | 73,05<br>72,80 | H | 7,74<br>7,51 | N | 8,97<br>8,59 |

Beispiel 52

2-n-Butyl-4-hydroxymethyl-5-chlor-1-[4-[(α-carboxy-α-phenyl)benzyloxy]benzyl]benzimidazol

Hergestellt analog Beispiel 1b aus 2-n-Butyl-4-hydroxymethyl-5-chlor-1-[4-[(α-methoxycarbonyl-α-phenyl)benzyloxy]benzyl]benzimidazol und 1 N Natronlauge in Ethanol.
Ausbeute: 50,0 % der Theorie,
Schmelzpunkt: 165 ° C (Zers.)

| $C_{29}H_{29}ClN_2O_4$ (505,02) | | | | | | |
|---|---|---|---|---|---|---|
| Ber.:<br>Gef.: | C | 68,97<br>68,50 | H | 5,79<br>5,72 | N | 5,55<br>5,51 |

Beispiel 53

2-n-Butyl-6-(p-methyl-phenylsulfonylamino)-1-[4-[(α-carboxy)benzyloxy]benzyl]benzimidazol

Hergestellt analog Beispiel 1b aus 2-n-Butyl-6-(p-methyl-phenylsulfonylamino)-1-[4-[(α-methoxycarbonyl)benzyloxy]benzyl]benzimidazol und 1 N Natronlauge in Ethanol.
Ausbeute: 81,0 % der Theorie,
Schmelzpunkt: 150 ° C

| $C_{33}H_{33}N_3O_5S$ (583,71) | | | | | | |
|---|---|---|---|---|---|---|
| Ber.:<br>Gef.: | C | 67,90<br>67,18 | H | 5,70<br>5,70 | N | 7,20<br>6,96 |

Beispiel 54

2-n-Butyl-6-(N-methyl-p-methyl-phenylsulfonylamino)-1-[4-[(α-carboxy)benzyloxy]benzyl]benzimidazol

Hergestellt analog Beispiel 1b aus 2-n-Butyl-6-(N-methyl-p-methyl-phenylsulfonylamino)-1-[4-[(α-methoxycarbonyl)benzyloxy]benzyl]benzimidazol und 1 N Natronlauge in Ethanol.
Ausbeute: 96,0 % der Theorie,
Schmelzpunkt: 218 ° C

| $C_{34}H_{35}N_3O_5S$ (597,71) | | | | | | |
|---|---|---|---|---|---|---|
| Ber.:<br>Gef.: | C | 68,32<br>67,82 | H | 5,90<br>5,84 | N | 7,03<br>6,85 |

Beispiel 55

2-n-Butyl-6-(N-n-pentyl-p-methyl-phenylsulfonylamino)-1-[4-[(α-carboxy)benzyloxy]benzyl]benzimidazol

Hergestellt analog Beispiel 1b aus 2-n-Butyl-6-(N-n-pentyl-p-methyl-phenylsulfonylamino)-1-[4-[(α-methoxycarbonyl)benzyloxy]benzyl]benzimidazol und 1 N Natronlauge in Ethanol.
Ausbeute: 94,0 % der Theorie,
Schmelzpunkt: 202 ° C

| $C_{38}H_{43}N_3O_5S$ (653,81) | | | | | | |
|---|---|---|---|---|---|---|
| Ber.:<br>Gef.: | C | 69,80<br>69,47 | H | 6,63<br>6,57 | N | 6,43<br>6,64 |

Beispiel 56

2-n-Butyl-1-[(4-[(α-carboxy)benzyloxy]-(α-methyl)benzyl]benzimidazol

a) 4-[(α-Ethoxycarbonyl)benzyloxy]acetophenon

Hergestellt analog Beispiel 1a aus 2-Brom-phenylessigsäure-ethylester und 4-Hydroxyacetophenon.
Ausbeute: 78,0 % der Theorie,
$R_f$-Wert: 0,58 (Kieselgel; Laufmittel: Toluol/Essigester = 85:15)

b) 1-[4-[(α-Ethoxycarbonyl)benzyloxy]phenyl]ethanol

2,3 g (7,7 mMol) 4-(α-Ethoxycarbonyl)benzyloxy]-acetophenon werden in 50 ml Ethanol gelöst und bei Raumtemperatur portionsweise mit 0,28 g (7,7 mMol) Natriumborhydrid versetzt. Die Reaktionsmischung wird 2 Stunden bei 40 ° C gerührt. Nach Abkühlung auf Raumtemperatur wird eingeengt und der Rückstand mit Wasser und verdünnter Salzsäure versetzt. Nach Extraktion mit Essigester wird die organische Phase mit Natriumsulfat getrocknet. Die Lösung wird eingedampft und der Rückstand über eine Kieselgelsäule (Korngröße: 0,063-0,02 mm) mit Toluol/Essigester (7:3) chromatographiert. Die einheitlichen Fraktionen werden vereinigt und eingedampft.
Ausbeute: 1,0 g (43 % der Theorie),
Öl, $R_f$-Wert: 0,50 (Kieselgel; Laufmittel: Toluol/Essigester = 7:3)

c) 1-[4-[(α-Ethoxycarbonyl)benzyloxy]phenyl]-1-methansulfonyloxy-ethan

0,5 g (1,7 mMol) 1-[4-[(α-Ethoxycarbonyl)benzyloxy]phenyl]ethanol und 0,22 g (2,2 mMol) Triethylamin werden in 10 ml Methylenchlorid gelöst. Bei 5 ° C werden 0,23 g (2,0 mMol) Mesylchlorid unter Rühren zugetropft. Nach einer Stunde bei Raumtemperatur wird das Reaktionsgemisch auf Eiswasser gegossen, mit Methylenchlorid extrahiert und über Natriumsulfat getrocknet. Das erhaltene Rohprodukt wird ohne weitere Reinigung direkt umgesetzt.

d) 2-n-Butyl-1-[4-[(α-ethoxycarbonyl)benzyloxy]-(α-methyl)benzyl]benzimidazol

Eine Mischung von 0,26 g (1,5 mMol) 2-n-Butyl-benzimidazol, 3 ml (1,7 mMol) Triethylamin und 1-[4-[-(α-Ethoxycarbonyl)benzyloxy]phenyl]-1-methansulfonyloxy-ethan (roh) werden 30 Minuten auf 80 ° C erwärmt. Nach Abkühlung wird Wasser zugesetzt und zweimal mit Essigester extrahiert. Die organischen Phasen werden vereinigt, getrocknet und eingedampft. Das Rohprodukt wird über eine Kieselgelsäule (Korngröße: 0,063-0,02 mm) mit Toluol/Essigester (8:2) chromatographiert. Die einheitlichen Fraktionen werden vereinigt und eingedampft.
Ausbeute: 0,10 g (15 % der Theorie),
$R_f$-Wert: 0,35 (Kieselgel; Laufmittel: Toluol/Essigester = 8:2)

e) 2-n-Butyl-1-[4-[(α-carboxy)benzyloxy]-(α-methyl)benzyl]benzimidazol

Hergestellt analog Beispiel 1b aus 2-n-Butyl-1-[4-[(α-ethoxycarbonyl)benzyloxy]-(α-methyl)benzyl]-benzimidazol und 1 N Natronlauge in Ethanol.
Ausbeute: 82 % der Theorie,
Schmelzpunkt: 109 ° C

| $C_{27}H_{28}N_2O_3$ (428,51) | | | | | | |
|---|---|---|---|---|---|---|
| Ber.:<br>Gef.: | C | 75,67<br>75,93 | H | 6,59<br>6,76 | N | 6,54<br>6,32 |

Beispiel 57

2-n-Butyl-1-[4-[α-(1H-tetrazol-5-yl)benzyloxy]benzyl]-6-(3-benzyl-3,4,5,6-tetrahydro-2(1H)pyrimidinon-1-yl)-benzimidazol

a) 5-Benzyl-1H-tetrazol

Hergestellt aus Benzylcyanid und Natriumazid/Ammoniumchlorid in Dimethylformamid.
Ausbeute: 70 % der Theorie,
Schmelzpunkt: 130-132 ° C

b) N-Triphenylmethyl-5-benzyl-tetrazol

Hergestellt aus 5-Benzyl-1H-tetrazol und Triphenylmethylchlorid.
Ausbeute: 84 % der Theorie,
Schmelzpunkt: 158-160 ° C

c) N-Triphenylmethyl-5-(α-brom)benzyl-tetrazol

2,0 g (5 mMol) N-Triphenylmethyl-5-benzyl-tetrazol, 0,89 g (5 mMol) N-Bromsuccinimid und 30 mg Azo-bisisobutyronitril werden in 25 ml Tetrachlorkohlenstoff gelöst und unter UV-Bestrahlung eine Stunde zum Sieden erhitzt. Nach Abkühlung wird das gebildete Succinimid abfiltriert und das Filtrat eingedampft.
Ausbeute: 74,6 % der Theorie,
Schmelzpunkt: 160-162 ° C

d) 2-n-Butyl-1-[(4-benzyloxy)benzyl]-6-(3-benzyl-3,4,5,6-tetrahydro-2(1H)pyrimidinon-1-yl)-benzimidazol

Hergestellt analog Beispiel 1a aus 2-n-Butyl-6-(3-benzyl-3,4,5,6-tetrahydro-2(1H)pyrimidinon-1-yl)-benzimidazol und 4-Benzyloxybenzylchlorid.
Ausbeute: 65,5 % der Theorie,
$R_f$-Wert: 0,45 (Kieselgel; Laufmittel: Methylenchlorid/Ethanol = 19:1)

e) 2-n-Butyl-1-[(4-hydroxy)benzyl]-6-(3-benzyl-3,4,5,6-tetrahydro-2(1H)pyrimidinon-1-yl)-benzimidazol

2,4 g 2-n-Butyl-1-[(4-benzyloxy)benzyl]-6-(3-benzyl-3,4,5,6-tetrahydro-2(1H)pyrimidinon-1-yl)-benzimidazol werden in 100 ml Ethanol und 30 ml Dimethylformamid gelöst, mit 2,4 g Palladium auf Aktivkohle (10%ig) versetzt und mit 5 bar Wasserstoff bei Raumtemperatur hydriert. Der Katalysator wird abfiltriert, das Filtrat wird eingedampft und der Rückstand über eine Kieselgelsäule (Korngröße: 0,063-0,02 mm) mit Methylenchlorid/Ethanol chromatographiert. Die einheitlichen Fraktionen werden vereinigt und eingedampft, der Rückstand wird mit Ether verrieben und abgesaugt.
Ausbeute: 1,1 g (55 % der Theorie),
Schmelzpunkt: 190-191 ° C

f) 2-n-Butyl-1-[4-[(α-(N-triphenylmethyl)tetrazol-5-yl)benzyloxy]benzyl]-6-(3-benzyl-3,4,5,6-tetrahydro-2(1H)-pyrimidinon-1-yl)-benzimidazol

0,5 g (1,1 mMol) 2-n-Butyl-1-[(4-hydroxy)benzyl]-6-(3-benzyl-3,4,5,6-tetrahydro-2(1H)pyrimidinon-1-yl)-benzimidazol, 0,74 g (5,4 mMol) Kaliumkarbonat und 0,93 g (1,1 mMol) N-Triphenylmethyl-5-[(α-brom)-benzyl]tetrazol werden in 10 ml Dimethylsulfoxid gelöst und 2 Stunden bei Raumtemperatur gerührt. Das Produkt wird durch Zusatz von Kochsalzlösung ausgefällt, abgesaugt, mit Wasser gewaschen und getrocknet.
Ausbeute: 0,9 g (97 % der Theorie),
Schmelzpunkt: ab 150 ° C (Zers.)

g) 2-n-Butyl-1-[4-[α-(1H-tetrazol-5-yl)benzyloxy]benzyl]-6-(3-benzyl-3,4,5,6-tetrahydro-2(1H)pyrimidinon-1-yl)benzimidazol

0,9 g (1,0 mMol) 2-n-Butyl-1-[4-[(α-N-triphenylmethyl)tetrazol-5-yl)benzyloxy]benzyl]-6-(3-benzyl-3,4,5,6-tetrahydro-2(1H)pyrimidinon-1-yl)-benzimidazol werden in 40 ml Ethanol, 10 ml Methylenchlorid und 12 ml 4N Salzsäure gelöst und 2 Stunden bei Raumtemperatur gerührt. Nach Zusatz von 30 ml Wasser wird mit Methylenchlorid extrahiert. Die organische Phase wird mit 1N Natronlauge gewaschen, mit verdünnter Essigsäure angesäuert und über Natriumsulfat getrocknet. Das Rohprodukt wird über eine Kieselgelsäule (Korngröße: 0,063-0,02 mm) mit Methylenchlorid/Ethanol (50:1 und 19:1) chromatographiert. Die einheitlichen Fraktionen werden vereinigt und eingedampft.
Ausbeute: 0,22 g (34 % der Theorie),
Schmelzpunkt: ab 134 ° C (Zers.)

| $C_{37}H_{38}N_8O_2$ (626,76) | | | | | | |
|---|---|---|---|---|---|---|
| Ber.:<br>Gef.: | C | 70,90<br>70,72 | H | 6,11<br>6,00 | N | 17,88<br>17,68 |

Beispiel 58

2-n-Propyl-4-methyl-1-[4-[α-(1H-tetrazol-5-yl)benzyloxy]benzyl]benzimidazol

a) 2-n-Propyl-4-methyl-1-[4-[(α-(N-triphenylmethyl)-tetrazol-5-yl)benzyloxy]benzyl-benzimidazol

Hergestellt analog Beispiel 57f aus 2-n-Propyl-4-methyl-1-[(4-hydroxy)benzyl]benzimidazol und N-Triphenylmethyl-5-[(α-brom)benzyl]tetrazol.
Ausbeute: 99 % der Theorie,
$R_f$-Wert: 0,68 (Kieselgel; Laufmittel: Methylenchlorid/Methanol = 19:1)

b) 2-n-Propyl-4-methyl-1-[4-[α-(1H-tetrazol-5-yl)benzyloxy]benzyllbenzimidazol

Hergestellt analog Beispiel 57g aus 2-n-Propyl-4-methyl-1-[4-[(α-(N-triphenylmethyl)tetrazol-5-yl)-benzyloxy]benzyl]benzimidazolund methanolischer Salzsäure.
Ausbeute: 63 % der Theorie,
Schmelzpunkt: amorph

| $C_{26}H_{26}N_6O$ (438,54) | | | | | | |
|---|---|---|---|---|---|---|
| Ber.:<br>Gef.: | C | 71,21<br>70,99 | H | 5,98<br>5,98 | N | 19,17<br>18,96 |

Beispiel 59

2-n-Propyl-4-methyl-6-(1-methyl-benzimidazol-2-yl)-1-[4-[α-(1H-tetrazol-5-yl)benzyloxy]benzyl]benzimidazol

a) 2-n-Propyl-4-methyl-6-(1-methyl-benzimidazol-2-yl)-1-[4-[(α-(N-triphenylmethyl)tetrazol-5-yl)benzyloxy]-benzyl]benzimidazol

Hergestellt analog Beispiel 57f aus 2-n-Propyl-4-methyl-6-(1-methyl-benzimidazol-2-yl)-1-[(4-hydroxy)-benzyl)benzimidazol (Synthese analog Beispiel 58d und 58e) und N-Triphenylmethyl-5-(α-brom)benzyl-tetrazol.
Ausbeute: 94 % der Theorie,
$R_f$-Wert: 0,70 (Kieselgel; Laufmittel: Methylenchlorid/Methanol = 9:1)

b) 2-n-Propyl-4-methyl-6-(1-methyl-benzimidazol-2-yl)-1-[4-[α-(1H-tetrazol-5-yl)benzyloxy]benzyl]-benzimidazol

Hergestellt analog Beispiel 57g aus 2-n-Propyl-4-methyl-6-(1-methyl-benzimidazol-2-yl)-1-[4-[(α-(N-triphenylmethyl)tetrazol-5-yl)benzyloxy]benzyl]benzimidazol und methanolischer Salzsäure.
Ausbeute: 62 % der Theorie,
Schmelzpunkt: 165-166 ° C

| $C_{34}H_{32}N_8O$ (568,69) | | | | | | |
|---|---|---|---|---|---|---|
| Ber.:<br>Gef.: | C | 71,81<br>71,68 | H | 5,67<br>5,60 | N | 19,71<br>19,55 |

Beispiel 60

2-n-Propyl-5-n-butyrylamino-3-[4-[(1-(1H-tetrazol-5-yl)-3-methyl)butyloxy]benzyl]imidazo[4,5-b]pyridin

a) 2-n-Propyl-5-n-butyrylamino-3-[4-[(1-cyano-3-methyl)butyloxy]benzyl]imidazo[4,5-b]pyridin

Hergestellt analog Beispiel 57f aus 2-n-Propyl-5-n-butyrylamino-3-[(4-hydroxy)benzyl]imidazo[4,5-b]-pyridin und 2-Brom-4-methyl-valeronitril.
Ausbeute: 85 % der Theorie,
$R_f$-Wert: 0,40 (Kieselgel; Laufmittel: Essigester/Petrolether = 1:1)

b) 2-n-Propyl-5-n-butyrylamino-3-[4-[(1-(1H-tetrazol-5-yl)-3-methyl)butyloxy]benzyl]imidazo[4,5-b]pyridin

Eine Mischung aus 800 mg (1,78 mMol) 2-n-Propyl-5-n-butyrylamino-3-[4-[(1-cyano-3-methyl)butyloxy)-benzyl]imidazo[4,5-b]pyridin, 2,2 g (3,3 mMol) Natriumazid und 1,8 g (3,3 mMol) Ammoniumchlorid in 8 ml Dimethylformamid wird 5 Stunden unter Rühren bei 130 ° C gerührt. Anschließend wird in Eiswasser gerührt, das ausgefallene Produkt abgesaugt, mit Wasser gewaschen, getrocknet und über eine Kieselgelsäule (Methylenchlorid/Ethanol = 19:1) gereinigt. Die die gewünschte Substanz enthaltende Fraktionen werden eingeengt, der erhaltene Rückstand mit Ether verrieben, abgesaugt und getrocknet. Beim Wiederholen des vorstehend beschriebenen Reinigungsschrittes erhält man 370 mg (42 % der Theorie) vom Schmelzpunkt 175-177 ° C.

| $C_{26}H_{34}N_8O_2$ (490,61) | | | | | | |
|---|---|---|---|---|---|---|
| Ber.:<br>Gef.: | C | 63,65<br>63,61 | H | 6,99<br>7,03 | N | 22,84<br>22,83 |

Beispiel 61

2-n-Butyl-4-hydroxymethyl-5-chlor-1-[4-[(1-(1H-tetrazol-5-yl)propyloxy]benzyl]benzimidazol-sesquihydrochlorid

Hergestellt analog Beispiel 60b aus 2-n-Butyl-4-hydroxymethyl-5-chlor-1-[4-(1-cyano-propyloxy)benzyl]-imidazol und Natriumazid/Ammoniumchlorid in Dimethylformamid.
Ausbeute: 78 % der Theorie,

| Öl, $C_{19}H_{25}ClN_6O_2$ x 1,5 HCl (459,59) | | | | | | |
|---|---|---|---|---|---|---|
| Ber.:<br>Gef.: | C | 49,65<br>49,41 | H | 5,81<br>6,03 | N | 18,28<br>18,52 |

Beispiel 62

2-n-Butyl-5-hydroxymethyl-4-chlor-1-[4-[(1-(1H-tetrazol-5-yl)propyloxy]benzyl]benzimidazol-sesquihydrochlorid

Hergestellt analog Beispiel 60b aus 2-n-Butyl-5-hydroxymethyl-4-chlor-1-[4-(1-cyano-propyloxy)benzyl]-imidazol und Natriumazid/Ammoniumchlorid in Dimethylformamid.
Ausbeute: 70 % der Theorie,

| Öl, $C_{19}H_{25}ClN_6O_2$ x 1,5 HCl (459,59) | | | | | | |
|---|---|---|---|---|---|---|
| Ber.:<br>Gef.: | C | 49,65<br>49,86 | H | 5,81<br>5,76 | N | 18,28<br>18,26 |

Beispiel 63

2-n-Butyl-1-[4-[(α-carboxy)benzylamino]benzyl]benzimidazolsemihydrat

a) 2-n-Butyl-1-[4-[(α-ethoxycarbonyl)benzylamino]benzyl]benzimidazol

1.0 g (3,6 mMol) 2-n-Butyl-1-[(4-amino)benzyl]benzimidazol, 0,87 g (3,6 mMol) 2-Brom-phenylessigsäureethylester und 0,5 g Natriumacetat-trihydrat werden in 20 ml Ethanol gelöst und 18 Stunden bei Raumtemperatur gerührt. Anschließend wird das Reaktionsgemisch noch 4 Stunden unter Rückfluß erhitzt. Das Solvens wird eingedampft, der Rückstand wird mit Wasser versetzt, mit verdünnter Ammoniaklösung alkalisch gestellt und mit Essigester extrahiert. Die vereinigten organischen Phasen werden mit Wasser gewaschen, über Magnesiumsulfat getrocknet und eingedampft. Der Rückstand wird über eine Kieselgelsäule (Korngröße: 0,063-0,02 mm) gereinigt, wobei als Elutionsmittel Petrolether mit 10-15 % Essigester verwendet wird. Die einheitlichen Fraktionen werden vereinigt und eingedampft. Ausbeute: 1,0 g (63 % der Theorie),
$R_f$-Wert: 0,53 (Kieselgel; Laufmittel: Petrolether/Essigester = 3:1)

b) 2-n-Butyl-1-[4-[(α-carboxy)benzylamino]benzyl]benzimidazol-semihydrat

Hergestellt analog Beispiel 1b aus 2-n-Butyl-1-[4-[(α-ethoxycarbonyl)benzylamino]benzyl]benzimidazol und 1N Natronlauge in Ethanol.
Ausbeute: 99 % der Theorie,

| $C_{26}H_{27}N_3O_2$ x 0,5 $H_2O$ (422,54) | | | | | | |
|---|---|---|---|---|---|---|
| Ber.:<br>Gef.: | C | 73,91<br>74,05 | H | 6,68<br>6,55 | N | 9,95<br>9,91 |

Beispiel 64

2-n-Butyl-1-[4-[(α-carboxy)-N-acetyl-benzylamino]benzyl]benzimidazol

a) 2-n-Butyl-1-[4-[(α-ethoxycarbonyl)-N-acetyl-benzylamino]benzyl]benzimidazol

0,5 g (1,1 mMol) 2-n-Butyl-1-[4-[(α-ethoxycarbonyl)benzylamino]benzyl)benzimidazol werden in 5 ml Acetanhydrid gelöst und 3 Stunden bei 120°C gerührt. Das Solvens wird abgezogen, der Rückstand wird über eine Kieselgelsäule (Korngröße: 0,063-0,02 mm) gereinigt, wobei als Elutionsmittel Essigester verwendet wird. Die einheitlichen Fraktionen werden vereinigt und eingedampft.
Ausbeute: 0,35 g (64 % der Theorie),
$R_f$-Wert: 0,50 (Kieselgel; Laufmittel: Methylenchlorid/Ethanol = 95:5)

b) 2-n-Butyl-1-[4-[(α-carboxy)-N-acetyl-benzylamino]benzyl]benzimidazol

Hergestellt analog Beispiel 1b aus 2-n-Butyl-1-[4-[(α-ethoxycarbonyl)-N-acetyl-benzylamino]benzyl]-benzimidazol und 1N Natronlauge in Ethanol.
Ausbeute: 74 % der Theorie,
Schmelzpunkt: 212-214°C

| $C_{28}H_{29}N_3O_3$ (455,56) | | | | | | |
|---|---|---|---|---|---|---|
| Ber.:<br>Gef.: | C | 73,82<br>73,50 | H | 6,42<br>6,58 | N | 9,12<br>9,28 |

Beispiel 65

2-n-Butyl-1-[4-[(2-carboxy-3-phenyl)propyl]benzyl]benzimidazol

a) 2-n-Butyl-1-[(4-hydroxymethyl)benzyl]benzimidazol

1,2 g (30 mMol) Lithiumaluminiumhydrid werden in 200 ml absolutem Tetrahydrofuran suspendiert. Bei Raumtemperatur wird eine Lösung von 10,6 g (30 mMol) 2-n-Butyl-1-[(4-ethoxycarbonyl)benzyl]-benzimidazol in 100 ml absolutem Tetrahydrofuran zugetropft. Die Mischung wird 2 Stunden bei Raumtemperatur gerührt und anschließend unter Kühlung mit wässriger Natronlauge langsam hydrolysiert. Die kristallisierten Salze werden abgesaugt, und das Filtrat wird eingeengt. Der Rückstand wird mit Wasser versetzt und mit Essigester extrahiert. Die vereinigten organischen Phasen werden mit Wasser gewaschen, getrocknet und eingedampft. Der Rückstand wird über eine Kieselgelsäule (Korngröße: 0,063-0,02 mm) gereinigt, wobei als Elutionsmittel Methylenchlorid/Methanol (30:1) verwendet wird. Die einheitlichen Fraktionen werden vereinigt und eingedampft.
Ausbeute: 6,5 g (74 % der Theorie),
$R_f$-Wert: 0,60 (Kieselgel; Laufmittel: Methylenchlorid/Methanol = 19:1)

b) 2-n-Butyl-1-[(4-chlormethyl)benzyl]benzimidazol

6,2 g (21 mMol) 2-n-Butyl-1-[(4-hydroxymethyl)benzyl]benzimidazol werden in 10 ml Thionylchlorid gelöst und 10 Minuten unter Rückfluß gekocht. Anschließend wird überschüssiges Thionylchlorid abdestilliert und der Rückstand mit Eiswasser versetzt. Nach der Neutralisation mit gesättigter Natriumhydrogencarbonatlösung wird mit Essigester extrahiert. Die vereinigten organischen Phasen werden mit Wasser gewaschen, getrocknet und eingeengt.

Ausbeute: 6,0 g (91 % der Theorie),
$R_f$-Wert: 0,65 (Kieselgel; Laufmittel: Methylenchlorid/Methanol = 19:1)

c) 2-n-Butyl-1-[4-[(2,2-bis-ethoxycarbonyl-3-phenyl)propyl]benzyl]benzimidazol

2,4 g (9,6 mMol) Benzylmalonsäurediethylester werden in 25 ml Dimethylsulfoxid gelöst, mit 1,1 g (9,6 mMol) Kalium-tert.butylat versetzt und 30 Minuten bei Raumtemperatur gerührt. Danach wird eine Lösung von 3,0 g (9,6 mMol) 2-n-Butyl-1-[(4-chlormethyl)benzyl]benzimidazol in 25 ml Dimethylsulfoxid zugetropft. Nach einer Stunde bei Raumtemperatur wird noch 15 Minuten auf 100°C erhitzt. Das Reaktionsgemisch wird mit Eiswasser versetzt. Das auskristallisierte Produkt wird abgesaugt und mit Essigester extrahiert. Die vereinigten organischen Phasen werden mit Wasser gewaschen, getrocknet und eingeengt.
Ausbeute: 4,2 g (83 % der Theorie),
$R_f$-Wert: 0,15 (Kieselgel; Laufmittel: Methylenchlorid/Methanol = 30:1)

d) 2-n-Butyl-1-[4-[(2-carboxy-3-phenyl)propyl)benzyl]benzimidazol

0,44 g (0,8 mMol) 2-n-Butyl-1-[4-[(2,2-bis-ethoxycarbonyl-3-phenyl)propyl]benzyl]benzimidazol und 0,12 g Natriumhydroxid werden in 30 ml Wasser aufgenommen und 8 Stunden unter Rückfluß gekocht. Danach wird mit Eisessig angesäuert. Das auskristallisierte Produkt wird abgesaugt und über eine Kieselgelsäule (Korngröße: 0,063-0,02 mm) gereinigt, wobei als Elutionsmittel Methylenchlorid/Methanol (19:1) verwendet wird. Die einheitlichen Faktionen werden vereinigt, eingedampft, mit Ether verrieben, abgesaugt und getrocknet.
Ausbeute: 0,1 g (39 % der Theorie),
Schmelzpunkt: 139-140°C

| $C_{28}H_{30}N_2O_2$ (426,56) | | | | | | |
|---|---|---|---|---|---|---|
| Ber.:<br>Gef.: | C | 78,84<br>78,72 | H | 7,09<br>6,96 | N | 6,57<br>6,56 |

Beispiel 66

2-n-Butyl-1-[4-[(2-carboxy-2-phenyl)ethyl)benzyl]benzimidazol

a) 2-n-Butyl-1-[4-[(2,2-bis-ethoxycarbonyl-2-phenyl)ethyl)benzyl]benzimidazol

Hergestellt analog Beispiel 65c aus 2-n-Butyl-1-[(4-chlormethyl)benzyl]benzimidazol und Phenylmalonsäurediethylester/Kalium-tert.butylat.
Ausbeute: 82 % der Theorie,
$R_f$-Wert: 0,20 (Kieselgel; Laufmittel: Methylenchlorid/Methanol = 40:1)

b) 2-n-Butyl-1-[4-[(2-carboxy-2-phenyl)ethyl)benzyl]benzimidazol

Hergestellt analog Beispiel 65d aus 2-n-Butyl-1-[4-[(2,2-bis-ethoxycarbonyl-2-phenyl)ethyl]benzyl]-benzimidazol und wässriger Natronlauge in Ethanol.
Ausbeute: 37 % der Theorie,
Schmelzpunkt: 171-172°C

| $C_{27}H_{28}N_2O_2$ (412,54) | | | | | | |
|---|---|---|---|---|---|---|
| Ber.:<br>Gef.: | C | 78,44<br>78,61 | H | 6,66<br>6,84 | N | 6,88<br>6,79 |

Beispiel 67

2-n-Propyl-5-(N-ethyl-cyclohexylcarbonylamino)-3-[4-[(α-carboxy)benzyloxy]benzyl]imidazo[4,5-b]pyridin

Hergestellt analog Beispiel 1b aus 2-n-Propyl-5-(N-ethylcyclohexylcarbonylamino)-3-[4-(α-ethoxycarbonyl)benzyloxy]benzyl]imidazo[4,5-b]pyridin und 1N Natronlauge in Ethanol.
Ausbeute: 87 % der Theorie,
Schmelzpunkt: 113-115 °C

| $C_{33}H_{38}N_4O_4$ (554,70) | | | | | | |
|---|---|---|---|---|---|---|
| Ber.:<br>Gef.: | C | 71,46<br>71,60 | H | 6,91<br>6,99 | N | 10,10<br>10,00 |

Beispiel 68

2-n-Propyl-5-(N-cyclohexylaminocarbonyl-ethylamino)-3-[4-[(α-carboxy)benzyloxy]benzyl-imidazo[4,5-b]-pyridin

Hergestellt analog Beispiel 1b aus 2-n-Propyl-5-(N-cyclohexylaminocarbonyl-ethylamino)-3-[4-(α-ethoxycarbonyl)-benzyloxy]benzyl]benzimidazol und 1N Natronlauge in Ethanol.
Ausbeute: 20 % der Theorie,
Schmelzpunkt: 183 °C

| $C_{33}H_{39}N_5O_4$ (569,72) | | | | | | |
|---|---|---|---|---|---|---|
| Ber.:<br>Gef.: | C | 69,57<br>69,80 | H | 6,90<br>6,69 | N | 12,29<br>11,97 |

Beispiel 69

2-n-Butyl-4-methyl-7-[α-(1H-tetrazol-5-yl)benzyloxy]-1-[4-[α-(1H-tetrazol-5-yl)benzyloxy]benzyl]benzimidazol

Hergestellt analog Beispiel 57g aus 2-n-Butyl-4-methyl-7-[α-(1-triphenylmethyl-tetrazol-5-yl)benzyloxy]-1-[4-[α-(1-triphenylmethyl)tetrazol-5-yl)benzyloxy]benzyl]benzimidazol und 4N Salzsäure.
Ausbeute: 52 % der Theorie,
Schmelzpunkt: ab 166 °C (Zers.)

| $C_{35}H_{34}H_{10}O_2$ (626,72) | | | | | | |
|---|---|---|---|---|---|---|
| Ber.:<br>Gef.: | C | 67,68<br>67,70 | H | 5,46<br>5,52 | N | 22,35<br>22,66 |

Beispiel 70

2-n-Butyl-5-methyl-6-(3-benzyl-3,4,5,6-tetrahydro-2(1H)pyrimidinon-1-yl)-3-[4-[α-(1H-tetrazol-5-yl)benzyloxy]-benzyl]imidazo[4,5-b]pyridin

Hergestellt analog Beispiel 57g aus 2-n-Butyl-5-methyl-6-(3-benzyl-3,4,5,6-tetrahydro-2(1H)pyrimidinon-1-yl)-3-[4-[α-(1-triphenylmethyl)tetrazol-5-yl)benzyloxy]benzyl]imidazo[4,5-b]pyridinund 4N Salzsäure.
Ausbeute: 75 % der Theorie,
Schmelzpunkt: 185-187 °C

| $C_{37}H_{39}N_9O_2$ (661,79) | | | | | | |
|---|---|---|---|---|---|---|
| Ber.:<br>Gef.: | C | 69,24<br>68,97 | H | 6,12<br>6,17 | N | 19,64<br>19,91 |

Beispiel 71

2-n-Butyl-5-dimethylaminocarbonylamino-1-[4-[α-(1H-tetrazol-5-yl)benzyloxy]benzyl]benzimidazol

Hergestellt analog Beispiel 57g aus 2-n-Butyl-5-dimethylaminocarbonylamino-1-[4-[α-(1-triphenylmethyl)tetrazol-5-yl)benzyloxy]benzyl]benzimidazol und 4N Salzsäure.
Ausbeute: 66 % der Theorie,
Schmelzpunkt: 207-209 ° C

| $C_{29}H_{32}N_8O_2$ (524,68) | | | | | | |
|---|---|---|---|---|---|---|
| Ber.:<br>Gef.: | C | 64,19<br>63,95 | H | 6,31<br>6,33 | N | 20,65<br>20,44 |

Beispiel 72

2-Ethyl-5,7-dimethyl-3-[4-[α-(1H-tetrazol-5-yl)benzyloxy]benzyl]imidazo[4,5-b]pyridin-semihydrat

Hergestellt analog Beispiel 57g aus 2-Ethyl-5,7-dimethyl-3-[4-[α-(1-triphenylmethyl)tetrazol-5-yl)-benzyloxy]benzyl]imidazo[4,5-b]pyridin-semihydrat und 4N Salzsäure.
Ausbeute: 83 % der Theorie,
Schmelzpunkt: 104-105 ° C

| $C_{25}H_{25}N_7O$ x 0,5 $H_2O$ (448,53) | | | | | | |
|---|---|---|---|---|---|---|
| Ber.:<br>Gef.: | C | 66,95<br>67,05 | H | 5,89<br>5,94 | N | 21,83<br>22,36 |

Beispiel 73

2-n-Propyl-4-methyl-1-[4-[α-(0-ethyl-phosphono)benzylamino]benzyl]benzimidazol-hydrat

a) 2-n-Propyl-4-methyl-1-[4-[α-(0,0-diethyl-phosphono)benzylamino]benzyl]benzimidazol

6,5 g (23,3 mMol) 2-n-Propyl-4-methyl-1-[(4-amino)benzyl]benzimidazol werden in 4,2 g (40 mMol) Benzaldehyd eingerührt. Die Mischung wird 2 Stunden bei Raumtemperatur gerührt. Anschließend werden 10,72 g (77,6 mMol) Diethylphosphit zugegeben, und die Mischung wird 45 Minuten auf 100 ° C erhitzt. Der nach Abkühlung auf Raumtemperatur gebildete Niederschlag wird mit 250 ml Ether verrieben, abgesaugt und an der Luft getrocknet. Anschließend wird aus 150 ml Petrolether/Isopropanol (2:1) umkristallisiert.
Ausbeute: 8,7 g (74 % der Theorie),
Schmelzpunkt: 110-115 ° C

b) 2-n-Propyl-4-methyl-1-[4-[α-(0-ethyl-phosphono)benzylamino]benzyl]benzimidazol-hydrat

0,5 g (1 mMol) 2-n-Propyl-4-methyl-1-[4-[α-(0,0-diethylphosphono)benzylamino]benzyl]benzimidazol und 1,0 g pulverisiertes Kaliumhydroxyd werden in 20 ml Methanol gelöst und 9 Stunden unter Rückfluß erhitzt. Nach Abkühlung auf Raumtemperatur werden 25 ml Eiswasser zugesetzt. Der pH-Wert wird durch Zusatz von Eisessig und konz. Ammoniaklösung auf 6.5 eingestellt. Nach Zusatz von festem Natriumchlorid wird 6 mal mit je 100 ml Essigester extrahiert. Die vereinigten organischen Phasen werden mit gesättigter

Kochsalzlösung gewaschen und über Magnesiumsulfat getrocknet. Nach Verdampfen des Solvens im Vakuum wird der Rückstand mit Ether verrieben und über Kaliumhydroxid getrocknet.
Ausbeute: 0,35 g (73 % der Theorie),
Schmelzpunkt: ab 158 ° C (Zers.)

| $C_{27}H_{32}N_3O_3P$ x $H_2O$ (495,57) | | | | | | |
|---|---|---|---|---|---|---|
| Ber.:<br>Gef.: | C | 65,44<br>65,66 | H | 6,92<br>6,52 | N | 8,48<br>8,49 |

Beispiel 74

2-n-Butyl-1-[4-[α-(0-ethyl-phosphono)benzylamino]benzyl]benzimidazol

a) 2-n-Butyl-1-[4-[α-(0,0-diethyl-phosphono)benzylamino]benzyl]benzimidazol

Hergestellt analog Beispiel 73a aus 2-n-Butyl-1-[(4-amino)benzyl]benzimidazol, Benzaldehyd und Diethylphosphit. Ausbeute: 51 % der Theorie, Schmelzpunkt: 149-153 ° C

b) 2-n-Butyl-1-[4-[α-(0-ethyl-phosphono)benzylamino]benzyl]benzimidazol

Hergestellt analog Beispiel 73b aus 2-n-Butyl-1-[4-α-(0,0-diethyl-phosphono)benzylamino]benzyl]-benzimidazol und methanolischer Kalilauge.
Ausbeute: 67 % der Theorie,
Schmelzpunkt: 118-123 ° C (Zers.)

| $C_{27}H_{32}N_3O_3P$ (477,55) | | | | | | |
|---|---|---|---|---|---|---|
| Ber.:<br>Gef.: | C | 67,91<br>67,74 | H | 6,76<br>7,02 | N | 8,80<br>8,64 |

Beispiel 75

2-n-Butyl-1-[4-[(α-carboxy)benzyloxy]benzyl]benzimidazol

a) 2-n-Butyl-1-[4-[(α-ethoxycarbonyl)benzyloxy)benzyl]benzimidazol

Hergestellt analog Beispiel 1a aus 2-n-Butylbenzimidazol und 4-[(α-Ethoxycarbonylbenzyloxy)benzyl]-bromid.
Ausbeute: 22,0 % der Theorie,
Öl, $R_f$-Wert: 0,65 (Kieselgel; Laufmittel: Essigester/Ethanol = 9:1)

| $C_{28}H_{30}N_2O_3$ (442,60) | | | | | | |
|---|---|---|---|---|---|---|
| Ber.:<br>Gef.: | C | 75,99<br>75,97 | H | 6,83<br>6,79 | N | 6,33<br>5,99 |

b) 2-n-Butyl-1-[4-[(α-carboxy)benzyloxy]benzyl]benzimidazol

Hergestellt analog Beispiel 1b aus 2-n-Butyl-1-[4-[(α-ethoxycarbonyl)benzyloxy]benzyl]benzimidazol und 1 N Natronlauge in Ethanol.
Ausbeute: 38,0 % der Theorie,
Schmelzpunkt: 223-225 ° C

| $C_{26}H_{26}N_2O_3$ (414,51) | | | | | | |
|---|---|---|---|---|---|---|
| Ber.:<br>Gef.: | C | 75,34<br>75,03 | H | 6,32<br>6,34 | N | 6,76<br>6,73 |

Beispiel 76

2-n-Propyl-7-methyl-3-[4-[(α-carboxy)benzyloxy]benzyl]imidazo[4,5-b]pyridin

a) 2-n-Propyl-7-methyl-3-[4-[(α-ethoxycarbonyl)benzyloxy]benzyl]imidazo[4,5-b]pyridin

Hergestellt analog Beispiel 1a aus 2-n-Propyl-7-methyl-imidazo[4,5-b]pyridin und 4-[(α-Ethoxycarbonyl)-benzyloxy]benzylbromid.
Ausbeute: 34,0 % der Theorie,
Öl, $R_f$-Wert: 0,40 (Kieselgel; Laufmittel: Methylenchlorid/Ethanol = 25:1)

b) 2-n-Propyl-7-methyl-3-[4-[(α-carboxy)benzyloxy]benzyl]imidazo[4 5-b]pyridin

Hergestellt analog Beispiel 1b aus 2-n-Propyl-7-methyl-3-[4-[(α-ethoxycarbonyl)benzyloxy]benzyl]-imidazo[4,5-b]pyridin und 1 N Natronlauge in Ethanol.
Ausbeute: 66,4 % der Theorie,
Schmelzpunkt: 108 ° C

| $C_{25}H_{25}N_3O_3$ (415,50) | | | | | | |
|---|---|---|---|---|---|---|
| Ber.:<br>Gef.: | C | 72,27<br>72,25 | H | 6,06<br>6,03 | N | 10,11<br>9,87 |

Beispiel 77

5,7-Dimethyl-2-ethyl-3-[4-[(α-carboxy)benzyloxy]benzyl]imidazo[4,5-b]pyridin-semihydrat

Hergestellt analog Beispiel 1b aus 5,7-Dimethyl-2-ethyl-3-[4-[(α-ethoxycarbonyl)benzyloxy]benzyl]-imidazol[4,5-b]pyridin und 1N Natronlauge in Ethanol.
Ausbeute: 89,3 % der Theorie,
Schmelzpunkt: 251-253 ° C,

| $C_{25}H_{25}N_3O_3$ x 0,5 $H_20$ (424,50) | | | | | | |
|---|---|---|---|---|---|---|
| Ber.:<br>Gef.: | C | 70,73<br>71,00 | H | 6,17<br>5,97 | N | 9,89<br>9,79 |

Beispiel 78

2-n-Butyl-1-[4-[(α-carboxy)benzyloxy]-3,5-dichlorbenzyl]-6-cyclohexylaminocarbonylamino-benzimidazol

Hergestellt analog Beispiel 1b aus 2-n-Butyl-1-[4-[(α-ethoxycarbonyl)benzyloxy]-3,5-dichlorbenzyl]-6-cyclohexylaminocarbonylamino-benzimidazol und 1N Natronlauge in Ethanol.
Ausbeute: 95,6 % der Theorie,
Schmelzpunkt: 251-253 ° C

| $C_{33}H_{36}Cl_2N_4O_4$ (623,58) | | | | | | | | |
|---|---|---|---|---|---|---|---|---|
| Ber.:<br>Gef.: | C | 63,56<br>63,39 | H | 5,82<br>5,88 | N | 8,98<br>8,87 | Cl | 11,37<br>11,85 |

Beispiel 79

2-n-Butyl-1-[4-[(α-carboxy)benzyloxy]-3-methoxybenzyl]-6-dimethylaminocarbonylamino-benzimidazol

Hergestellt analog Beispiel 1b aus 2-n-Butyl-1-[4-[(α-ethoxycarbonyl)benzyloxy]-3-methoxybenzyl]-6-dimethylaminocarbonylamino-benzimidazol und 2N Natronlauge in Ethanol.
Ausbeute: 87,5 % der Theorie,
Schmelzpunkt: 170-172 °C

| $C_{30}H_{34}N_4O_5$ (530,62) | | | | | | |
|---|---|---|---|---|---|---|
| Ber.:<br>Gef.: | C | 67,90<br>67,64 | H | 6,46<br>6,25 | N | 10,56<br>10,33 |

Beispiel 80

2-n-Butyl-1-[4-[(α-carboxy)benzyloxy]-3-methoxybenzyl]-5-dimethylaminocarbonylamino-benzimidazol

Hergestellt analog Beispiel 1b aus 2-n-Butyl-1-[4-[(α-ethoxycarbonyl)benzyloxy]-3-methoxybenzyl]-5-dimethylaminocarbonylamino-benzimidazol und 2N Natronlauge in Ethanol.
Ausbeute: 78,6 % der Theorie,
Schmelzpunkt: 154-156 °C

| $C_{30}H_{34}N_4O_5$ (530,62) | | | | | | |
|---|---|---|---|---|---|---|
| Ber.:<br>Gef.: | C | 67,90<br>67,74 | H | 6,46<br>6,37 | N | 10,56<br>10,24 |

Beispiel 81

2-n-Propyl-1-[4-[(α-carboxy)benzyloxy]-3,5-dimethoxybenzyl]-6-(1-methyl-benzimidazol-2-yl)-4-methyl-benzimidazol-hydrat

Hergestellt analog Beispiel 1b aus 2-n-Propyl-1-[4-[(α-ethoxycarbonyl)benzyloxy]-3,5-dimethoxybenzyl]-6-(1-methyl-benzimidazol-2-yl)-4-methyl-benzimidazol und 2N Natronlauge in Ethanol.
Ausbeute: 91,4 % der Theorie,
Schmelzpunkt: 148-150 °C

| $C_{36}H_{36}N_4O_5 \times H_2O$ (622,72) | | | | | | |
|---|---|---|---|---|---|---|
| Ber.:<br>Gef.: | C | 69,44<br>69,77 | H | 6,25<br>6,09 | N | 9,00<br>8,92 |

Beispiel 82

2-n-Propyl-1-[4-[(α-carboxy)benzyloxy]-3,5-dibrombenzyl]-6-(1-methyl-benzimidazol-2-yl)-4-methyl-benzimidazol-hydrat

Hergestellt analog Beispiel 1b aus 2-n-Propyl-1-[4-[(α-ethoxycarbonyl)benzyloxy]-3,5-dibrombenzyl]-6-(1-methyl-benzimidazol-2-yl)-4-methyl-benzimidazol und 2N Natronlauge in Ethanol.
Ausbeute: 58,4 % der Theorie,
Schmelzpunkt: 229-230 ° C

| $C_{34}H_{30}Br_2N_4O_3$ x $H_2O$ (720,46) | | | | | | |
|---|---|---|---|---|---|---|
| Ber.:<br>Gef.: | C | 56,68<br>56,78 | H | 4,47<br>4,64 | N | 7,77<br>7,75 |

Beispiel 83

2-n-Propyl-6-(2,3-dimethylsuccinimino)-1-[4-[(α-carboxy)benzyloxy]-3,5-dimethoxybenzyl]-benzimidazol-semihydrat

Hergestellt analog Beispiel 1b aus 2-n-Propyl-6-(2,3-dimethylsuccinimino)-1-[4-[(α-ethoxycarbonyl)-benzyloxy]-3,5-dimethoxybenzyl]-benzimidazol und 2N Natronlauge in Ethanol.
Ausbeute: 13,8 % der Theorie,
Schmelzpunkt: 129-131 ° C

| $C_{34}H_{37}N_3O_7$ x O,5 $H_2O$ (608,69) | | | | | | |
|---|---|---|---|---|---|---|
| Ber.:<br>Gef.: | C | 67,09<br>67,29 | H | 6,29<br>6,37 | N | 6,90<br>6,86 |

Beispiel 84

2-n-Butyl-6-(1-cyclohexen-1,2-dicarbonylimino)-1-[4-[(α-carboxy)benzyloxy]benzyl]benzimidazol-semihydrat

Hergestellt analog Beispiel 1b aus 2-n-Butyl-6-(1-cyclohexen-1,2-dicarbonylimino)-1-[4-[(α-ethoxycarbonyl)benzyloxy]benzyl]benzimidazol und 1N Natronlauge in Ethanol.
Ausbeute: 23,3 % der Theorie,
Schmelzpunkt: 247-249 ° C

| $C_{34}H_{33}N_3O_5$ x 0,5 $H_2O$ (572,66) | | | | | | |
|---|---|---|---|---|---|---|
| Ber.:<br>Gef.: | C | 71,30<br>71,47 | H | 5,98<br>6,05 | N | 7,33<br>7,15 |

Beispiel 85

2-n-Propyl-4-methyl-1-[4-[(α-carboxy)-2-chlorbenzyloxy]benzyl]-benzimidazol

Hergestellt analog Beispiel 1b aus 2-n-Propyl-4-methyl-1-[4-[(α-ethoxycarbonyl)-2-chlorbenzyloxy]-benzyl]benzimidazol und 2N Natronlauge in Ethanol.
Ausbeute: 40,7 % der Theorie,
Schmelzpunkt: sintern ab 105 ° C

| $C_{26}H_{25}ClN_2O_3$ (448,97) | | | | | | | | |
|---|---|---|---|---|---|---|---|---|
| Ber.:<br>Gef.: | C | 70,00<br>69,94 | H | 5,62<br>5,72 | N | 6,28<br>6,29 | Cl | 7,91<br>7,92 |

Beispiel 86

2-n-Propyl-4-chlor-6-(1-oxo-2-isoindolin-2-yl)-1-[4-[(α-carboxy)benzyloxy]benzyl]benzimidazol

Hergestellt analog Beispiel 1b aus 2-n-Propyl-4-chlor-6-(1-oxo-2-isoindolin-2-yl)-1-[4-[(α-ethoxycarbonyl)benzyloxy]benzyl]benzimidazol und 2N Natronlauge in Ethanol.
Ausbeute: 61,0 % der Theorie,
Schmelzpunkt: 252-254 ° C

| $C_{33}H_{28}ClN_3O_4$ (566,06) | | | | | | | | |
|---|---|---|---|---|---|---|---|---|
| Ber.:<br>Gef.: | C | 70,00<br>69,81 | H | 5,02<br>5,22 | N | 7,42<br>7,87 | Cl | 6,25<br>6,54 |

Beispiel 87

2-n-Butyl-6-(1-cyclohexen-1,2-dicarbonylimino)-1-[4-[α-(1H-tetrazol-5-yl)benzyloxy]benzyl]benzimidazol

Hergestellt analog Beispiel 57g aus 2-n-Butyl-6-(1-cyclohexen-1,2-dicarbonylimino)-1-[4-[(α-(N-triphenylmethyl)tetrazol-5-yl)benzyloxy]benzyl]benzimidazol und 85%iger Ameisensäure in Methylenchlorid.
Ausbeute: 43,7 % der Theorie,
Schmelzpunkt: 149-151 ° C

| $C_{34}H_{33}N_7O_3$ (587,68) | | | | | | |
|---|---|---|---|---|---|---|
| Ber.:<br>Gef.: | C | 69,49<br>69,40 | H | 5,66<br>5,83 | N | 16,68<br>16,31 |

Beispiel 88

2-n-Butyl-5-methyl-6-dimethylaminocarbonylamino-3-[4-[α-(1H-tetrazol-5-yl)benzyloxy)benzyl]imidazol[4,5-b]pyridin

Hergestellt analog Beispiel 57g aus 2-n-Butyl-5-methyl-6-dimethylaminocarbonylamino-3-[4-[(α-(N-triphenylmethyl)tetrazol-5-yl)benzyloxy]benzyl]imidazol[4,5-b]pyridin und 4N Salzsäure in Ethanol.
Ausbeute: 75 % der Theorie,
Schmelzpunkt: 198-200 ° C

| $C_{29}H_{33}N_9O_2$ (539,64) | | | | | | |
|---|---|---|---|---|---|---|
| Ber.:<br>Gef.: | C | 64,54<br>64,37 | H | 6,16<br>6,24 | N | 23,36<br>23,57 |

Beispiel 89

2-n-Butyl-6-(cyclohexylaminocarbonyl-N-methylamino)-1-[4-[α-(1H-tetrazol-5-yl)benzyloxy]benzyl]-benzimidazol

Hergestellt analog Beispiel 57g aus 2-n-Butyl-6-(cyclohexylaminocarbonyl-N-methylamino)-1-[4-[(α-(N-triphenylmethyl)tetrazol-5-yl)benzyloxy]benzyl]benzimidazol und 4N Salzsäure in Ethanol.
Ausbeute: 85,7 % der Theorie,
Schmelzpunkt: 195-197 ° C

| $C_{34}H_{40}N_8O_2$ (592,74) | | | | | | |
|---|---|---|---|---|---|---|
| Ber.:<br>Gef.: | C | 68,89<br>68,82 | H | 6,80<br>6,91 | N | 18,90<br>18,77 |

Beispiel 90

5,7-Dimethyl-2-ethyl-3-[4-[α-(1H-tetrazol-5-yl)benzyloxy]-3,5-dichlorbenzyl]imidazo[4,5-b]pyridin-hydrat

Hergestellt analog Beispiel 57g aus 5,7-Dimethyl-2-ethyl-3-[4-[(α-(N-triphenylmethyl)tetrazol-5-yl)-benzyloxy]-3,5-dichlorbenzyl]imidazo[4,5-b]pyridin und 85%iger Ameisensäuren in Methylenchlorid.
Ausbeute: 77,2 % der Theorie,
Schmelzpunkt: 143-145 ° C

| $C_{25}H_{23}Cl_2N_7O$ x $H_2O$ (526,42) | | | | | | | | |
|---|---|---|---|---|---|---|---|---|
| Ber.:<br>Gef.: | C | 57,04<br>57,51 | H | 4,78<br>4,82 | N | 18,62<br>19,03 | Cl | 13,47<br>13.38 |

Beispiel 91

2-n-Butyl-6-cyclohexylaminocarbonylamino-1-[4-[α-(1H-tetrazol-5-yl)benzyloxy]-3,5-dichlorbenzyl]-benzimidazol-semihydrat

Hergestellt analog Beispiel 57g aus 2-n-Butyl-6-cyclohexylaminocarbonylamino-1-[4-[(α-(N-triphenylmethyl)tetrazol-5-yl)benzyloxy]-3,5-dichlorbenzyl]-benzimidazol und 85%ige Ameisensäure in Methylenchlorid.
Ausbeute: 83 % der Theorie,
Schmelzpunkt: 129-131 ° C

| $C_{33}H_{36}Cl_2N_8O_2$ x 0,5 $H_2O$ (655,62) | | | | | | |
|---|---|---|---|---|---|---|
| Ber.:<br>Gef.: | C | 60,36<br>60,40 | H | 5,68<br>5,78 | N | 17,07<br>17,19 |

Beispiel 92

2-n-Butyl-6-dimethylaminocarbonylamino-1-[4-[α-(1H-tetrazol-5-yl)benzyloxy]-3-methoxybenzyl]benzimidazol

Hergestellt analog Beispiel 57g aus 2-n-Butyl-6-dimethylaminocarbonylamino-1-[4-[(α-(N-triphenylmethyl)tetrazol-5-yl)benzyloxy]-3-methoxybenzyl]benzimidazol und 4N Salzsäure in Ethanol.
Ausbeute: 75 % der Theorie,
Schmelzpunkt: 158-162 ° C

| $C_{30}H_{34}N_8O_3$ (554,65) | | | | | | |
|---|---|---|---|---|---|---|
| Ber.:<br>Gef.: | C | 64,97<br>64,71 | H | 6,18<br>6,20 | N | 20,20<br>19,91 |

Beispiel 93

2-n-Propyl-4-methyl-6-(1-methyl-benzimidazol-2-yl)-1-[4-[α-(1H-tetrazol-5-yl)benzyloxy]-3,5-dimethoxybenzyl]-benzimidazol-hydrat

Hergestellt analog Beispiel 57g aus 2-n-Propyl-4-methyl-6-(1-methyl-benzimidazol-2-yl)-1-[4-[(α-(N-triphenylmethyl)tetrazol-5-yl)benzyloxy]-3,5-dimethoxybenzyl]benzimidazol und 85%iger Ameisensäure in Methylenchlorid.
Ausbeute: 83 % der Theorie,
Schmelzpunkt: 152-154°C

| $C_{36}H_{36}N_8O_3$ x $H_2O$ (646,75) | | | | | | |
|---|---|---|---|---|---|---|
| Ber.:<br>Gef.: | C | 66,86<br>67,08 | H | 5,97<br>5,99 | N | 17,33<br>16,97 |

Beispiel 94

2-n-Propyl-4-methyl-6-(2,3-dimethylsuccinimino)-1-[4-[α-(1H-tetrazol-5-yl)benzyloxy]-3,5-dimethoxybenzyl]-benzimidazol-semihydrat

Hergestellt analog Beispiel 57g aus 2-n-Propyl-4-methyl-6-(2,3-dimethylsuccinimino)-1-[4-[(α-(N-triphenylmethyl)tetrazol-5-yl)benzyloxy]-3,5-dimethoxybenzyl]benzimidazol und 85%iger Ameisensäure in Methylenchlorid.
Ausbeute: 74,6 % der Theorie,
Schmelzpunkt: 192-194°C

| $C_{34}H_{37}N_7O_5$ x 0,5 $H_2O$ (632,71) | | | | | | |
|---|---|---|---|---|---|---|
| Ber.:<br>Gef.: | C | 64,54<br>64,46 | H | 6,05<br>6,10 | N | 15,50<br>15,25 |

Beispiel 95

2-n-Propyl-4-methyl-6-(1-methyl-benzimidazol-2-yl)-1-[4-[α-(1H-tetrazol-5-yl)benzyloxy]-3,5-dibrombenzyl]-benzimidazol

Hergestellt analog Beispiel 57g aus 2-n-Propyl-4-methyl-6-(1-methyl-benzimidazol-2-yl)-1-[4-[(α-(N-triphenylmethyl)tetrazol-5-yl)benzyloxy]-3,5-dibrombenzyl]benzimidazol und 85%iger Ameisensäure in Methylenchlorid.
Ausbeute: 21,6 % der Theorie,
Schmelzpunkt: 132-134°C

| $C_{34}H_{30}Br_2N_8O$ (726,47) | | | | | | | | |
|---|---|---|---|---|---|---|---|---|
| Ber.:<br>Gef.: | C | 56,21<br>56,53 | H | 4,16<br>4,50 | N | 15,42<br>15,42 | Br | 21,99<br>22,03 |

Beispiel 96

2-n-Butyl-5-dimethylaminocarbonylamino-1-[4-[α-(1H-tetrazol-5-yl)benzyloxy]-3-methoxybenzyl]-benzimidazol

Hergestellt analog Beispiel 57g aus 2-n-Butyl-5-dimethylaminocarbonylamino-1-[4-[(α-(N-triphenylmethyl)tetrazol-5-yl)benzyloxy]-3-methoxybenzyl]benzimidazol und 4N Salzsäure in Ethanol.
Ausbeute: 88,2 % der Theorie,
Schmelzpunkt: 181-185 °C

| $C_{30}H_{34}N_8O_3$ (554,66) | | | | | | |
|---|---|---|---|---|---|---|
| Ber.:<br>Gef.: | C | 64,96<br>65,18 | H | 6,18<br>5,92 | N | 20,20<br>19,97 |

Beispiel 97

5,7-Dimethyl-2-ethyl-3-[4-[α-(O-ethyl-phosphono)benzyloxy]benzyl]imidazo[4,5-b]pyridin Natriumsalz-semihydrat

Hergestellt analog Beispiel 73b aus 5,7-Dimethyl-2-ethyl-3-[4-[α-(O,O-diethylphosphono)benzyloxy]-benzyl]imidazo[4,5-b]pyridin und Kaliumhydroxid in Methanol.
Ausbeute: 52 % der Theorie,
Schmelzpunkt: ab 150 °C (Zers.)

| $C_{26}H_{30}N_4NaO_3P \times 0,5\ H_2O$ (509,52) | | | | | | |
|---|---|---|---|---|---|---|
| Ber.:<br>Gef.: | C | 61,29<br>60,95 | H | 6,33<br>6,34 | N | 11,00<br>10,98 |

Beispiel 98

2-n-Propyl-4-methyl-1-[4-[α-(O-ethyl-phosphono)-N-methylbenzylamino]benzyl]benzimidazol-semihydrat

a) 2-n-Propyl-4-methyl-1-[4-[α-(O,O-diethylphosphono)-N-methyl-benzylamino[benzyl]benzimidazol

1,1 g (2 mMol) 2-n-Propyl-4-methyl-1-[4-[a-(O,O-diethylphosphono)-benzylamino]benzyl]benzimidazol werden in 1 ml Ameisensäure gelöst und mit 2 ml einer 37%igen Formalinlösung versetzt. Die Mischung wird 2 Stunden bei 100 °C gerührt, abgekühlt und auf Eis gegossen. Nach Zusatz von konz. Ammoniak wird 2 x mit Essigester extrahiert. Die vereinigten organischen Phasen werden mit gesättigter Kochsalzlösung gewaschen und über Magnesiumsulfat getrocknet. Die organische Phase wird im Vakuum eingedampft und der erhaltene Rückstand über eine Kieselgelsäule (Korngröße: 0,063-0,02 mm, Essigester/Petrolether = 1:1 bis 1:2) gereinigt. Die einheitlichen Fraktionen werden vereinigt und im Vakuum eingeengt.
Ausbeute: 0,60 g (58 % der Theorie),
$R_f$-Wert: 0,45 (Kieselgel; Laufmittel: Petrolether/Essigester = 1:1)

b) 2-n-Propyl-4-methyl-1-[4-[α-(O-ethyl-phosphono)-N-methyl-benzylamino]benzyl]benzimidazol-semihydrat

Hergestellt analog Beispiel 73b aus 2-n-Propyl-4-methyl-1-[4-[α-(O,O-diethylphosphono)-N-methyl-benzylamino]benzyl]benzimidazol und Kaliumhydroxid in Methanol.
Ausbeute: 77 % der Theorie,
Schmelzpunkt: ab 106 °C (Zers.)

| $C_{28}H_{34}N_3O_3P$ x 0,5 $H_2O$ (500,59) | | | | | | |
|---|---|---|---|---|---|---|
| Ber.:<br>Gef.: | C | 67,18<br>67,25 | H | 7,05<br>7,02 | N | 8,40<br>8,42 |

Beispiel 99

2-n-Propyl-4-methyl-1-[4-[α-(O-ethyl-phosphono)benzylamino]benzyl]benzimidazol-hydrobromid

0,5 g (1,0 mMol) 2-n-Propyl-4-methyl-1-[4-[α-(0,O-diethylphosphono)benzylamino]benzyl]benzimidazol werden in 10 ml 48%iger Bromwasserstoffsäure gelöst und 5 Stunden zum Rückfluß erhitzt. Nach Abkühlung auf Raumtemperatur wird durch Zusatz von konzentriertem Ammoniak und Eisessig auf pH 6 eingestellt. Der gebildete Niederschlag wird abgesaugt und bei 40°C über Kaliumhydroxid getrocknet.
Ausbeute: 47 % der Theorie,
Schmelzpunkt: ab 140°C (Zers.)

| $C_{25}H_{28}N_3O_3P$ x HBr (530,42) | | | | | | |
|---|---|---|---|---|---|---|
| Ber.:<br>Gef.: | C | 56,61<br>56,48 | H | 5,51<br>5,80 | N | 7,92<br>8,05 |

Beispiel 100

2-n-Propyl-4-methyl-1-[4-[(α-methansulfonylaminocarbonyl)benzyloxy]benzyl]benzimidazol

410 mg (1 mMol) 2-n-Propyl-4-methyl-1-[4-[(α-carboxy)benzyloxy]benzyl]benzimidazol werden in 100 ml Thionylchlorid gelöst, mit einem Tropfen Dimethylformamid versetzt und 1 Stunde unter Rückfluß erhitzt. Anschließend wird nach Zusatz von Toluol zur Trockene eingeengt und in 30 ml Aceton aufgenommen. Nach Zusatz von 1 ml Triethylamin und 150 mg (1,58 mMol) Methansulfonsäureamid wird 2 Stunden bei Raumtemperatur gerührt. Anschließend wird das Reaktionsgemisch eingeengt, der Rückstand über eine Kieselgelsäule (Korngröße: 0,063 bis 0,02 mm; Methylenchlorid/Ethanol = 50:1, 19:1, 9:1 und 4:1) gereinigt. Die einheitlichen Fraktionen werden vereinigt, eingedampft und der erhaltene Rückstand mit Ether verrieben und getrocknet.
Ausbeute: 15,3 % der Theorie,
Schmelzpunkt: 139-141°C

| $C_{27}H_{29}N_3O_4S$ (491,60) | | | | | | |
|---|---|---|---|---|---|---|
| Ber.:<br>Gef.: | C | 65,95<br>65,67 | H | 5,94<br>6,27 | N | 8,54<br>8,89 |

Beispiel 101

2-Ethyl-4-methyl-1-[4-[(α-carboxy)benzyloxy]benzyl]-6-(n-butansultam-1-yl)benzimidazol

Hergestellt analog Beispiel 1b aus 2-Ethyl-4-methyl-1-[4-[(α-ethoxycarbonyl)benzyloxy]benzyl]-6-(n-butansultam-1-yl)benzimidazol und 2N Natronlauge in Ethanol.

Beispiel 102

2-Ethyl-4-methyl-1-[4-[α-(1H-tetrazol-5-yl)benzyloxy]benzyl]-6-(n-butansultam-1-yl)benzimidazol

Hergestellt analog Beispiel 57g aus 2-Ethyl-4-methyl-1-[4-[(α-(N-triphenylmethyl)tetrazol-5-yl)-benzyloxy]benzyl]-6-(n-butansultam-1-yl)-benzimidazol und 4N Salzsäure in Ethanol.

Beispiel 103

2-n-Butyl-1-[4-[α-(ethoxycarbonyl)-α-(2-pyridyl)methyloxy]benzyl]-6-(N-cyclohexylaminocarbonyl-methylamino)-benzimidazol

Hergestellt analog Beispiel 1a aus 2-n-Butyl-1-(4-hydroxybenzyl)-6-(N-cyclohexylaminocarbonyl-methylamino)-benzimidazol und α-Brom-pyridyl-2-essigsäure-ethylester.
Ausbeute: 90,0 % der Theorie,
$R_f$-Wert: 0,70 (Kieselgel; Essigester/Ethanol/Ammoniak = 90:10:1)

| $C_{33}H_{44}N_5O_4$ (574,76) | | | | | | |
|---|---|---|---|---|---|---|
| Ber.:<br>Gef.: | C | 70,20<br>69,97 | H | 7,40<br>7,26 | N | 11,69<br>11,21 |

Beispiel 104

2-n-Butyl-3-[4-[(α-carboxy)benzyloxy]-3-methoxybenzyl]-5-methyl-6-dimethylaminocarbonylamino-imidazo-[4,5-b]pyridin

Hergestellt analog Beispiel 1b aus 2-n-Butyl-3-[4-[(α-ethoxycarbonyl)benzyloxy]-3-methoxybenzyl]-5-methyl-6-dimethylaminocarbonylamino-imidazo[4,5-b]pyridin und 2 N Natronlauge in Ethanol.
Ausbeute: 91,8 % der Theorie,
Schmelzpunkt: 174-176 °C
$R_f$-Wert: 0,60 (Kieselgel; Essigester/Ethanol/Ammoniak = 50:45:5)

| $C_{30}H_{35}N_5O_5$ (545,65) | | | | | | |
|---|---|---|---|---|---|---|
| Ber.:<br>Gef.: | C | 66,04<br>66,25 | H | 6,47<br>6,49 | N | 12,84<br>12,95 |

Beispiel 105

2-n-Butyl-1-[4-[α-(ethoxycarbonyl)-α-(2-pyridyl)methyloxy]benzyl]-6-dimethylaminocarbonylamino-benzimidazol

Hergestellt analog Beispiel 1a aus 2-n-Butyl-1-(4-hydroxybenzyl)-6-dimethylaminocarbonylamino-benzimidazol und α-Brom-pyridyl-2-essigsäure-ethylester.
Ausbeute: 87,5 % der Theorie,
$R_f$-Wert: 0,50 (Kieselgel; Essigester/Ethanol/Ammoniak = 90:10:1)

| $C_{30}H_{35}N_5O_4$ (529,65) | | | | | | |
|---|---|---|---|---|---|---|
| Ber.:<br>Gef.: | C | 68,03<br>68,09 | H | 6,66<br>6,83 | N | 13,22<br>13,06 |

Beispiel 106

2-n-Butyl-1-[4-[α-(ethoxycarbonyl)-α-(2-pyridyl)methyloxy]benzyl]-6-cyclohexylcarbonylamino-benzimidazol

Hergestellt analog Beispiel 1a aus 2-n-Butyl-1-(4-hydroxybenzyl)-6-cyclohexylcarbonylamino-benzimidazol und α-Brompyridyl-2-essigsäure-ethylester.
Ausbeute: 75,3 % der Theorie,
$R_f$-Wert: 0,50 (Kieselgel; Essigester/Ethanol/Ammoniak = 90:10:1)

| $C_{34}H_{40}N_4O_4$ (568,73) | | | | | | |
|---|---|---|---|---|---|---|
| Ber.:<br>Gef.: | C | 71,80<br>71,82 | H | 7,09<br>7,21 | N | 9,35<br>9,83 |

Beispiel 107

2-n-Butyl-3-[4-[α-(1H-tetrazol-5-yl)benzyloxy]-3-methoxybenzyl]-5-methyl-6-dimethylaminocarbonylamino-imidazo[4,5-b]pyridin

Hergestellt analog Beispiel 57g aus 2-n-Butyl-3-[4-[α-(N-triphenylmethyl-tetrazol-5-yl)benzyloxy]-3-methoxybenzyl]-5-methyl-6-dimethylaminocarbonylamino-imidazo[4,5-b]pyridin und 4 N Salzsäure in Ethanol.
Ausbeute: 60,0 % der Theorie,
Schmelzpunkt: 197-199 ° C
$R_f$-Wert: 0,60 (Kieselgel; Essigester/Ethanol/Ammoniak = 50:45:5)

| $C_{30}H_{35}N_9O_3$ (569,68) | | | | | | |
|---|---|---|---|---|---|---|
| Ber.:<br>Gef.: | C | 63,25<br>62,94 | H | 6,19<br>6,13 | N | 22,13<br>21,87 |

Beispiel 108

2-n-Butyl-1-[4-[α-(1H-tetrazol-5-yl)-α-(2-pyridyl)methoxy]benzyl]-6-dimethylaminocarbonylamino-benzimidazol

Hergestellt analog Beispiel 57g aus 2-n-Butyl-1-[4-[α-(N-triphenylmethyl-tetrazol-5-yl)-α-(2-pyridyl)-methoxy)benzyl]-6-dimethylaminocarbonylamino-benzimidazol und 4 N Salzsäure in Ethanol.
Ausbeute: 25,0 % der Theorie,
Schmelzpunkt: 197-203 ° C
$R_f$-Wert: 0,50 (Kieselgel; Essigester/Ethanol/Ammoniak = 50:45:5)
$C_{28}H_{31}N_9O_2$ (525,63)
Massenspektrum: (M + H) = 526

Beispiel 109

2-Ethyl-3-[4-[α-(1H-tetrazol-5-yl)benzyloxy]benzyl]-5-methyl-6-dimethylaminocarbonylamino-imidazo[4,5-b]-pyridin

Hergestellt analog Beispiel 57g aus 2-Ethyl-3-[4-[α-(N-triphenylmethyl-tetrazol-5-yl)benzyloxy)benzyl]-5-methyl-6-dimethylaminocarbonylamino-imidazo[4,5-b]pyridin und 4 N Salzsäure in Ethanol.
Ausbeute: 50,0 % der Theorie,
Schmelzpunkt: 234-236 ° C

| $C_{27}H_{29}N_9O_2$ (511,60) | | | | | | |
|---|---|---|---|---|---|---|
| Ber.:<br>Gef.: | C | 63,39<br>63,36 | H | 5,71<br>5,87 | N | 24,64<br>24,52 |

Beispiel 110

2-n-Butyl-4-methyl-1-[4-[α-(1H-tetrazol-5-yl)benzyloxy]benzyl]-6-(2,3-dimethylsuccinimino)benzimidazol

Hergestellt analog Beispiel 57g aus 2-n-Butyl-4-methyl-1-[4-[α-(N-triphenylmethyl-tetrazol-5-yl)-benzyloxy)benzyl]-6-(2,3-dimethylsuccinimino)benzimidazol und 4 N Salzsäure in Ethanol.
Ausbeute: 80,0 % der Theorie,
Schmelzpunkt: 175-177 ° C

| $C_{33}H_{35}N_7O_3$ (577,70) | | | | | | |
|---|---|---|---|---|---|---|
| Ber.:<br>Gef.: | C | 68,61<br>68,45 | H | 6,11<br>6,24 | N | 16,97<br>17,00 |

Beispiel 111

2-n-Butyl-4-methyl-1-[4-[(α-carboxy)benzyloxy]benzyl]-6-cyclohexylcarbonylamino-benzimidazol

Hergestellt analog Beispiel 1b aus 2-n-Butyl-4-methyl-1-[4-[(α-ethoxycarbonyl)benzyloxy)benzyl]-6-cyclohexylcarbonylamino-benzimidazol und 2 N Natronlauge in Ethanol.
Ausbeute: 81,8 % der Theorie,
Schmelzpunkt: 191-193 ° C

| $C_{34}H_{39}N_3O_4$ (553,70) | | | | | | |
|---|---|---|---|---|---|---|
| Ber.:<br>Gef.: | C | 73,75<br>73,70 | H | 7,10<br>6,94 | N | 7,58<br>7,48 |

Beispiel 112

2-n-Butyl-4-methyl-1-[4-[α-(1H-tetrazol-5-yl)benzyloxy]benzyl]-6-cyclohexylcarbonylamino-benzimidazol

Hergestellt analog Beispiel 57g aus 2-n-Butyl-4-methyl-1-[4-[α-(N-triphenylmethyl-tetrazol-5-yl)-benzyloxy]benzyl]-6-cyclohexylcarbonylamino-benzimidazol und 4 N Salzsäure in Ethanol.
Ausbeute: 85,0 % der Theorie,
Schmelzpunkt: 195-198 ° C

| $C_{34}H_{39}N_7O_2$ (577,72) | | | | | | |
|---|---|---|---|---|---|---|
| Ber.:<br>Gef.: | C | 70,68<br>70,81 | H | 6,80<br>6,98 | N | 16,97<br>16,97 |

Beispiel 113

2-n-Butyl-4-methyl-1-[4-[α-(carboxy)benzyloxy]benzyl]-6-tert.butylcarbonylamino-benzimidazol

Hergestellt analog Beispiel 1b aus 2-n-Butyl-4-methyl-1-[4-[(α-ethoxycarbonyl)benzyloxy]benzyl]-6-tert.butylcarbonylamino-benzimidazol und 2 N Natronlauge in Ethanol.
Ausbeute: 78,6 % der Theorie,
Schmelzpunkt: 179-181 ° C

| $C_{32}H_{37}N_3O_4$ (527,67) | | | | | | |
|---|---|---|---|---|---|---|
| Ber.: Gef.: | C | 72,34 72,51 | H | 7,07 6,95 | N | 7,96 7,65 |

Beispiel 114

2-n-Butyl-4-methyl-1-[4-[α-(tetrazol-5-yl)benzyloxy]benzyl]-6-tert.butylcarbonylamino-benzimidazol

Hergestellt analog Beispiel 57g aus 2-n-Butyl-4-methyl-1-[4-[α-(N-triphenylmethyl-tetrazol-5-yl)-benzyloxy]benzyl]-6-tert.butylcarbonylamino-benzimidazol und 4 N Salzsäure in Ethanol.
Ausbeute: 75,0 % der Theorie,
Schmelzpunkt: 191-193 °C

| $C_{32}H_{37}N_7O_2$ (551,70) | | | | | | |
|---|---|---|---|---|---|---|
| Ber.: Gef.: | C | 69,67 69,42 | H | 7,76 7,80 | N | 17,77 17,68 |

Beispiel 115

2-Ethoxy-7-ethoxycarbonyl-1-[4-(α-ethoxycarbonyl)benzyloxy]benzyl]benzimidazol

Hergestellt analog Beispiel 63a aus 2-Ethoxy-7-ethoxycarbonyl-1-(4-hydroxy)benzyl-benzimidazol und 2-Brom-phenylessigsäureethylester.
Ausbeute: 45,5 % der Theorie,
Öl, $R_f$-Wert. 0,30 (Kieselgel; Essigester/Petrolether = 30:70)

Beispiel 116

2-Ethoxy-7-carboxy-1-[4-(α-carboxy)benzyloxy]benzyl]benzimidazol-semihydrat

Hergestellt analog Beispiel 1b aus 2-Ethoxy-7-ethoxycarbonyl-1-[4-[(α-ethoxycarbonyl)benzyloxy]-benzyl]benzimidazol und 1N Natronlauge in Ethanol bei 80 °C.
Ausbeute: 90,9 % der Theorie,
Schmelzpunkt: 180-182 °C

| $C_{25}H_{22}N_2O_6$ x 0,5 $H_2O$ (455,46) | | | | | | |
|---|---|---|---|---|---|---|
| Ber.: Gef.: | C | 65,92 65,89 | H | 5,09 4,97 | N | 6,15 6,17 |

Beispiel 117

2-Ethoxy-7-ethoxycarbonyl-1-[4-(α-(carboxy)benzyloxy]benzyl]benzimidazol

Hergestellt analog Beispiel 1b aus 2-Ethoxy-7-ethoxycarbonyl-1-[4-[(α-(ethoxycarbonyl)benzyloxy]-benzyl]benzimidazol und 1N Natronlauge in Ethanol bei Raumtemperatur.
Ausbeute: 59,6 % der Theorie,
Schmelzpunkt: 185-187 °C

| $C_{27}H_{26}N_2O_6$ (474,50) | | | | | | |
|---|---|---|---|---|---|---|
| Ber.:<br>Gef.: | C | 68,34<br>67,84 | H | 5,52<br>5,64 | N | 5,90<br>5,90 |

Beispiel 118

2-Ethoxy-7-carboxy-1-[4-[α-(1H-tetrazol-5-yl)benzyloxy]benzyl]benzimidazol-semihydrat

Hergestellt analog Beispiel 1b aus 2-Ethoxy-7-ethoxycarbonyl-1-[4-[(α-(N-triphenylmethyl)tetrazol-5-yl)-benzyloxy]benzyl]benzimidazolund 1N Natronlauge in Ethanol.
Ausbeute: 78,3 % der Theorie,
Schmelzpunkt: 154-156 ° C (Zers.)

| $C_{25}H_{22}N_6O_4$ x 0,5 $H_2O$ (479,50) | | | | | | |
|---|---|---|---|---|---|---|
| Ber.:<br>Gef.: | C | 62,62<br>62,76 | H | 4,83<br>4,92 | N | 17,52<br>17,24 |

Beispiel 119

2-Ethoxy-1-[4-[(α-carboxy)benzyloxy]benzyl)-1H-benzimidazol-4-carbonsäure

Hergestellt analog Beispiel 1b aus 2-Ethoxy-1-[4-[(α-ethoxycarbonyl)benzyloxy]benzyl]-4-ethoxycarbonyl-1H-benzimidazol und 1N Natronlauge in Ethanol.
Ausbeute: 34,9 % der Theorie,
Schmelzpunkt: 131-133 ° C
$R_f$-Wert: 0,25 (Kieselgel; Essigester/Ethanol/Ammoniak = 80:40:2)

Bei den nachfolgenden pharmazeutischen Anwendungsbeispielen kann als Wirksubstanz jede geeignete Verbindung der Formel I, insbesondere die Verbindungen A bis I des pharmakologischen Versuchsberichtes eingesetzt werden:

Beispiel I

| Ampullen, enthaltend 50 mg Wirkstoff pro 5 ml | |
|---|---|
| Wirkstoff<br>$KH_2PO_4$<br>$Na_2HPO_4$ x $2H_20$<br>NaCl<br>Wasser für Injektionszwecke ad | 50 mg<br>2 mg<br>50 mg<br>12 mg<br>5 ml |

Herstellung:

In einem Teil des Wassers werden die Puffersubstanzen und das Isotonans gelöst. Der Wirkstoff wird zugegeben und nach vollständiger Lösung mit Wasser auf das Nennvolumen aufgefüllt.

Beispiel II

| Ampullen, enthaltend 100 mg Wirkstoff pro 5 ml | |
|---|---|
| Wirkstoff | 100 mg |
| Methylglucamin | 35 mg |
| Glykofurol | 1000 mg |
| Polyethylenglykol-Polypropylenglykol-Blockpolymer | 250 mg |
| Wasser für Injektionszwecke ad | 5 ml |

Herstellung:

In einem Teil des Wassers wird Methylglucamin gelöst und der Wirkstoff unter Rühren und Erwärmen in Lösung gebracht. Nach Zugabe der Lösungsmittel wird mit Wasser auf das Nennvolumen aufgefüllt.

Beispiel III

| Tabletten, enthaltend 50 mg Wirkstoff | |
|---|---|
| Wirkstoff | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

Herstellung:

Der Wirkstoff, $CaHPO_4$, Milchzucker und Maisstärke werden mit einer wässrigen PVP-Lösung gleichmäßig befeuchtet. Die Masse wird durch ein 2-mm-Sieb gegeben, im Umlufttrockenschrank bei 50 °C getrocknet und erneut gesiebt.

Nach Zumischen des Schmiermittels wird das Granulat auf einer Tablettiermaschine verpresst.

Beispiel IV

| Dragees, enthaltend 50 mg Wirkstoff | |
|---|---|
| Wirkstoff | 50,0 mg |
| Lysin | 25,0 mg |
| Milchzucker | 60,0 mg |
| Maisstärke | 34,0 mg |
| Gelatine | 10,0 mg |
| Magnesiumstearat | 1,0 mg |
| | 180,0 mg |

Herstellung:

Der Wirkstoff wird mit den Hilfsstoffen gemischt und mit einer wässrigen Gelatine-Lösung befeuchtet. Nach Siebung und Trocknung wird das Granulat mit Magnesiumstearat vermischt und zu Kernen verpresst.

Die so hergestellten Kerne werden nach bekannten Verfahren mit einer Hülle überzogen. Der Dragiersuspension oder -lösung kann Farbstoff zugegeben werden.

Beispiel V

| Dragees, enthaltend 100 mg Wirkstoff | |
|---|---|
| Wirkstoff | 100,0 mg |
| Lysin | 50,0 mg |
| Milchzucker | 86,0 mg |
| Maisstärke | 50,0 mg |
| Polyvinylpyrrolidon | 2,8 mg |
| Mikrokristalline Cellulose | 60,0 mg |
| Magnesiumstearat | 1,2 mg |
| | 350,0 mg |

Herstellung:

Der Wirkstoff wird mit den Hilfsstoffen gemischt und mit einer wässrigen PVP-Lösung befeuchtet. Die feuchte Masse wird durch ein 1,5-mm-Sieb gegeben und bei 45 °C getrocknet. Nach dem Trocknen wird erneut gesiebt und das Magnesiumstearat zugemischt. Diese Mischung wird zu Kernen verpreßt.

Die so hergestellten Kerne werden nach bekannten Verfahren mit einer Hülle überzogen. Der Dragiersuspension oder -lösung können Farbstoffe zugegeben werden.

Beispiel VI

| Kapseln, enthaltend 250 mg Wirkstoff | |
|---|---|
| Wirkstoff | 250,0 mg |
| Maisstärke | 68,5 mg |
| Magnesiumstearat | 1,5 mg |
| | 320,0 mg |

Herstellung:

Wirkstoff und Maisstärke werden gemischt und mit Wasser befeuchtet. Die feuchte Masse wird gesiebt und getrocknet. Das trockene Granulat wird gesiebt und mit Magnesiumstearat gemischt. Die Endmischung wird in Hartgelatinekapseln Größe 1 abgefüllt.

Beispiel VII

| Orale Suspension, enthaltend 50 mg Wirkstoff pro 5 ml | |
|---|---|
| Wirkstoff | 50,0 mg |
| Hydroxyethylcellulose | 50,0 mg |
| Sorbinsäure | 5,0 mg |
| Sorbit 70%ig | 600,0 mg |
| Glycerin | 200,0 mg |
| Aroma | 15,0 mg |
| Wasser ad | 5,0 ml |

Herstellung:

Destilliertes Wasser wird auf 70 ° C erhitzt. Hierin wird unter Rühren Hydroxyethylcellulose gelöst. Durch Zugabe von Sorbitlösung und Glycerin wird auf Raumtemperatur abgekühlt. Bei Raumtemperatur werden Sorbinsäure, Aroma und Wirkstoff zugegeben. Zur Entlüftung der Suspension wird unter Rühren evakuiert. Eine Dosis = 50 mg ist enthalten in 5,0 ml.

Beispiel VIII

| Suppositorien, enthaltend 100 mg Wirkstoff | |
|---|---|
| Wirkstoff | 100,0 mg |
| Adeps solidus | 1600.0 mg |
| | 1700,0 mg |

Herstellung:

Das Hartfett wird geschmolzen. Bei 40 ° C wird die gemahlene Wirksubstanz in der Schmelze homogen dispergiert. Es wird auf 38 ° C abgekühlt und in schwach vorgekühlte Suppositorienformen ausgegossen.

**Patentansprüche**

**1.** Phenylalkylderivate der allgemeinen Formel

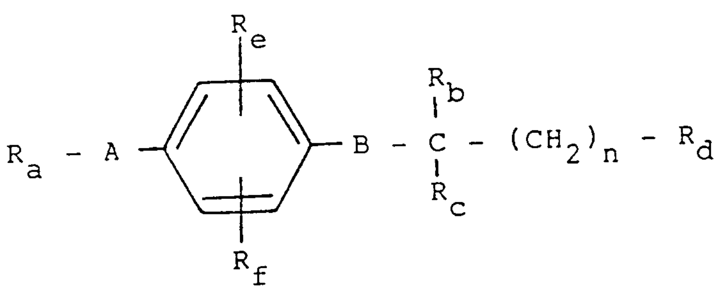

,(I)

in der

n die Zahl 0 oder 1,

A eine geradkettige oder verzweigte Alkylengruppe,

B ein Sauerstoffatom, eine Carbonyl-, Hydroxymethylen-, Sulfenyl-, Sulfinyl- oder Sulfonylgruppe, eine geradkettige oder verzweigte Alkylengruppe, eine Alkylidengruppe mit 2 bis 4 Kohlenstoffatomen, eine 1,1-Cycloalkylengruppe oder eine gegebenenfalls durch eine Alkylgruppe oder durch eine Alkanoylgruppe mit 1 bis 4 Kohlenstoffatomen substituierte Iminogruppe,

$R_a$ ein Chlor- oder Bromatom, eine Hydroxy-, Alkylsulfonyloxy-, Phenylsulfonyloxy- oder Phenylalkylsulfonyloxygruppe oder eine Gruppe der Formel

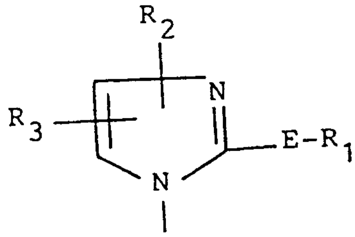 ,

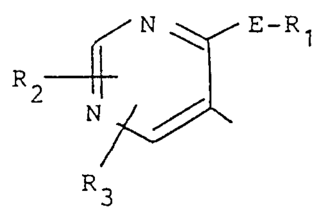

,

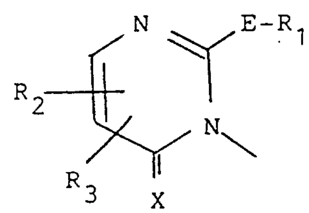

,

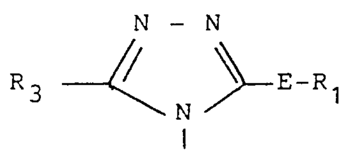

,

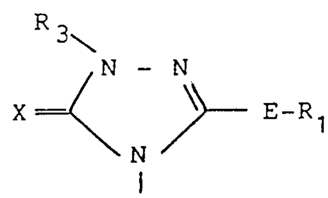

,

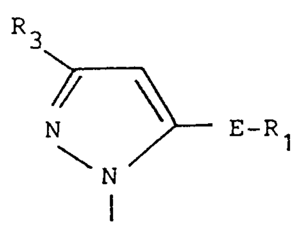

,

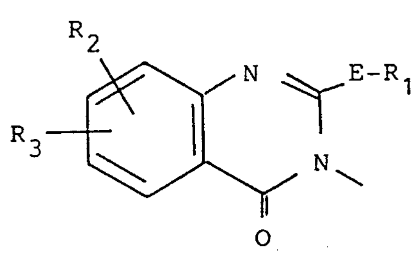

,

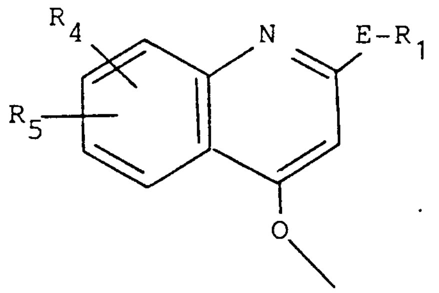

,

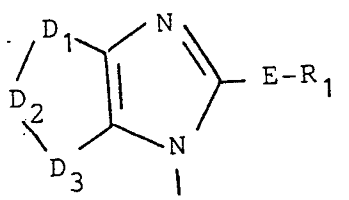

oder

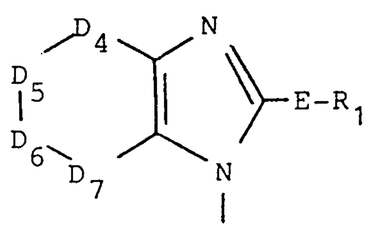

,

in denen einer der Reste $D_1$, $D_2$ oder $D_3$ eine Methylen- oder Iminogruppe und die verbleibenden Reste der Reste $D_1$ bis $D_3$ jeweils Methingruppen, wobei zusätzlich eine Methingruppe durch ein Stickstoffatom ersetzt sein kann und eine der Methingruppen durch den Rest $R_5$ und gegebenenfalls eine weitere der Methingruppen durch den Rest $R_4$ substituiert sein kann,

null, einer oder zwei der Reste $D_4$, $D_5$, $D_6$ oder $D_7$ ein Stickstoffatom und die verbleibenden Reste $D_4$, $D_5$, $D_6$ oder $D_7$ je-weils Methingruppen, wobei zusätzlich eine Methingruppe durch den Rest $R_4$ und eine weitere Methingruppe durch den Rest $R_5$ substituiert sein kann,

E eine Kohlenstoff-Kohlenstoff-Bindung, ein Sauerstoff- oder Schwefelatom, eine Hydroxymethylen- oder Carbonylgruppe oder eine gegebenenfalls durch eine Alkylgruppe mit 1 bis 6 Kohlenstoffatomen, durch eine Cycloalkylgruppe, durch eine Alkanoylgruppe mit 2 bis 5 Kohlenstoffatomen, durch eine Allyl-, Phenyl- oder Benzylgruppe substituierte Iminogruppe,

X ein Sauerstoff- oder Schwefelatom oder eine gegebenfalls durch eine Alkyl-, Phenyl- oder Phenylalkylgruppe substituierte Iminogruppe,

$R_1$ eine geradkettige oder verzweigte Alkylgruppe mit 1 bis 9 Kohlenstoffatomen, eine geradkettige oder verzweigte Alkenyl- oder Alkinylgruppe mit jeweils 2 bis 6 Kohlenstoffatomen, wobei die vorstehend erwähnten gesättigten und ungesättigten Alkylteile jeweils durch eine Cycloalkylgruppe, durch ein Fluor-, Chlor- oder Bromatom, durch eine Hydroxy-, Amino-, Alkylamino-, Dialkylamino- oder $\alpha,\alpha$-

Difluoroethangruppe substituiert sein können, eine Perfluoroalkylgruppe mit 1 bis 4 Kohlenstoffatomen oder eine Cycloalkylgruppe, die durch eine Trifluoromethylgruppe oder eine Alkylgruppe mono- oder disubstituiert sein kann,

$R_2$ ein Wasserstoff-, Fluor-, Chlor- oder Bromatom, eine Alkyl- oder Perfluoralkylgruppe mit jeweils 1 bis 5 Kohlenstoffatomen, eine Cyano- oder Nitrogruppe,

$R_3$ ein Wasserstoffatom, eine Cyanogruppe, eine gegebenfalls durch eine Hydroxy- oder Alkoxygruppe substituierte Alkylgruppe mit 1 bis 6 Kohlenstoffatomen, eine Perfluoralkylgruppe mit 1 bis 6 Kohlenstoffatomen, eine gegebenenfalls durch Fluoratome substituierte Alkenylgruppe mit 3 bis 6 Kohlenstoffatomen, eine Phenylalkyl- oder Phenylalkenylgruppe mit 2 bis 4 Kohlenstoffatomen im Alkenylteil, eine Alkylgruppe mit 1 bis 5 Kohlenstoffatomen, die endständig durch eine Imidazol-1-yl-, Triazolyl-, Tetrazolyl-, Phthalimido-, $R_6COO$-, $R_7S$-, $R_7SO$-, $R_7SO_2$-, $R_7CO$-, $R_7NHCOO$-, $R_7NHCO$-, $R_7NHCONR_7$-, $R_8COHR_7$- oder $R_8SO_2NR_7$-Gruppe substituiert ist, wobei

die Triazolylgruppe zusätzlich mit einer Acetoxy- oder Alkylgruppe mono- oder disubstituiert sein kann und

$R_6$ eine Alkyl- oder Perfluoralkylgruppe mit jeweils 1 bis 8 Kohlenstoffatomen, eine Cycloalkyl-, Phenyl-, Benzyl-, Phenylethyl-, Adamantyl-, Naphthyl-, Naphthylmethyl- oder Naphthylethylgruppe,

$R_7$ ein Wasserstoffatom und die für $R_6$ vorstehend erwähnten Bedeutungen besitzt,

$R_8$ die für $R_7$ vorstehend erwähnten Bedeutungen besitzt und eine gegebenenfalls in 4-Stellung durch eine Alkyl- oder Phenylgruppe substituierte Piperazinogruppe, eine Pyrrolidino-, Piperidino-, Hexamethylenimino-, Morpholino-, $R_7O$- oder $(R_7)_2N$-Gruppe darstellen,

$R_4$ ein Wasserstoff-, Fluor-, Chlor- oder Bromatom, eine Trifluormethylgruppe, eine Cycloalkylgruppe, eine gegebenenfalls durch eine Cycloalkylgruppe, durch eine Hydroxy-, Alkoxy-, Alkylamino-, Dialkylamino-, Alkoxycarbonyl-, Alkylaminocarbonyl- oder Dialkylaminocarbonylgruppe substituierte Alkylgruppe mit 1 bis 6 Kohlenstoffatomen und

$R_5$ ein Wasserstoff-, Fluor-, Chlor- oder Bromatom,

eine geradkettige oder verzweigte Alkyl- oder Perfluoralkylgruppe mit jeweils 1 bis 6 Kohlenstoffatomen, eine Alkenyl- oder Alkinylgruppe mit jeweils 2 bis 6 Kohlenstoffatomen, wobei die vorstehend erwähnten Alkyl- und Alkenylteile jeweils durch eine Heteroaryl-, Hydroxy-, Alkoxy-, Amino-, Alkylamino-, Dialkylamino-, Alkylcarbonylamino-, N-Alkyl-alkylcarbonylamino-, Carboxy-, Alkoxycarbonyl-, Alkylcarbonyloxy-, Piperidinocarbonyl-, Morpholinocarbonyl-, Aminocarbonyl-, Alkylaminocarbonyl-, Dialkylaminocarbonyl-, Tetrazol-5-yl-, Tetrazol-5-yl-aminocarbonyl-, Alkylsulfenyl-, Alkylsulfinyl-, Alkylsulfonyl-, Aminosulfonyl-, Alkylaminosulfonyl-, Dialkylaminosulfonyl-, Alkylsulfonylaminocarbonyl-, Heteroarylaminosulfonyl- oder Alkylcarbonylaminosulfonylgruppe mono- oder disubstituiert sein können,

eine Alkoxygruppe mit 1 bis 7 Kohlenstoffatomen, die in 2-, 3-, 4-, 5-, 6- oder 7-Position durch eine Imidazolyl-, Tetrazolyl-, Benzimidazolyl- oder Tetrahydrobenzimidazolylgruppe substituiert ist, oder eine gegebenenfalls im Alkoxyteil durch eine 1H-Tetrazol-5-yl- oder 1-Triphenylmethyl-tetrazol-5-yl-gruppe substituierte Phenylalkoxygruppe,

eine Carboxygruppe oder eine Gruppe, die in vivo metabolisch in eine Carboxygruppe umgewandelt wird,

eine Alkylsulfonyloxygruppe mit 1 bis 4 Kohlenstoffatomen, eine Benzolsulfonyloxy- oder Phenylalkansulfonyloxygruppe,

eine gegebenenfalls am Stickstoffatom durch eine Alkylgruppe mit 1 bis 6 Kohlenstoffatomen, durch eine Phenyl-, Cycloalkyl-, Phenylalkyl-, Cycloalkylalkyl-, Bicyclohexyl- oder Biphenylgruppe substituierte Acylaminogruppe, in welcher der Acylrest eine Alkanoylgruppe mit 1 bis 7 Kohlenstoffatomen, eine

Alkoxycarbonylgruppe mit insgesamt 2 bis 4 Kohlenstoffatomen, eine Alkylsulfonylgruppe mit 1 bis 6 Kohlenstoffatomen, eine Benzoyl-, Benzolsulfonyl-, Phenylalkansulfonyl-, Naphthalinsulfonyl-, Cycloalkylcarbonyl-, Phenylalkanoyl- oder Cycloalkylalkanoylgruppe darstellt, wobei die vorstehend erwähnten Phenylkerne jeweils durch ein Fluor-, Chlor- oder Bromatom, durch eine Methyl- oder Methoxygruppe mono- oder disubstituiert und die Substituenten gleich oder verschieden sein können,

eine Phthalimino-, Homophthalimino-, 2-Carboxyphenylcarbonylamino- oder 2-Carboxyphenylmethylaminogruppe, wobei eine Carbonylgruppe in einer Phthaliminogruppe durch eine Methylen-, Alkylmethylen- oder Dialkyl-methylengruppe ersetzt sowie eine Methylengruppe in einer Homophthaliminogruppe durch eine oder zwei Alkylgruppen substituiert sein kann, und zusätzlich die vorstehend erwähnten Phenylkerne durch Alkyl- oder Alkoxygruppen mono- oder disubstituiert und gleichzeitig ganz oder teilweise hydriert sein können, wobei die Substituenten gleich oder verschieden sein können,

eine gegebenenfalls durch eine oder zwei Alkylgruppen oder durch eine Tetramethylen- oder Pentamethylengruppe substituierte 5-, 6- oder 7-gliedrige Alkylenimino- oder Alkenyleniminogruppe, in welcher eine Methylengruppe durch eine Carbonyl- oder Sulfonylgruppe ersetzt sein kann,

eine Bicycloalkan-2,3-dicarbonsäureimino- oder Bicycloalken2,3-dicarbonsäureiminogruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann,

eine Glutarsäureiminogruppe, in der die n-Propylengruppe perfluoriert, durch ein oder zwei Alkylgruppen oder durch eine Tetramethylen- oder Pentamethylengruppe substituiert sein kann,

eine gegebenenfalls durch eine Alkyl- oder Phenylgruppe mono- oder oder disubstituierte Maleinsäureimido- oder Succinimidogruppe, wobei die Substituenten gleich oder verschieden sein können,

einen über ein Kohlenstoffatom oder über eine Iminogruppe gebundenen 5-gliedrigen heteroaromatischen Ring, der eine Iminogruppe, ein Sauerstoff- oder Schwefelatom oder eine Iminogruppe und ein Sauerstoff-, Schwefel- oder Stickstoffatom enthält, oder einen über ein Kohlenstoffatom gebundenen 6-gliedrigen heteroaromatischen Ring, der 1 oder 2 Stickstoffatome enthält, wobei sowohl an die 5-gliedrigen als auch an die 6-gliedrigen heteroaromatischen Ringe jeweils über zwei benachbarte Kohlenstoffatome eine n-Propylen-, n-Butylen- oder 1,3-Butadienylgruppe oder über eine Iminogruppe und ein benachbartes Kohlenstoffatom eine n-Propylen-, n-Butylen- oder 1,3-Butadienylgruppe angefügt ist und in einem so gebildeten anellierten Pyridinring eine Methingruppe durch ein Stickstoffatom und eine Vinylengruppe in 3-, 4-Stellung zu dem Stickstoffatom des gebildeten Pyridinringes durch ein Schwefelatom oder in einem so gebildeten anellierten Phenylring eine oder zwei Methingruppen durch N-Atome ersetzt sein können, wobei zusätzlich die vorstehend erwähnten ankondensierten aromatischen oder heteroaromatischen Ringe im Kohlenstoffgerüst durch ein Fluor-, Chlor- oder Bromatom, durch eine Alkyl-, Alkoxy-, Hydroxy-, Phenyl-, Nitro-, Amino-, Alkylamino-, Dialkylamino-, Alkanoylamino-, Cyano-, Carboxy-, Alkoxycarbonyl-, Aminocarbonyl-, Alkylaminocarbonyl-, Dialkylaminocarbonyl-, Trifluormethyl-, Alkanoyl-, Aminosulfonyl-, Alkylaminosulfonyl- oder Dialkylaminosulfonylgruppe monosubstituiert oder durch Fluor- oder Chloratome, durch Methyl-, Methoxy- oder Hydroxygruppen disubstituiert sein können und eine gegebenenfalls in einem Imidazolring vorhandene NH-Gruppe durch eine Alkylgruppe mit 1 bis 6 Kohlenstoffatomen oder durch eine Cycloalkylgruppe substituiert sein kann,

einen über ein Kohlenstoffatom gebundenen Pyrrolidin-, Piperidin- oder Pyridinring, wobei an den Pyridinring über zwei benachbarte Kohlenstoffatome ein Phenylrest ankondensiert und eine zum N-Atom benachbarte Methylengruppe in einem Pyrrolidin- oder Piperidinring durch eine Carbonylgruppe ersetzt sein kann,

eine gegebenenfalls durch eine Alkyl-, Phenylalkyl-, Tetramethylen-, Pentamethylen- oder Hexamethylengruppe substituierte Imidazolidindiongruppe,

eine Pyridazin-3-on- oder Dihydro-pyridazin-3-on-gruppe, die in 2-Stellung durch eine gegebenenfalls durch eine Phenylgruppe substituierte Alkylgruppe und im Kohlenstoffgerüst zusätzlich durch 1 oder 2

Alkylgruppen substituiert sein kann, oder

eine $R_{11}$-$NR_{10}$-CO-$NR_9$-Gruppe, in der

$R_9$ ein Wasserstoffatom, eine Alkylgruppe mit 1 bis 8 Kohlenstoffatomen oder Phenylalkylgruppe,

$R_{10}$ ein Wasserstoffatom, eine Alkylgruppe mit 1 bis 8 Kohlenstoffatomen, eine Alkenylgruppe mit 3 bis 5 Kohlenstoffatomen, eine Phenylalkylgruppe oder eine Cycloalkylgruppe mit 5 bis 7 Kohlenstoffatomen,

$R_{11}$ ein Wasserstoffatom oder eine Alkylgruppe mit 1 bis 6 Kohlenstoffatomen oder

einer der Reste $R_9$, $R_{10}$ oder $R_{11}$ auch eine Bicyclohexyl- oder Biphenylylgruppe oder

$R_{10}$ und $R_{11}$ zusammen mit dem dazwischen liegenden Stickstoffatom eine geradkettige Alkyleniminogruppe mit 4 bis 6 Kohlenstoffatomen oder eine Morpholinogruppe oder

$R_9$ und $R_{11}$ zusammen eine Alkylengruppe mit 2 bis 4 Kohlenstoffatomen darstellen,

$R_b$ eine Cyano-, Trifluormethylcarbonylamino-, Trifluormethylcarbonylaminomethyl-, Trifluormethylsulfonylamino-, Trifluormethylsulfonylaminomethyl-, Alkylsulfonylamino-, Alkylsulfonylaminomethyl-, Arylsulfonylamino-, Arylsulfonylaminomethyl-, Aralkylsulfonylamino-, Aralkylsulfonylaminomethyl-, Arylsulfonylaminocarbonyl-, Benzylsulfonylaminocarbonyl-, Sulfo-, Aminosulfonyl-, Alkylaminosulfonyl-, Aralkylaminosulfonyl-, Arylaminosulfonyl-, Alkylcarbonylaminosulfonyl-, Aralkylcarbonylaminosulfonyl-,Arylcarbonylaminosulfonyl-, Sulfomethyl-, Aminosulfonylmethyl-, Alkylaminosulfonylmethyl-, Aralkylaminosulfonylaminomethyl-, Arylaminosulfonylmethyl-, Alkylcarbonylaminosulfonylmethyl-, Aralkylcarbonylaminosulfonylmethyl-, Arylcarbonylaminosulfonylmethyl-, Phosphino-, O-Alkyl-phosphino-, O-Aralkyl-phosphino-, O-Aryl-phosphino-, Phosphono-, O-Alkyl-phosphono-, O-Aralkyl-phosphono-, O-Arylphosphono-, O,O-Dialkylphosphono-, Phosphono-methyl-, O-Alkyl-phosphono-methyl-, O-Aralkyl-phosphono-methyl-, O-Arylphosphono-methyl-, O,O-Dialkylphosphono-methyl-, Phosphato-, O-Alkyl-phosphato-, O-Aralkyl-phosphato-, O-Aryl-phosphato- oder O,O-Dialkoxy-phosphorylgruppe, eine gegebenenfalls durch eine Alkyl-, Trifluormethyl-, Phenylalkyl- oder Triphenylmethylgruppe substituierte 1H-Tetrazolyl-, 1H-Tetrazolylalkyl-, 1H-Tetrazolylaminocarbonyl- oder Triazolylgruppe, eine Alkylsulfonylaminocarbonyl- oder Perfluoralkylsulfonylaminocarbonylgruppe mit jeweils 1 bis 6 Kohlenstoffatomen im Alkylteil, eine Carboxygruppe, eine Gruppe, die in vivo metabolisch in eine Carboxygruppe übergeführt wird, oder eine Aralkoxycarbonylgruppe,

$R_c$ ein Wasserstoffatom, eine Alkyl-, Aralkyl-, Aryl-, Carboxy- oder Alkoxycarbonylgruppe,

$R_d$ eine geradkettige oder verzweigte Alkylkette mit 1 bis 10 Kohlenstoffatomen, eine geradkettige oder verzweigte Alkenyl- oder Alkinylgruppe mit jeweils 2 bis 10 Kohlenstoffatomen, eine Cycloalkyl- oder Cycloalkylalkylgruppe, eine gegebenenfalls durch Fluor-, Chlor- oder Bromatome, durch Methyl- oder Methoxygruppen mono- oder disubstituierte Phenylgruppe, eine Biphenyl-, Naphthyl- oder Heteroarylgruppe,

$R_e$ und $R_f$ Wasserstoffatome und, wenn

$R_5$ eine Phthalimino-, Homophthalimino-, 2-Carboxyphenylcarbonylamino- oder 2-Carboxyphenylmethylaminogruppe, wobei eine Carbonylgruppe in einer Phthaliminogruppe durch eine Methylen-, Alkylmethylen- oder Dialkyl-methylengruppe ersetzt sowie eine Methylengruppe in einer Homophthaliminogruppe durch eine oder zwei Alkylgruppen substituiert sein kann, und zusätzlich die vorstehend erwähnten Phenylkerne durch Alkyl- oder Alkoxygruppen mono- oder disubstituiert und gleichzeitig ganz oder teilweise hydriert sein können, wobei die Substituenten gleich oder verschieden sein können,

eine Carboxygruppe oder eine Gruppe, die in vivo metabolisch in eine Carboxygruppe umgewandelt wird,

eine gegebenenfalls durch eine oder zwei Alkylgruppen oder durch eine Tetramethylen- oder Pentamethylengruppe substituierte 5-, 6- oder 7-gliedrige Alkylenimino- oder Alkenyleniminogruppe, in welcher eine Methylengruppe durch eine Carbonyl- oder Sulfonylgruppe ersetzt sein kann,

eine Bicycloalkan-2,3-dicarbonsäureimino- oder Bicycloalken-2,3-dicarbonsäureiminogruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann,

eine Glutarsäureiminogruppe, in der die n-Propylengruppe perfluoriert, durch ein oder zwei Alkylgruppen oder durch eine Tetramethylen- oder Pentamethylengruppe substituiert sein kann,

eine gegebenenfalls durch eine Alkyl- oder Phenylgruppe mono- oder disubstituierte Maleinsäureimido- oder Succinimidogruppe, wobei die Substituenten gleich oder verschieden sein können,

einen über ein Kohlenstoffatom oder über eine Iminogruppe gebundenen 5-gliedrigen heteroaromatischen Ring, der eine Iminogruppe, ein Sauerstoff- oder Schwefelatom oder eine Iminogruppe und ein Sauerstoff-, Schwefel- oder Stickstoffatom enthält, oder einen über ein Kohlenstoffatom gebundenen 6-gliedrigen heteroaromatischen Ring, der 1 oder 2 Stickstoffatome enthält, wobei sowohl an die 5-gliedrigen als auch an die 6-gliedrigen heteroaromatischen Ringe jeweils über zwei benachbarte Kohlenstoffatome eine n-Propylen-, n-Butylen- oder 1,3-Butadienylgruppe oder über eine Iminogruppe und ein benachbartes Kohlenstoffatom eine n-Propylen-, n-Butylen- oder 1,3-Butadienylgruppe angefügt ist und in einem so gebildeten anellierten Pyridinring eine Methingruppe durch ein Stickstoffatom und eine Vinylengruppe in 3-, 4-Stellung zu dem Stickstoffatom des gebildeten Pyridinringes durch ein Schwefelatom oder in einem so gebildeten anellierten Phenylring eine oder zwei Methingruppen durch N-Atome ersetzt sein können, wobei zusätzlich die vorstehend erwähnten ankondensierten aromatischen oder heteroaromatischen Ringe im Kohlenstoffgerüst durch ein Fluor-, Chlor- oder Bromatom, durch eine Alkyl-, Alkoxy-, Hydroxy-, Phenyl-, Nitro-, Amino-, Alkylamino-, Dialkylamino-, Alkanoylamino-, Cyano-, Carboxy-, Alkoxycarbonyl-, Aminocarbonyl-, Alkylaminocarbonyl-, Dialkylaminocarbonyl-, Trifluormethyl-, Alkanoyl-, Aminosulfonyl-, Alkylaminosulfonyl- oder Dialkylaminosulfonylgruppe monosubstituiert oder durch Fluor- oder Chloratome, durch Methyl-, Methoxy- oder Hydroxygruppen disubstituiert sein können und eine gegebenenfalls in einem Imidazolring vorhandene NH-Gruppe durch eine Alkylgruppe mit 1 bis 6 Kohlenstoffatomen oder durch eine Cycloalkylgruppe substituiert sein kann, oder

einen über ein Kohlenstoffatom gebundenen Pyrrolidin-, Piperidin- oder Pyridinring, wobei an den Pyridinring über zwei benachbarte Kohlenstoffatome ein Phenylrest ankondensiert und eine zum N-Atom benachbarte Methylengruppe in einem Pyrrolidin- oder Piperidinring durch eine Carbonylgruppe ersetzt sein kann,

eine gegebenenfalls durch eine Alkyl-, Phenylalkyl-, Tetramethylen-, Pentamethylen- oder Hexamethylengruppe substituierte Imidazolidindiongruppe,

eine Pyridazin-3-on- oder Dihydro-pyridazin-3-on-gruppe, die in 2-Stellung durch eine gegebenenfalls durch eine Phenylgruppe substituierte Alkylgruppe und im Kohlenstoffgerüst zusätzlich durch 1 oder 2 Alkylgruppen substituiert sein kann, oder

eine $R_{11}$-$NR_{10}$-CO-$NR_9$-Gruppe, in der $R_9$, $R_{10}$ und $R_{11}$ wie vorstehend erwähnt definiert sind, darstellt,

auch $R_e$ ein Wasserstoff-, Fluor-, Chlor- oder Bromatom, eine Alkyl- oder Alkoxygruppe und

$R_f$ ein Fluor-, Chlor- oder Bromatom oder eine Alkoxygruppe bedeuten,

deren Isomerengemische, deren Tautomere, deren Enantiomere und deren Salze mit anorganischen oder organischen Säuren oder Basen,

wobei, soweit nichts anderes erwahnt wurde,

die vorstehend erwähnten Alkyl-, Alkylen- oder Alkoxyteile jeweils 1 bis 4 Kohlenstoffatome und die

Cycloalkylteile jeweils 3 bis 7 Kohlenstoffatome enthalten können, sowie

unter "eine Arylgruppe" eine gegebenenfalls durch ein Fluor-, Chlor- oder Bromatom, durch eine Hydroxy-, Alkyl-, Alkoxy-, Phenylalkoxy-, Phenyl-, Nitro-, Amino-, Alkylamino-, Dialkylamino-, Alkanoylamino-, Cyano-, Carboxy-, Alkoxycarbonyl-, Aminocarbonyl-, Alkylaminocarbonyl-, Dialkylaminocarbonyl-, Trifluormethyl-, Alkanoyl-, Aminosulfonyl-, Alkylaminosulfonyl- oder Dialkylaminosulfonylgruppe mono- oder disubstituierte Phenylgruppe, wobei der Alkylteil jeweils 1 bis 4 Kohlenstoffatome enthalten kann, oder eine Naphthylgruppe,

unter "eine Heteroarylgruppe" ein 5-gliedriger heteroaromatischer Ring, der eine Iminogruppe, ein Sauerstoff- oder Schwefelatom oder eine Iminogruppe und ein Sauerstoff-, Schwefel- oder Stickstoffatom oder eine Iminogruppe und 2 oder 3 Stickstoffatome enthält, und ein 6-gliedriger heteroaromatischer Ring, der 1, 2 oder 3 Stickstoffatome enthält, wobei die vorstehend erwähnten Ringe zusätzlich durch ein Fluor-, Chlor- oder Bromatom, durch eine Alkyl-, Alkoxy-, Hydroxy-, Phenyl-, Nitro-, Amino-, Alkylamino-, Dialkylami- no-, Alkanoylamino-, Cyano-, Carboxy-, Alkoxycarbonyl-, Aminocarbonyl-, Alkylaminocarbonyl-, Dialkylaminocarbonyl-, Trifluormethyl-, Alkanoyl-, Aminosulfonyl-, Alkylaminosulfonyl- oder Dialkylaminosulfonylgruppe mono- oder disubstituiert sein können, und

unter dem vorstehend erwähnten Begriff "eine Gruppe, die in vivo metabolisch in eine Carboxygruppe umgewandelt wird," beispielsweise deren Ester der Formeln

- CO - OR',
- CO - O - (HCR") - O - CO - R''' und
- CO - O - (HCR") - O - CO - OR'''

in denen
R' eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Pivaloyloxymethyl-, Phthalidylmethyl-, (1,3-Dioxa-2-oxo-4-methyl-cyclopenten-5-yl)-methyl-, Methoxymethyl- oder Cinnamylgruppe,

R" ein Wasserstoffatom oder eine Methylgruppe und

R''' eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe bedeuten, zu verstehen ist.

**2.** Phenylalkylderivate der allgemeinen Formel I gemäß Anspruch 1, in denen $R_a$ bis $R_f$, A, B und n mit der Maßgabe wie im Anspruch 1 vorstehend erwähnt definiert sind, daß $D_4$ bis $D_7$ mit den zusätzlichen Maßgaben (a) bis (j) Methingruppen darstellen oder

$D_7$ ein Stickstoffatom und die Reste $D_4$, $D_5$ und $D_6$ mit den zusätzlichen Maßgaben (a) bis (g) und (k) bis (m) oder (a) bis (g) und (n) bis (p) Methingruppen darstellen oder

einer der Reste $D_4$, $D_5$ oder $D_6$ ein Stickstoffatom und die verbleibenden Reste der Reste $D_4$ bis $D_6$ sowie $D_7$ Methingruppen darstellen oder

zwei der Reste $D_4$ bis $D_7$ Stickstoffatome und die verbleibenden Reste der Reste $D_4$ bis $D_7$ Methingruppen darstellen,

mit den zusätzlichen Maßgaben, daß entweder
(a) n die Zahl 1 darstellt oder
(b) E mit Ausnahme der Kohlenstoff-Kohlenstoff-Bindung die für E vorstehend erwähnten Bedeutungen besitzt oder
(c) A mit Ausnahme der Methylengruppe die für A eingangs erwähnten Bedeutungen besitzt oder
(d) B mit Ausnahme des Sauerstoffatoms die für B vorstehend erwähnten Bedeutungen besitzt oder
(e) $R_b$ mit Ausnahme der Carboxylgruppe die für $R_b$ vorstehend erwähnten Bedeutungen besitzt oder

(f) $R_c$ mit Ausnahme des Wasserstoffatoms die für $R_c$ vorstehend erwähnten Bedeutungen besitzt oder
(g) $R_d$ mit Ausnahme der Phenylgruppe die für $R_d$ vorstehend erwähnten Bedeutungen besitzt oder
(h) $R_1$ mit Ausnahme der n-Butylgruppe die für $R_1$ vorstehend erwähnten Bedeutungen besitzt oder
(i) $R_4$ mit Ausnahme der Methylgruppe in Position 7 die für $R_4$ vorstehend erwähnten Bedeutungen besitzt oder
(j) $R_5$ mit Ausnahme des Wasserstoffatoms die für $R_5$ vorstehend erwähnten Bedeutungen besitzt, oder, daß,
(k) $R_1$ mit Ausnahme der Ethylgruppe die für $R_1$ vorstehend erwähnten Bedeutungen besitzt oder
(l) $R_4$ mit Ausnahme der Methylgruppe in Position 7 die für $R_4$ vorstehend erwähnten Bedeutungen besitzt oder
(m) $R_5$ mit Ausnahme der Methylgruppe in Position 5 die für $R_5$ vorstehend erwähnten Bedeutungen besitzt oder
(n) $R_1$ mit Ausnahme der n-Propylgruppe für $R_1$ vorstehenderwähnten Bedeutungen besitzt oder
(o) $R_4$ mit Ausnahme des Wasserstoffatoms die für $R_4$ vorstehend erwähnten Bedeutungen besitzt oder
(p) $R_5$ mit Ausnahme des Wasserstoffatoms die für $R_5$ vorstehend erwähnten Bedeutungen besitzt und

die übrigen Reste die vorstehend erwähnten Bedeutungen besitzen,

wobei zusätzlich eine bei den vorstehend erwähnten Definitionen der Reste $D_4$ bis $D_7$ erwähnte Methingruppe durch den Rest $R_4$ und eine weitere bei den vorstehend erwähnten Definitionen der Reste $D_4$ bis $D_7$ erwähnte Methingruppe durch den Rest $R_5$ substituiert sein kann, bedeuten,

deren Isomerengemische, deren Tautomere, deren Enantiomere und deren Salze mit anorganischen oder organischen Säuren oder Basen.

**3.** Phenylalkylderivate der allgemeinen Formel I gemäß Anspruch 1 mit Ausnahme von
(i) 2-n-Butyl-1-[4-[(α-carboxy)benzyloxy]benzyl]benzimidazol,
(ii) 2-n-Propyl-7-methyl-3-[4-[(α-carboxy)benzyloxy]benzyl]imidazo[4,5-b]pyridin und
(iii) 5,7-Dimethyl-2-ethyl-3-[4-[(α-carboxy)benzyloxy]benzyl]imidazo[4,5-b]pyridin
diejenigen, in denen

n die Zahl 0 oder 1,

A eine geradkettige oder verzweigte Alkylengruppe,

B ein Sauerstoffatom, eine geradkettige oder verzweigte Alkylengruppe mit 1 bis 3 Kohlenstoffatomen oder eine gegebenenfalls durch eine Alkylgruppe oder durch eine Alkanoylgruppe mit 1 bis 4 Kohlenstoffatomen substituierte Iminogruppe,

$R_a$ eine Gruppe der Formel

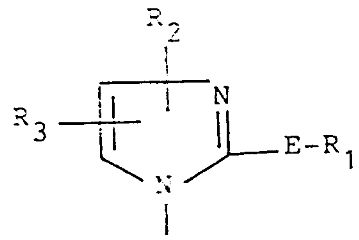

,

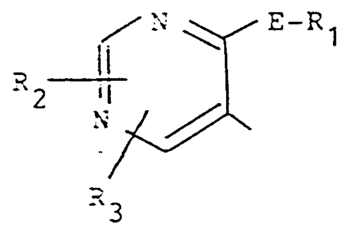

,

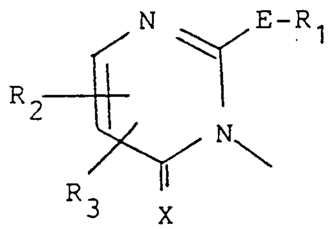

,

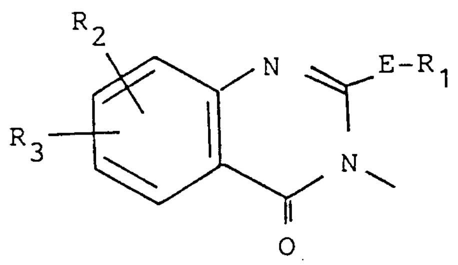

,

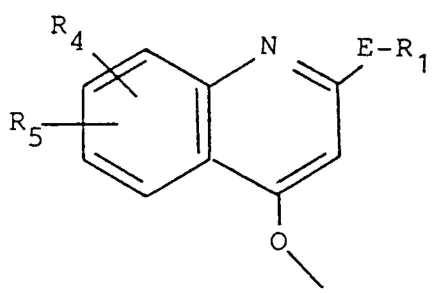

oder

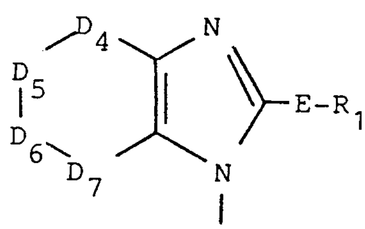

,

in denen null, einer oder zwei der Reste $D_4$, $D_5$, $D_6$ oder $D_7$ ein Stickstoffatom und die verbleibenden Reste $D_4$, $D_5$, $D_6$ oder $D_7$ jeweils Methingruppen, wobei zusätzlich eine Methingruppe durch den Rest $R_4$ und eine weitere Methingruppe durch den Rest $R_5$ substituiert sein kann,

E eine Kohlenstoff-Kohlenstoff-Bindung, ein Sauerstoff- oder Schwefelatom oder eine gegebenenfalls durch eine Alkylgruppe mit 1 bis 4 Kohlenstoffatomen substituierte Iminogruppe,

X ein Sauerstoff- oder Schwefelatom,

$R_1$ eine geradkettige oder verzweigte Alkylgruppe mit 1 bis 6 Kohlenstoffatomen, eine geradkettige oder verzweigte Alkenyl- oder Alkinylgruppe mit jeweils 2 bis 6 Kohlenstoffatomen, wobei die vorstehend erwähnten gesättigten und ungesattigten Alkylteile jeweils durch ein Fluor-, Chlor- oder Bromatom, durch eine Hydroxy- oder Aminogruppe substituiert sein können, oder eine Cycloalkylgruppe mit 3 bis 6 Kohlenstoffatomen,

$R_2$ ein Wasserstoff-, Fluor-, Chlor- oder Bromatom, eine Alkyl- oder Perfluoralkylgruppe mit jeweils 1 bis 5 Kohlenstoffatomen, eine Cyano- oder Nitrogruppe,

$R_3$ ein Wasserstoffatom, eine Cyanogruppe, eine gegebenfalls durch eine Hydroxy- oder Alkoxygruppe substituierte Alkylgruppe mit 1 bis 3 Kohlenstoffatomen, eine Perfluoralkylgruppe mit 1 bis 3 Kohlenstoffatomen, eine gegebenenfalls durch Fluoratome substituierte Alkenylgruppe mit 3 bis 6 Kohlenstoffatomen, eine Alkylgruppe mit 1 bis 3 Kohlenstoffatomen, die endständig durch eine $R_6COO$, $R_7S$-, $R_7SO$-, $R_7SO_2$-, $R_7CO$-, $R_7NHCOO$-, $R_7NHCO$-, $R_7NHCONR_7$-, $R_8CONR_7$- oder $R_8SO_2NR_7$-Gruppe substituiert ist, wobei

$R_6$ eine Alkyl- oder Perfluoralkylgruppe mit jeweils 1 bis 4 Kohlenstoffatomen, eine Cycloalkyl-, Phenyl-, Benzyl- oder Phenylethylgruppe,

$R_7$ ein Wasserstoffatom und die für $R_6$ vorstehend erwähnten Bedeutungen besitzt,

$R_8$ die für $R_7$ vorstehend erwähnten Bedeutungen besitzt, darstellen,

$R_4$ ein Wasserstoff-, Fluor-, Chlor- oder Bromatom, eine Trifluormethylgruppe, eine Cycloalkylgruppe, eine gegebenenfalls durch eine Hydroxy-, Alkoxy- oder Alkoxycarbonylgruppe substituierte Alkylgruppe mit 1 bis 6 Kohlenstoffatomen und $R_5$ ein Wasserstoff-, Fluor-, Chlor- oder Bromatom,

eine geradkettige oder verzweigte Alkyl- oder Perfluoralkylgruppe mit jeweils 1 bis 6 Kohlenstoffatomen, eine Alkenyl- oder Alkinylgruppe mit jeweils 2 bis 6 Kohlenstoffatomen, wobei die vorstehend erwähnten Alkyl- und Alkenylteile jeweils durch eine Heteroaryl-, Hydroxy-, Alkoxy-, Amino-, Alkylamino-, Dialkylamino-, Alkylcarbonylamino-, N-Alkyl-alkylcarbonylamino-, Carboxy-, Alkoxycarbonyl-, Alkylcarbonyloxy-, Aminocarbonyl-, Alkylaminocarbonyl-, Dialkylaminocarbonyl- oder Tetrazol-5-yl-gruppe mono- oder disubstituiert sein können,

eine Alkoxygruppe mit 1 bis 5 Kohlenstoffatomen oder eine $\alpha$-(1H-Tetrazol-5-yl)-benzyloxygruppe,

eine Carboxygruppe oder eine Gruppe, die in vivo metabolisch in eine Carboxygruppe umgewandelt wird,

eine Alkylsulfonyloxygruppe mit 1 bis 4 Kohlenstoffatomen, eine Benzolsulfonyloxy- oder Phenylalkansulfonyloxygruppe,

eine gegebenenfalls am Stickstoffatom durch eine Alkylgruppe mit 1 bis 6 Kohlenstoffatomen, durch eine Phenyl-, Cycloalkyl-, Phenylalkyl- oder Cycloalkylalkylgruppe substituierte Acylaminogruppe, in welcher der Acylrest eine Alkanoylgruppe mit 1 bis 5 Kohlenstoffatomen, eine Alkoxycarbonylgruppe mit insgesamt 2 bis 4 Kohlenstoffatomen, eine Alkylsulfonylgruppe mit 1 bis 6 Kohlenstoffatomen, eine Benzoyl-, Benzolsulfonyl-, Cycloalkylcarbonyl-, Phenylalkanoyl- oder Cycloalkylalkanoylgruppe darstellt, wobei die vorstehend erwähnten Phenylkerne jeweils durch ein Fluor-, Chlor- oder Bromatom, durch eine Methyl- oder Methoxygruppe mono- oder disubstituiert und die Substituenten gleich oder verschieden sein können,

eine Phthalimino- oder Homophthaliminogruppe, wobei eine Carbonylgruppe in einer Phthaliminogruppe durch eine Methylen-, Alkyl-methylen- oder Dialkyl-methylengruppe ersetzt sowie eine Methylengruppe in einer Homophthaliminogruppe durch eine oder zwei Alkylgruppen substituiert sein kann, und zusätzlich die vorstehend erwähnten Phenylkerne durch Alkyl- oder Alkoxygruppen mono- oder disubstituiert und gleichzeitig ganz oder teilweise hydriert sein können, wobei die Substituenten gleich oder verschieden sein können,

eine gegebenenfalls durch eine oder zwei Alkylgruppen oder durch eine Tetramethylen- oder Pentamethylengruppe substituierte 5-, 6- oder 7-gliedrige Alkyleniminogruppe, in welcher eine Methylengruppe durch eine Carbonyl- oder Sulfonylgruppe ersetzt sein kann,

eine gegebenenfalls durch eine Alkyl- oder Phenylgruppe mono- oder disubstituierte Maleinsäureimido- oder Succinimidogruppe, wobei die Substituenten gleich oder verschieden sein können,

einen über ein Kohlenstoffatom oder über eine Iminogruppe gebundenen 5-gliedrigen heteroaromatischen Ring, der eine Iminogruppe, ein Sauerstoff- oder Schwefelatom oder eine Iminogruppe und ein Sauerstoff-, Schwefel- oder Stickstoffatom enthält, oder einen über ein Kohlenstoffatom gebundenen 6-gliedrigen heteroaromatischen Ring, der 1 oder 2 Stickstoffatome enthält, wobei sowohl an die 5-gliedrigen als auch an die 6-gliedrigen heteroaromatischen Ringe jeweils über zwei benachbarte Kohlenstoffatome eine n-Propylen-, n-Butylen- oder 1,3-Butadienylgruppe oder über eine Iminogruppe und ein benachbartes Kohlenstoffatom eine n-Propylen-, n-Butylen- oder 1,3-Butadienylgruppe angefügt ist und in einem so gebildeten anellierten Pyridinring eine Methingruppe durch ein Stickstoffatom und eine Vinylengruppe in 3-, 4-Stellung zu dem Stickstoffatom des gebildeten Pyridinringes durch ein Schwefelatom oder in einem so gebildeten anellierten Phenylring eine oder zwei Methingruppen durch N-Atome ersetzt sein können, wobei zusätzlich die vorstehend erwähnten ankondensierten aromatischen oder heteroaromatischen Ringe im Kohlenstoffgerüst durch ein Fluor-, Chlor- oder Bromatom, durch eine Alkyl-, Alkoxy-, Hydroxy-, Phenyl-, Nitro-, Amino-, Alkylamino-, Dialkylamino-, Alkanoylamino-, Cyano-, Carboxy-, Alkoxycarbonyl-, Aminocarbonyl-, Alkylaminocarbonyl-, Dialkylaminocarbonyl-, Trifluormethyl-, Alkanoyl-, Aminosulfonyl-, Alkylaminosulfonyl- oder Dialkylaminosulfonylgruppe monosubstituiert oder durch Fluor- oder Chloratome, durch Methyl-, Methoxy- oder Hydroxygruppen disubstituiert sein können und eine gegebenenfalls in einem Imidazolring vorhandene NH-Gruppe durch eine Alkylgruppe mit 1 bis 6 Kohlenstoffatomen substituiert sein kann, oder

eine $R_{11}$-$NR_{10}$-CO-$NR_9$-Gruppe, in der

$R_9$ ein Wasserstoffatom, eine Alkylgruppe mit 1 bis 6 Kohlenstoffatomen oder Phenylalkylgruppe,

$R_{10}$ ein Wasserstoffatom, eine Alkylgruppe mit 1 bis 6 Kohlenstoffatomen, eine Phenylalkylgruppe oder eine Cycloalkylgruppe mit 5 bis 7 Kohlenstoffatomen,

$R_{11}$ ein Wasserstoffatom oder eine Alkylgruppe mit 1 bis 6 Kohlenstoffatomen oder

$R_{10}$ und $R_{11}$ zusammen mit dem dazwischen liegenden Stickstoffatom eine geradkettige Alkyleniminogruppe mit 4 bis 6 Kohlenstoffatomen oder eine Morpholinogruppe oder

$R_9$ und $R_{11}$ zusammen eine Alkylengruppe mit 2 bis 4 Kohlenstoffatomen darstellen,

$R_b$ eine Cyano-, Carboxy-, Arylsulfonylaminocarbonyl-, Benzylsulfonylaminocarbonyl-, Sulfo-, Alkylcarbonylaminosulfonyl-, Aralkylcarbonylaminosulfonyl-, Arylcarbonylaminosulfonyl-, Alkylcarbonylaminosulfonylmethyl-, Aralkylcarbonylaminosulfonylmethyl-, Arylcarbonylaminosulfonylmethyl-, Phosphino-, O-Alkyl-phosphino-, Phosphono-, O-Alkyl-phosphono-, O,O-Dialkylphosphono-, Phosphono-methyl-, O-Alkyl-phosphonomethyl-, O,O-Dialkylphosphono-methyl-, Phosphato- oder O-Alkyl-phosphatogruppe, eine gegebenenfalls durch eine Phenylalkyl- oder Triphenylmethylgruppe substituierte 1H-Tetrazolyl- oder 1H-Tetrazolylalkylgruppe, eine Alkylsulfonylaminocarbonyl- oder Perfluoralkylsulfonylaminocarbonylgruppe mit jeweils 1 bis 6 Kohlenstoffatomen im Alkylteil, eine Gruppe, die in vivo metabolisch in eine Carboxygruppe umgewandelt wird, oder eine Aralkoxycarbonylgruppe,

$R_c$ ein Wasserstoffatom, eine Methyl-, Phenyl-, Benzyl-, Carboxy- oder Alkoxycarbonylgruppe und

$R_d$ eine geradkettige oder verzweigte Alkylkette mit 1 bis 6 Kohlenstoffatomen, eine geradkettige oder verzweigte Alkenyl- oder Alkinylgruppe mit jeweils 2 bis 6 Kohlenstoffatomen, eine Cycloalkyl- oder Cycloalkylalkylgruppe, eine gegebenenfalls durch Fluor-, Chlor- oder Bromatome, durch Methyl- oder Methoxygruppen mono- oder disubstituierte Phenylgruppe, eine Biphenyl-, Naphthyl- oder Heteroarylgruppe,

$R_e$ und $R_f$ Wasserstoffatome und, wenn

$R_5$ eine Phthalimino- oder Homophthaliminogruppe, wobei eine Carbonylgruppe in einer Phthaliminogruppe durch eine Methylen-, Alkyl-methylen- oder Dialkyl-methylengruppe ersetzt sowie eine Methylengruppe in einer Homophthaliminogruppe durch eine oder zwei Alkylgruppen substituiert sein kann, und zusätzlich die vorstehend erwähnten Phenylkerne durch Alkyl- oder Alkoxygruppen mono- oder disubstituiert und gleichzeitig ganz oder teilweise hydriert sein können, wobei die Substituenten gleich

oder verschieden sein können,

eine Carboxygruppe oder eine Gruppe, die in vivo metabolisch in eine Carboxygruppe umgewandelt wird,

eine gegebenenfalls durch eine oder zwei Alkylgruppen oder durch eine Tetramethylen- oder Pentamethylengruppe substituierte 5-, 6- oder 7-gliedrige Alkyleniminogruppe, in welcher eine Methylengruppe durch eine Carbonyl- oder Sulfonylgruppe ersetzt sein kann,

eine gegebenenfalls durch eine Alkyl- oder Phenylgruppe mono- oder disubstituierte Maleinsäureimido- oder Succinimidogruppe, wobei die Substituenten gleich oder verschieden sein können,

einen über ein Kohlenstoffatom oder über eine Iminogruppe gebundenen 5-gliedrigen heteroaromatischen Ring, der eine Iminogruppe, ein Sauerstoff- oder Schwefelatom oder eine Iminogruppe und ein Sauerstoff-, Schwefel- oder Stickstoffatom enthält, oder einen über ein Kohlenstoffatom gebundenen 6-gliedrigen heteroaromatischen Ring, der 1 oder 2 Stickstoffatome enthält, wobei sowohl an die 5-gliedrigen als auch an die 6-gliedrigen heteroaromatischen Ringe jeweils über zwei benachbarte Kohlenstoffatome eine n-Propylen-, n-Butylen- oder 1,3-Butadienylgruppe oder über eine Iminogruppe und ein benachbartes Kohlenstoffatom eine n-Propylen-, n-Butylen- oder 1,3-Butadienylgruppe angefügt ist und in einem so gebildeten anellierten Pyridinring eine Methingruppe durch ein Stickstoffatom und eine Vinylengruppe in 3-, 4-Stellung zu dem Stickstoffatom des gebildeten Pyridinringes durch ein Schwefelatom oder in einem so gebildeten anellierten Phenylring eine oder zwei Methingruppen durch N-Atome ersetzt sein können, wobei zusätzlich die vorstehend erwähnten ankondensierten aromatischen oder heteroaromatischen Ringe im Kohlenstoffgerüst durch ein Fluor-, Chlor- oder Bromatom, durch eine Alkyl-, Alkoxy-, Hydroxy-, Phenyl-, Nitro-, Amino-, Alkylamino-, Dialkylamino-, Alkanoylamino-, Cyano-, Carboxy-, Alkoxycarbonyl-, Aminocarbonyl-, Alkylaminocarbonyl-, Dialkylaminocarbonyl-, Trifluormethyl-, Alkanoyl-, Aminosulfonyl-, Alkylaminosulfonyl- oder Dialkylaminosulfonylgruppe monosubstituiert oder durch Fluor- oder Chloratome, durch Methyl-, Methoxy- oder Hydroxygruppen disubstituiert sein können und eine gegebenenfalls in einem Imidazolring vorhandene NH-Gruppe durch eine Alkylgruppe mit 1 bis 6 Kohlenstoffatomen substituiert sein kann, oder

eine $R_{11}$-$NR_{10}$-CO-$NR_9$-Gruppe, in der $R_9$ bis $R_{11}$ wie vorstehend erwähnt definiert sind,

auch $R_e$ ein Wasserstoff-, Fluor-, Chlor- oder Bromatom, eine Alkyl- oder Alkoxygruppe und

$R_f$ ein Fluor-, Chlor- oder Bromatom oder eine Alkoxygruppe bedeuten,

deren Isomerengemische, deren Tautomere, deren Enantiomere und deren Salze mit anorganischen oder organischen Säuren oder Basen,

wobei, soweit nichts anderes erwähnt wurde,

der vorstehend erwähnte Begriff "eine Gruppe, die in vivo metabolisch in eine Carboxygruppe umgewandelt wird," wie im Anspruch 1 definiert ist,

die vorstehend erwähnten Alkyl-, Alkylen- oder Alkoxyteile jeweils 1 bis 4 Kohlenstoffatome und die Cycloalkylteile jeweils 3 bis 7 Kohlenstoffatome enthalten können, sowie

unter "eine Arylgruppe" eine gegebenenfalls durch ein Fluor-, Chlor- oder Bromatom, durch eine Hydroxy-, Alkyl-, Alkoxy-, Phenylalkoxy-, Phenyl- oder Nitrogruppe mono- oder disubstituierte Phenylgruppe, wobei der Alkylteil jeweils 1 bis 4 Kohlenstoffatome enthalten kann, oder eine Naphthylgruppe und

unter "eine Heteroarylgruppe" ein 5-gliedriger heteroaromatischer Ring, der eine Iminogruppe, ein Sauerstoff- oder Schwefelatom oder eine Iminogruppe und ein Sauerstoff-, Schwefel- oder Stickstoffatom oder eine Iminogruppe und 2 oder 3 Stickstoffatome enthält, und ein 6-gliedriger heteroaromatischer Ring, der 1, 2 oder 3 Stickstoffatome enthält, wobei die vorstehend erwähnten Ringe zusätzlich durch ein Fluor-, Chlor- oder Bromatom, durch eine Alkyl-, Alkoxy-, Hydroxy-, Phenyl- oder Nitrogruppe

mono- oder disubstituiert sein können, zu verstehen ist.

**4.** Phenylalkylderivate der allgemeinen Formel I gemäß Anspruch 1 mit Ausnahme von
(i) 2-n-Butyl-1-[4-[(α-carboxy)benzyloxy]benzyl]benzimidazol,
(ii) 2-n-Propyl-7-methyl-3-[4-[(α-carboxy)benzyloxy]benzyl]imidazo[4,5-b]pyridin und
(iii) 5,7-Dimethyl-2-ethyl-3-[4-[(α-carboxy)benzyloxy]benzyl]imidazo[4,5-b]pyridin
diejenigen, in denen

n die Zahl 0,

A eine Methylen-, Ethylen- oder Ethylidengruppe,

B ein Sauerstoffatom, eine Methylen-, Imino-, Methylimino- oder Acetyliminogruppe,

$R_a$ eine Gruppe der Formel

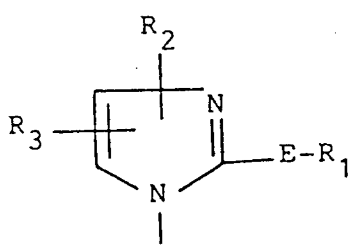

,

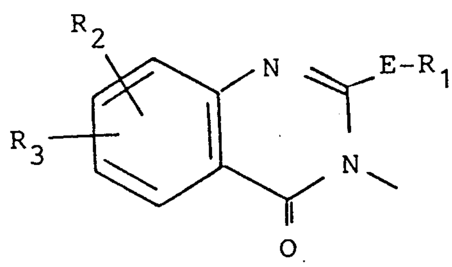

,

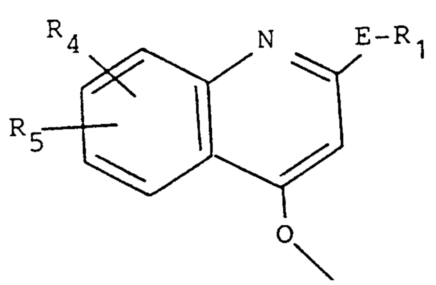

oder

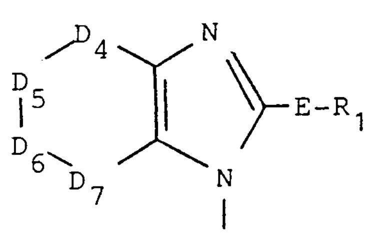

,

in denen null, einer oder zwei der Reste $D_4$, $D_5$, $D_6$ oder $D_7$ ein Stickstoffatom und die verbleibenden Reste $D_4$, $D_5$, $D_6$ oder $D_7$ jeweils Methingruppen, wobei zusätzlich eine Methingruppe durch den Rest $R_4$ und eine weitere Methingruppe durch den Rest $R_5$ substituiert sein kann,

E ein Sauerstoffatom oder eine Kohlenstoff-Kohlenstoff-Bindung,

$R_1$ eine geradkettige oder verzweigte Alkylgruppe mit 1 bis 6 Kohlenstoffatomen,

$R_2$ ein Wasserstoff-, Fluor-, Chlor- oder Bromatom,

$R_3$ eine Hydroxyalkylgruppe mit 1 oder 2 Kohlenstoffatomen,

$R_4$ ein Wasserstoffatom oder eine Alkylgruppe mit 1 bis 3 Kohlenstoffatomen und

$R_5$ ein Wasserstoff-, Fluor-, Chlor- oder Bromatom,

eine geradkettige oder verzweigte Alkylgruppe mit 1 bis 3 Kohlenstoffatomen oder eine $\alpha$-(1H-Tetrazol-5-yl)-benzyloxygruppe,

eine Amino- oder Nitrogruppe,

eine Carboxygruppe oder eine Alkoxycarbonylgruppe mit insgesamt 2 bis 4 Kohlenstoffatomen,

eine gegebenenfalls am Stickstoffatom durch eine Alkylgruppe mit 1 bis 5 Kohlenstoffatomen substituierte Acylaminogruppe, in welcher der Acylrest eine Alkanoylgruppe mit 1 bis 5 Kohlenstoffatomen oder eine Benzolsulfonylgruppe darstellt, wobei der vorstehend erwähnte Phenylkern durch eine Methyl- oder Methoxygruppe mono- oder disubstituiert und die Substituenten gleich oder verschieden sein können,

eine gegebenenfalls durch eine oder zwei Methylgruppen substituierte 5-, 6- oder 7-gliedrige Alkyleniminogruppe, in welcher eine Methylengruppe durch eine Carbonyl- oder Sulfonylgruppe ersetzt sein kann, oder eine 2,3-Dimethylsuccinimido-, Phthalimino-, Homophthalimino- oder Isoindolin-1-on-yl-gruppe,

eine gegebenenfalls in 1-Stellung durch eine Alkylgruppe mit 1 bis 3 Kohlenstoffatomen substituierte Benzimidazol-2-yl-gruppe oder

eine $R_{11}$-$NR_{10}$-CO-$NR_9$-Gruppe, in der

$R_9$ ein Wasserstoffatom, eine Alkylgruppe mit 1 bis 5 Kohlenstoffatomen oder eine Phenylalkylgruppe mit 1 bis 3 Kohlenstoffatomen im Alkylteil,

$R_{10}$ ein Wasserstoffatom, eine Alkylgruppe mit 1 bis 5 Kohlenstoffatomen oder eine Cyclohexylgruppe,

$R_{11}$ ein Wasserstoffatom, eine Benzylgruppe oder eine Alkylgruppe mit 1 bis 5 Kohlenstoffatomen oder

$R_9$ und $R_{11}$ zusammen eine Alkylengruppe mit 2 oder 3 Kohlenstoffatomen darstellen,

$R_b$ eine Carboxy-, 1H-Tetrazolyl- oder 0-Alkyl-phosphono- oder Alkylsulfonylaminocarbonylgruppe mit jeweils 1 bis 3 Kohlenstoffatomen im Alkylteil,

$R_c$ ein Wasserstoffatom oder eine Phenylgruppe,

$R_d$ eine geradkettige oder verzweigte Alkylkette mit 1 bis 4 Kohlenstoffatomen, eine Cyclohexyl-, Cyclohexylmethyl-, Phenyl-, Biphenyl-, Methoxyphenyl-, Chlorphenyl-, Pyridyl- oder Naphthylgruppe,

$R_e$ und $R_f$ Wasserstoffatome und, wenn

$R_5$ eine gegebenenfalls in 1-Stellung durch eine Alkylgruppe mit 1 bis 3 Kohlenstoffatomen substituierte Benzimidazol-2-yl-gruppe,

eine 2,3-Dimethylsucciniminogruppe,

eine gegebenenfalls durch eine oder zwei Methylgruppen substituierte 5-, 6- oder 7-gliedrige Alkyleniminogruppe, in welcher eine Methylengruppe durch eine Carbonyl- oder Sulfonylgruppe ersetzt sein kann, oder

eine $R_{11}$-$NR_{10}$-CO-$NR_9$-Gruppe, in der $R_9$ bis $R_{11}$ wie vorstehend erwähnt definiert sind,

auch $R_e$ ein Wasserstoff-, Chlor- oder Bromatom oder eine Methoxygruppe und

$R_f$ ein Chlor- oder Bromatom oder eine Methoxygruppe bedeuten,

deren Isomerengemische, deren Tautomere, deren Enantiomere und deren Salze.

**5.** Folgende Verbindungen der allgemeinen Formel I gemäß Anspruch 1:
(a) 2-n-Propyl-4-methyl-1-[4-[(α-carboxy)benzyloxy]benzyl]benzimidazol,
(b) 2-n-Butyl-1-[4-[(α-carboxy)benzyloxy]benzyl]-6-dimethylaminocarbonylamino-benzimidazol,
(c) 2-n-Propyl-6-(1-methyl-benzimidazol-2-yl)-4-methyl-1-[4-[(α-carboxy)benzyloxy]benzyl]-benzimidazol,
(d) 2-Methyl-4-[4'-[(α-carboxy)benzyloxy]benzyloxy]chinolin,
(e) 2-n-Butyl-8-methyl-3-[4-[(α-carboxy)benzyloxy]benzyl]chinazolin-4-on-semihydrat,
(f) 2-n-Propyl-4-methyl-6-(1-methyl-benzimidazol-2-yl)-1-[4-[α-(1H-tetrazol-5-yl)benzyloxy]benzyl]-benzimidazol,
(g) 2-n-Butyl-6-(N-propionyl-methylamino)-1-[4-[(1-carboxy-3-methyl)butyloxy]benzyl]benzimidazol,
(h) 2-n-Butyl-5-methyl-6-(3-benzyl-3,4,5,6-tetrahydro-2(1H)-pyrimidinon-1-yl)-3-[4-[α-(1H-tetrazol-5-yl)benzyloxy]benzyl]imidazol[4,5-b]pyridin und
(i) 2-n-Butyl-1-[4-[(α-(α-ethyl-phosphono)benzylamino]benzyl]benzimidazol,
deren Salze mit anorganischen oder organischen Säuren oder Basen.

**6.** Physiologisch verträgliche Salze der Verbindungen nach mindestens einem der Ansprüche 1 bis 5 mit anorganischen oder organischen Säuren oder Basen.

**7.** Arzneimittel, enthaltend eine Verbindung nach mindestens einem der Ansprüche 1 bis 5 oder ein physiologisch verträgliches Salz gemäß Anspruch 6 neben gegebenenfalls einem oder mehreren inerten Trägerstoffen und/oder Verdünnungsmitteln.

**8.** Verwendung einer Verbindung nach mindestens einem der Ansprüche 1 bis 6 zur Herstellung eines Arzneimittels mit Angiotensin-antagonistischer Wirkung.

**9.** Verfahren zur Herstellung eines Arzneimittels gemäß Anspruch 8, dadurch gekennzeichnet, daß auf nichtchemischem Wege eine Verbindung nach mindestens einem der Ansprüche 1 bis 6 in einen oder mehrere inerte Trägerstoffe und/oder Verdünnungsmittel eingearbeitet wird.

**10.** Verfahren zur Herstellung der Phenylalkylderivate gemäß den Ansprüchen 1 bis 6, dadurch gekennzeichnet, daß
a) eine Verbindung der allgemeinen Formel

$R_a$ - H ,(II)

in der
$R_a$ wie in den Ansprüchen 1 bis 5 definiert ist, mit einer Verbindung der allgemeinen Formel

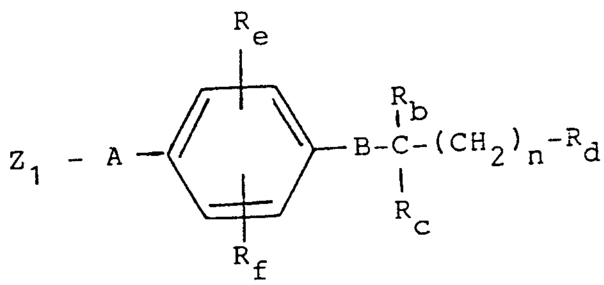

,(III)

in der

A, B, n und $R_c$ bis $R_f$ wie in den Ansprüchen 1 bis 5 definiert sind und

$Z_1$ eine nukleophile Austrittsgruppe darstellt, umgesetzt und erforderlichenfalls eine so erhaltene Verbindung hydrolysiert wird oder

b) eine Verbindung der allgemeinen Formel

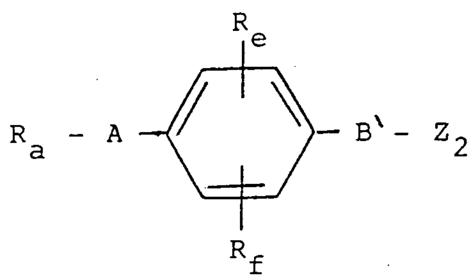

,(IV)

in der

$R_a$, $R_e$, $R_f$ und A wie in den Ansprüchen 1 bis 5 definiert sind,

B' eine geradkettige oder verzweigte Alkylengruppe mit 1 bis 4 Kohlenstoffatomen und

$Z_2$ eine nukleophile Austrittsgruppe bedeuten, mit einer Verbindung der allgemeinen Formel

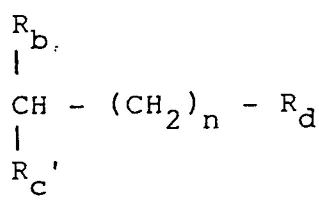

,(V)

$R_b$, $R_d$ und n wie in den Ansprüchen 1 bis 5 definiert sind und

$R_c$' eine Alkoxycarbonylgruppe darstellt, umgesetzt und erforderlichenfalls eine so erhaltene Verbindung hydrolysiert oder hydrolysiert und decarboxyliert wird oder

c) zur Herstellung von Verbindungen der allgemeinen Formel I, in der B ein Sauerstoff- oder Schwefelatom oder eine gegebenenfalls durch eine Alkylgruppe substituierte Iminogruppe darstellt, eine Verbindung der allgemeinen Formel

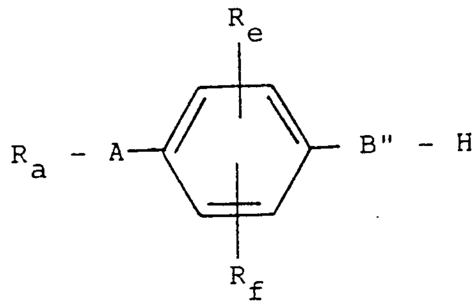

,(VI)

in der

$R_a$, $R_e$, $R_f$ und A wie in den Ansprüchen 1 bis 5 definiert sind und

B" ein Sauerstoff- oder Schwefelatom oder eine gegebenenfalls durch eine Alkylgruppe substituierte Iminogruppe darstellt, mit einer Verbindung der allgemeinen Formel

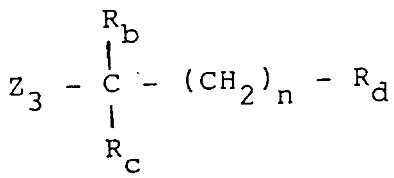

,(VII)

in der

$R_b$ bis $R_d$ und n wie in den Ansprüchen 1 bis 5 definiert sind und

$Z_3$ eine nukleophile Austrittsgruppe bedeutet, umgesetzt und erforderlichenfalls eine so erhaltene Verbindung hydrolysiert wird oder

d) zur Herstellung einer Verbindung der allgemeinen Formel I, in der $R_b$ eine Carboxygruppe darstellt, eine Verbindung der allgemeinen Formel

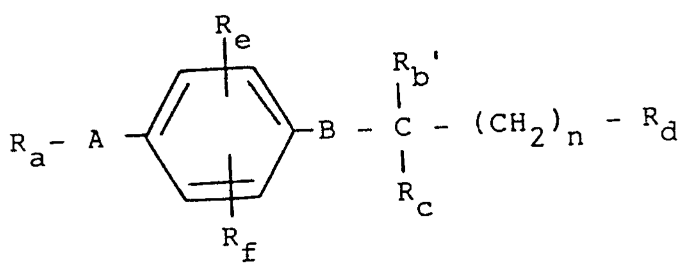

,(VIII)

in der

$R_a$, $R_c$ bis $R_f$, A, B und n wie in den Ansprüchen 1 bis 5 definiert sind und

$R_b$' eine mittels Hydrolyse, Thermolyse oder Hydrogenolyse in eine Carboxygruppe überführbare Gruppe darstellt, in eine entsprechende Carboxyverbindung übergeführt wird oder

e) zur Herstellung einer Verbindung der allgemeinen Formel I, in der $R_b$ eine 1H-Tetrazolylgruppe darstellt, ein Schutzrest von einer Verbindung der allgemeinen Formel

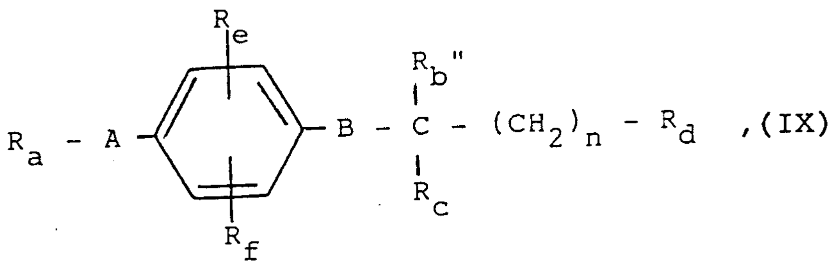

in der

$R_a$, $R_c$ bis $R_f$, A, B und n wie in den Ansprüchen 1 bis 5 definiert sind und

$R_b$" eine in 1- oder 2-Stellung durch einen Schutzrest geschützte 1H-Tetrazolylgruppe darstellt, abgespalten wird oder

f) zur Herstellung einer Verbindung der allgemeinen Formel I, in der $R_b$ eine 1H-Tetrazolylgruppe darstellt, eine Verbindung der allgemeinen Formel

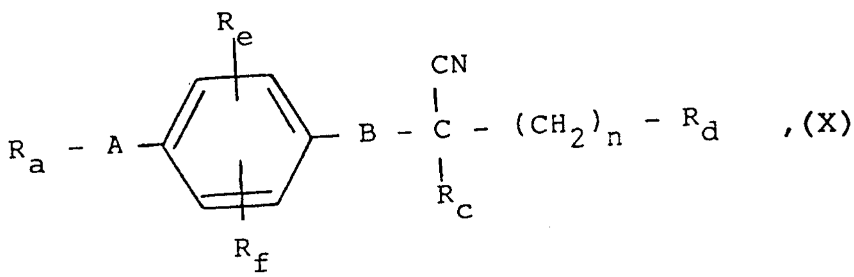

in der

$R_a$, $R_c$ bis $R_f$, A, B und n wie in den Ansprüchen 1 bis 5 definiert sind, mit Stickstoffwasserstoffsäure oder deren Salzen umgesetzt wird oder

g) zur Herstellung von Verbindungen der allgemeinen Formel I, in der $R_a$ eine Gruppe der Formel

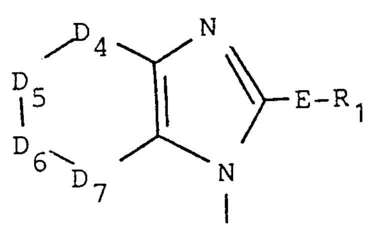

darstellt, eine Verbindung der allgemeinen Formel

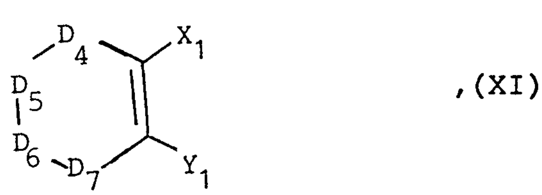

in der

$D_1$ bis $D_7$ wie in den Ansprüchen 1 bis 5 definiert sind,

einer der Reste $X_1$ oder $Y_1$ eine Gruppe der allgemeinen Formel

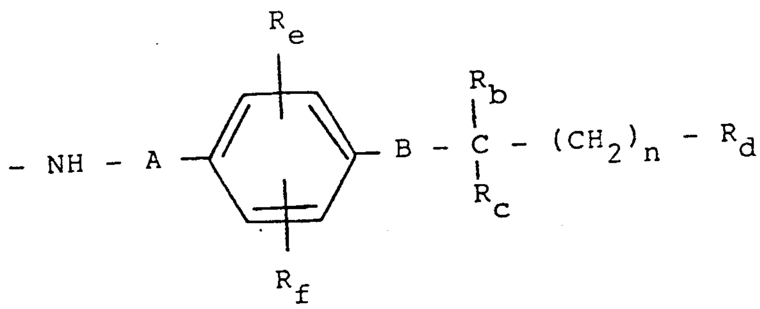

und der andere der Reste $X_1$ oder $Y_1$ eine Gruppe der allgemeinen Formel

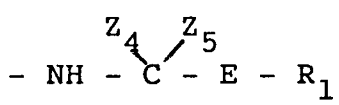

darstellen, wobei $R_1$, A, B, E, n und $R_b$ bis $R_f$ wie in den Ansprüchen 1 bis 5 definiert sind,
$Z_4$ und $Z_5$, die gleich oder verschieden sein können, gegebenenfalls substituierte Aminogruppen oder gegebenenfalls durch niedere Alkylgruppen substituierte Hydroxy- oder Mercaptogruppen oder
$Z_4$ und $Z_5$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert wird oder
h) zur Herstellung von Verbindungen der allgemeinen Formel I, in der $R_5$ eine $R_{11}$-$NR_{10}$-CO-$NR_9$-Gruppe darstellt, eine Verbindung der allgemeinen Formel

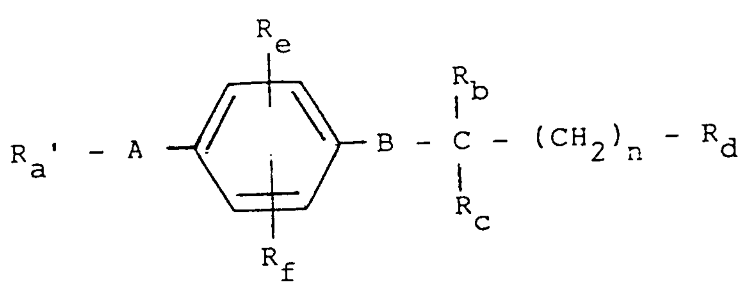
,(XII)

in der
$R_b$ bis $R_f$, A, B und n wie in den Ansprüchen 1 bis 5 definiert sind und
$R_a$' einen der für $R_a$ in den Ansprüchen 1 bis 5 erwähnten Reste darstellt, in dem $R_5$ eine Aminogruppe, eine Alkylaminogruppe mit 1 bis 8 Kohlenstoffatomen oder eine Phenylalkylaminogruppe mit 1 bis 4 Kohlenstoffatomen im Alkylteil darstellt, mit einer Verbindung der Formel

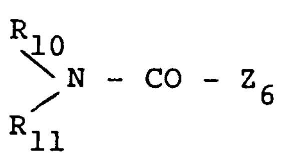
,(XIII)

in der
$R_{10}$ ein Wasserstoffatom, eine Alkylgruppe mit 1 bis 8 Kohlenstoffatomen, eine Alkenylgruppe mit 3 bis 5 Kohlenstoffatomen, eine Phenylalkylgruppe oder eine Cycloalkylgruppe mit 5 bis 7 Kohlenstoffatomen,
$R_{11}$ ein Wasserstoffatom oder eine Alkylgruppe mit 1 bis 6 Kohlenstoffatomen oder

einer der Reste $R_{10}$ oder $R_{11}$ auch eine Bicyclohexyl- oder Biphenylylgruppe oder
$R_{10}$ und $R_{11}$ zusammen mit dem dazwischen liegenden Stickstoffatom eine geradkettige Alkyleniminogruppe mit 4 bis 6 Kohlenstoffatomen oder eine Morpholinogruppe und
$Z_6$ eine nukleophile Austrittsgruppe oder auch, wenn einer der Reste $R_{10}$ oder $R_{11}$ ein Wasserstoffatom darstellt, $Z_6$ zusammen mit $R_{10}$ oder $R_{11}$ eine Stickstoff-Kohlenstoff-Bindung darstellen, umgesetzt wird oder
i) zur Herstellung von Verbindungen der allgemeinen Formel I, in der $R_5$ eine gegebenenfalls am Stickstoffatom durch eine Alkylgruppe mit 1 bis 6 Kohlenstoffatomen, durch eine Phenyl-, Cycloalkyl-, Phenylalkyl-, Cycloalkylalkyl-, Bicyclohexyl- oder Biphenylgruppe substituierte Acylaminogruppe, in welcher der Acylrest eine Alkanoylgruppe mit 1 bis 7 Kohlenstoffatomen, eine Alkoxycarbonylgruppe mit insgesamt 2 bis 4 Kohlenstoffatomen, eine Alkylsulfonylgruppe mit 1 bis 6 Kohlenstoffatomen, eine Benzoyl-, Benzolsulfonyl-, Phenylalkansulfonyl-, Naphthalinsulfonyl-, Cycloalkylcarbonyl-, Phenylalkanoyl- oder Cycloalkylalkanoylgruppe darstellt, wobei die vorstehend erwähnten Phenylkerne jeweils durch ein Fluor-, Chlor- oder Bromatom, durch eine Methyl- oder Methoxygruppe mono- oder disubstituiert und die Substituenten gleich oder verschieden sein können, eine Phthalimino- oder Homophthaliminogruppe, in denen der Phenylkern jeweils durch Alkyloder Alkoxygruppen mit jeweils 1 bis 3 Kohlenstoffatomen mono- oder disubstituiert sein kann, oder eine gegebenenfalls durch eine Alkyl- oder Phenylgruppe mono- oder disubstituierte Maleinsäureimido- oder Succinimidogruppe, wobei die Substituenten gleich oder verschieden sein können, darstellt, eine Verbindung der Formel

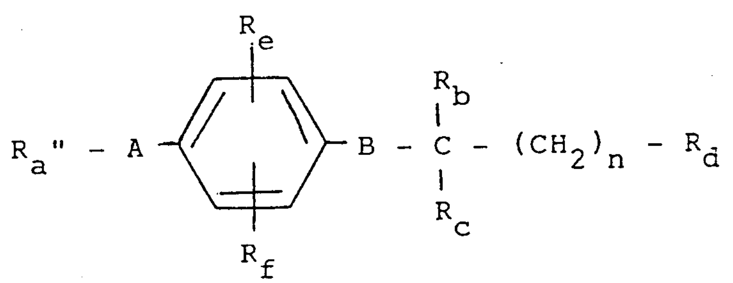

,(XIV)

in der
$R_b$ bis $R_f$, A, B und n wie in den Ansprüchen 1 bis 5 definiert sind und
Ra" einen der für $R_a$ in den Ansprüchen 1 bis 5 erwähnten Reste darstellt, in dem $R_5$ eine gegebenenfalls durch eine Alkylgruppe mit 1 bis 6 Kohlenstoffatomen, durch eine Phenyl-, Cycloalkyl-, Phenylalkyl-, Cycloalkylalkyl-, Bicyclohexyl- oder Biphenylgruppe substituierte Aminogruppe darstellt, mit einer Verbindung der Formel

$HO - U - R_{12}$ ,(XV)

in der
U eine Carbonyl- oder Sulfonylgruppe und
$R_{12}$ eine Alkylgruppe mit 1 bis 6 Kohlenstoffatomen, eine Alkoxygruppe mit 1 bis 3 Kohlenstoffatomen, eine Phenyl-, Phenylalkyl-, Naphthyl- oder Cycloalkylgruppe, wobei die vorstehend erwähnten Phenylkerne jeweils durch ein Fluor-, Chlor- oder Bromatom, durch eine Methyl- oder Methoxygruppe mono- oder disubstituiert und die Substituenten gleich oder verschieden sein können, oder auch, wenn U eine Carbonylgruppe darstellt, ein Wasserstoffatom, eine Phenylgruppe, die in o-Stellung durch eine Carboxy- oder Carboxymethylgruppe substituiert ist und in der der Phenylkern durch Alkyl- oder Alkoxygruppen mit jeweils 1 bis 3 Kohlenstoffatomen mono- oder disubstituiert sowie zusätzlich ganz oder teilweise hydriert sein kann, eine 2-Carboxy-ethyl- oder 2-Carboxy-ethenylgruppe, in welcher der Ethyl- und Ethenylteil jeweils durch eine Alkyl- oder Phenylgruppe mono- oder disubstituiert sein kann, bedeuten, oder mit deren reaktionsfähigen Derivaten acyliert wird oder
j) zur Herstellung von Verbindungen der allgemeinen Formel I, in der $R_b$ eine Phosphono- oder O-Alkyl-phosphonogruppe darstellt, eine Verbindung der allgemeinen Formel

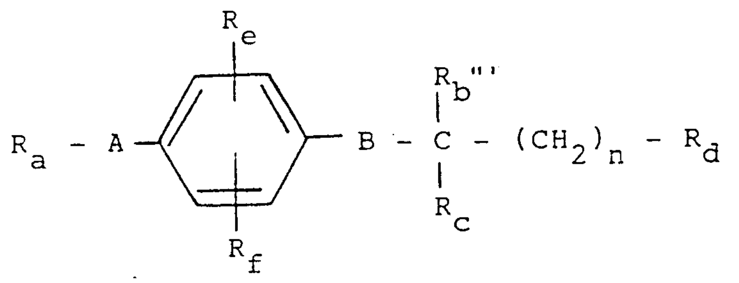

,(XVI)

in der
A, B, $R_a$, $R_c$ bis $R_f$ und n wie in den Ansprüchen 1 bis 5 definiert sind und
$R_b'''$ eine O-Alkyl- oder O,O-Dialkyl-phosphonogruppe darstellt, in welcher der Alkylteil jeweils 1 bis 5 Kohlenstoffatome enthalten kann, hydrolysiert wird und

gewünschtenfalls anschließend eine so erhaltene Verbindung der allgemeinen Formel I, in der $R_2$ oder $R_5$ eine Nitrogruppe darstellt, mittels Reduktion in eine entsprechende Verbindung der allgemeinen Formel I, in der $R_2$ oder $R_5$ eine Aminogruppe darstellt, übergeführt wird oder

eine so erhaltene Verbindung der allgemeinen Formel I, in der B eine Iminogruppe darstellt, mittels Alkanoylierung in eine entsprechende Verbindung der allgemeinen Formel I übergeführt werden, in der B eine durch eine Alkanoylgruppe mit 1 bis 4 Kohlenstoffatomen substituierte Iminogruppe darstellt, übergeführt wird oder

eine so erhaltene Verbindung der allgemeinen Formel I, in der B eine Iminogruppe darstellt, mittels Alkylierung in eine entsprechende Verbindung der allgemeinen Formel I übergeführt werden, in der B eine durch eine Alkylgruppe mit 1 bis 3 Kohlenstoffatomen substituierte Iminogruppe darstellt, übergeführt wird oder

eine so erhaltene Verbindung der allgemeinen Formel I, in der $R_b$ oder $R_b$ und $R_5$ jeweils eine Carboxygruppe darstellen, mittels Veresterung in eine entsprechende Verbindung der allgemeinen Formel I, in der $R_b$ oder $R_b$ und $R_5$ eine Gruppe darstellt, die in vivo metabolisch in eine Carboxygruppe umgewandelt wird, übergeführt wird oder

eine so erhaltene Verbindung der allgemeinen Formel I, in der $R_b$ eine Carboxygruppe darstellt, nach Überführung in ein entsprechendes Säurehalogenid mittels Umsetzung mit einem entsprechenden Sulfonamid in eine Verbindung der allgemeinen Formel I, in der $R_b$ eine Alkansulfonylaminocarbonyl-, Perfluoralkansulfonylaminocarbonyl-, Arylsulfonylaminocarbonyl- oder Benzylsulfonylaminocarbonylgruppe darstellt, übergeführt wird und

erforderlichenfalls ein während der Umsetzungen zum Schutze von reaktiven Gruppen verwendeter Schutzrest abgespalten wird und/oder

gewünschtenfalls anschließend ein so erhaltenes Isomerengemisch einer Verbindung der allgemeinen Formel I mittels Isomerentrennung in ihre Isomeren oder Enantiomeren aufgetrennt wird oder

eine so erhaltene Verbindung der allgemeinen Formel I in ihr Salz, insbesondere für die pharmazeutische Anwendung in ihr physiologisch verträgliches Salz mit einer anorganischen oder organischen Säure oder Base, übergeführt wird.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung

EP 92 11 1591

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl. 5) |
|---|---|---|---|
| D,A | EP-A-0 412 848 (IMPERIAL CHEMICAL INDUSTRIES PLC)<br>--- | | C07D235/08<br>A61K31/415<br>C07D235/18<br>C07D215/22<br>C07D239/74<br>C07D403/14<br>C07D471/04<br>A61K31/47<br>A61K31/505<br>A61K31/41<br>A61K31/44 |
| D,A | EP-A-0 412 594 (MERCK & CO. INC.)<br>--- | | |
| D,A | EP-A-0 419 048 (MERCK & CO. INC.)<br>--- | | |
| D,A | EP-A-0 409 332 (MERCK & CO. INC.)<br>--- | | |
| D,A | EP-A-0 411 766 (MERCK & CO. INC.)<br>----- | | |
| | | | **RECHERCHIERTE SACHGEBIETE (Int. Cl. 5)** |
| | | | C07D<br>A61K |

## UNVOLLSTÄNDIGE RECHERCHE

**Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.**
**Vollständig recherchierte Patentansprüche:**
**Unvollständig recherchierte Patentansprüche:**
**Nicht recherchierte Patentansprüche:**
**Grund für die Beschränkung der Recherche:**

Siehe Ergänzungsblatt C

| Recherchenort | Abschlußdatum der Recherche | Prüfer |
|---|---|---|
| DEN HAAG | 04 NOVEMBER 1992 | DE BUYSER I.A.F. |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
..................................................
& : Mitglied der gleichen Patentfamilie, übereinstimmendes Dokument

EPO FORM 1503 03.82 (P04E09)

EP 92 11 1591 -C-

Vollständig recherchierte Patentansprüche : 5
Unvollständig recherchierte Patentansprüche: 1-4,6-10

Grund : Die Abfassung der Ansprüche ist nicht klar und knapp zu fassen (Art. 83-84, EPA) und enthalt eine so grosse Zahl Verbindungen dass eine vollständige Recherche auf ökonomischer Gründe nicht möglich ist (Siehe Richtlinien für die Prüfung im Europaïschen Patentamt, Teil B, Kapittel III,2 (Umfang der Recherche)). Geleitet durch den Sinn der Anfrage und die erfinderische Idee als offenbart in die Beschreibung der vorliegende Anfrage, ist die Recherche gegründet auf die Beispiele.